# EUROPEAN PATENT APPLICATION

(11) **EP 4 088 782 A1**
(43) Date of publication of application: **16.11.2022**
(21) Application number: 21173432.2
(22) Date of filing: 11.05.2021
(51) Int. Cl.: A61P 31/14, C07K 16/10, A61K 39/12, A61K 39/00

(54) **ANTIBODIES**

(71) Applicant: ModiQuest B.V., 5349 AB Oss (NL)
(72) Inventor: RAATS, Jozef Maria Hendrik, 6525 XV Nijmegen (NL)
(74) Representative: J A Kemp LLP

(57) **Abstract**

The invention relates to antibodies and cocktails of antibodies useful for the prevention, treatment and/or diagnosis of coronavirus infections, and diseases and/or complications associated with coronavirus infections, including COVID-19.

## Description

### Field of invention

The invention relates to antibodies and cocktails of antibodies useful for the prevention, treatment and/or diagnosis of coronavirus infections, and diseases and/or complications associated with coronavirus infections, including COVID-19.

### Background of the invention

The novel Coronavirus disease 2019 (COVID-19) is caused by a new severe acute respiratory syndrome coronavirus 2 (SARS-CoV-2). An infection with SARS-CoV-2 is initiated by the spike protein (S). This protein binds to the cellular receptor angiotensinconverting enzyme 2 (ACE2) during infection. The homotrimeric S protein is comprised of monomers which consist of a N-terminal S1 subunit responsible for receptor binding and a C-terminal S2 subunit responsible for membrane fusion. The receptor-binding domain (RBD) of S interacts with ACE2, and is a primary target for neutralizing antibodies. In patients infected with COVID-19 and vaccinated animals there is a dominance of RBD-directed antibodies in the neutralizing antibody response.

The RBD comprises a core structure, and a receptor-binding motif (RBM), which mediates direct contact with ACE2. The RBM contains antigenic sites that are capable of eliciting potent neutralizing antibodies. Several highly potent neutralizing monoclonal antibodies (mAbs) targeting the RBM have been isolated from COVID-19 patients. In addition, a number of core region-directed antibodies, cross-reactive to SARS-CoV, have also been identified. Despite its important critical function, the RBD domain of SARS-CoV-2 continuously evolves. Clear evidence is provided by the emergence of viral isolates with diverse amino acid changes in both the RBM and core regions of the RBD (22). Since the binding to ACE2 tolerates a substantial number of mutations (23-24), the concern of neutralization escape is raised. This might limit the therapeutic potential of neutralizing antibodies. A recent study, using a recombinant Vesicular Stomatitis Virus (VSV) that expresses S of SARS-CoV-2, demonstrated the rapid occurrence of escape mutants in the presence of RBD-specific single mAbs (25).

Antibody cocktails are promising treatments that can prevent SARS-CoV-2 escape (1, 2). For instance it was shown that two antibodies that bound to non-overlapping epitopes on the receptor-binding domain (RBD) prevented viral escape (3). Multiple antibody cocktails in clinical trials (4, 5, 6, 7) are being evaluated for neutralization activities.

### Summary of the invention

The inventors have generated a number of antibodies having promising characteristics. The antibodies (i) bind to S1 or RBD; (ii) have high neutralizing efficiency against SARS-CoV-2; (iii) have high binding affinities; and/or (iv) maintain binding affinity for known variants of SARS-CoV-2. The inventors have also identified a cocktail of four antibodies that target the RBD, have characteristics (i) to (iv), particularly maintaining binding affinity for the South African RBD mutant (having mutations N501Y, K417N, E484K), and cooperate with each other to prevent escape mutations. The mAbs bind to non-overlapping epitopes of the RBD and independently block RBD and ACE2 interaction. The cocktail prevents SARS-CoV-2 escape mutations through a mechanism of imposing stronger mutational constraints on the RBD than individual mAbs. Hence, these antibodies are particularly useful both singly and in combination for treating and detecting SARS-CoV-2.

Accordingly, an aspect of the invention provides an antibody or an antigen-binding fragment thereof that binds to the spike protein of coronavirus SARS-CoV-2, wherein the antibody comprises a set of three heavy chain complementarity determining regions (CDRH1, CDRH2 and CDRH3) and three heavy chain complementarity determining regions (CDRL1, CDRL2 and CDRL3), wherein the set of CDRH1, CDRH2, CDRH3, CDRL1, CDRL2 and CDRL3 amino acid sequences are selected from: (a) SEQ ID NOs: 1170, 1171, 1172, 1174, 1175 and 1176; (b) SEQ ID NOs: 1186, 1187, 1188, 1190, 1191 and 1192; (c) SEQ ID NOs: 1202, 1203, 1204, 1206, 1207 and 1208; and (d) SEQ ID NOs: 1194, 1195, 1196, 1198, 1199 and 1200; or are selected from (a) SEQ ID NOs: 1170, 1171, 1172, 1174, 1175 and 1176; (b) SEQ ID NOs: 1186, 1187, 1188, 1190, 1191 and 1192; (c) SEQ ID NOs: 1202, 1203, 1204, 1206, 1207 and 1208; (d) SEQ ID NOs: 1194, 1195, 1196, 1198, 1199 and 1200; (e) SEQ ID NOs: 770, 771, 724, 774, 775 and 776; (f) SEQ ID NOs: 810, 811, 812, 814, 815 and 816; and (g) SEQ ID NOs: 786, 787, 788, 790, 791 and 792.

Alternatively, the invention provides an antibody or an antigen-binding fragment thereof that binds to the spike protein of coronavirus SARS-CoV-2, wherein the antibody comprises the six CDRs, or the heavy and/or light chain variable regions, of an antibody in any one of Tables 1 to 6, or wherein the antibody comprises the six CDRs, or the heavy and/or light chain variable regions, of an antibody selected from Ab391, Ab371, Ab411, Ab378, Ab579, Ab580, Ab430, Ab433, Ab381, Ab379, Ab387, Ab564, Ab415 and Ab413 as described herein, or selected from Ab391, Ab371, Ab411, Ab378, Ab579, Ab580, Ab430, Ab433, Ab381, Ab379, Ab387, Ab564, Ab415, Ab413, Ab431, Ab419 and Ab413 as described herein.

The invention also provides a combination of antibodies comprising two or more of the antibodies of the invention, or a combination comprising or consisting of one of each of the four antibodies according to (a) to (d) above; or (a), (b), (d) and (e); or (a), (b), (d) and (f); or (a), (b), (d) and (g) above.

The invention also provides a pharmaceutical composition comprising the antibody or combination of antibodies, and optionally at least one pharmaceutically acceptable diluent or carrier.

The invention also provides the antibody, the combination of antibodies or the pharmaceutical composition according to the invention, for use in a method of treating or preventing a disease or a complication associated with coronavirus infection.

The invention also provides a method of treating a subject comprising administering a therapeutically effective amount of the antibody, the combination of antibodies or the pharmaceutical composition to the subject, or for treating or preventing a coronavirus infection, or a disease or complication associated with coronavirus infection.

The invention also provides the antibody, the combination of antibodies, or the pharmaceutical composition, for use in a method for treatment of a human or animal body by therapy, or for use in a method of treating or preventing a coronavirus infection, or a disease or complication associated with coronavirus infection.

The invention also provides the use of the antibody, the combination of antibodies or the pharmaceutical composition according to the invention in the manufacture of a medicament for treating a subject, or for treating or preventing a coronavirus infection, or a disease or complication associated with coronavirus infection.

The invention also provides a method of identifying the presence of coronavirus, or a protein or a protein fragment thereof, in a sample, comprising: (i) contacting the sample with an antibody or combination of antibodies of the invention; and (ii) detecting the presence or absence of an antibody-antigen complex, wherein the presence of the antibody-antigen complex indicates the presence of coronavirus, or a protein or a protein fragment thereof, in the sample. The invention also provides a method of treating or preventing coronavirus infection, or a disease or complication associated therewith, in a subject, comprising identifying the presence of coronavirus according to the method of the invention, and treating the subject with an anti-viral or an anti-inflammatory agent.

The invention also provides one or more polynucleotides that encode the antibody or combination of antibodies according to the invention, one or more vectors comprising the one or more polynucleotides, or a host cell comprising the one or more vectors.

The invention also provides a method for producing an antibody of the invention, the method comprising culturing a host cell of the invention and isolating the antibody from the culture.

The invention also provides the use of the antibody, the combination of antibodies, or the pharmaceutical composition according to the invention for preventing, treating and/or diagnosing coronavirus infection, or a disease or complication associated therewith.

The disclosure will now be described in more detail, by way of example and not limitation, and by reference to the accompanying drawings. Many equivalent modifications and variations will be apparent, to those skilled in the art when given this disclosure. Accordingly, the exemplary embodiments of the disclosure set forth are considered to be illustrative and not limiting. Various changes to the described embodiments may be made without departing from the scope of the invention, which is defined by the claims. All documents cited herein, whether supra or infra, are expressly incorporated by reference in their entirety.

### Brief description of the figures

**Fig 1** **-** ELISA with SARS-Cov2 RBD and South African variant **B.1.351** RBD.
Fig 2 - ELISA with MERS S1, SARS-CoV1 S1, and SARS-Cov2 RBD.
**Fig 3** - Neutralisation of Ace-2 - SARS-CoV2 binding.
**Fig 4** - ELISA binding assay. ScFv/VHH-hinge-hIgGFc antibodies in supernatant (1 µg/mL) were tested for binding to RBD.
**Fig 5** - ELISA neutralization assay. ScFv/VHH-hinge-hIgGFc antibodies in supernatant (end conc. 65 µg/mL) were tested for their ability to neutralize RBD domain from binding to the ACE2-receptor in the SARS-CoV-2 Surrogate Virus Neutralization Test Kit from GenScript.
**Fig 6** - Epitope binning of antibodies. (A) In-tandem cross-competition assay set-up. (B-M) In-tandem assay of two antibodies binding to RBD. (N) Binning map where "+" denotes additional binding and "-" denotes cross-competition.
**Fig 7** - (A) ELISA binding assay to RBD mutants. ScFv/VHH-Fc antibodies in supernatant (1 µg/m L) were tested for binding to various RBD mutants; (B) ELISA binding assay to RBD and S1 mutants. Full size IgG1 antibodies in PBS from 0.01 to 1 ug/ml were tested in ELISA for binding to various mutants of RBD and S1 protein.

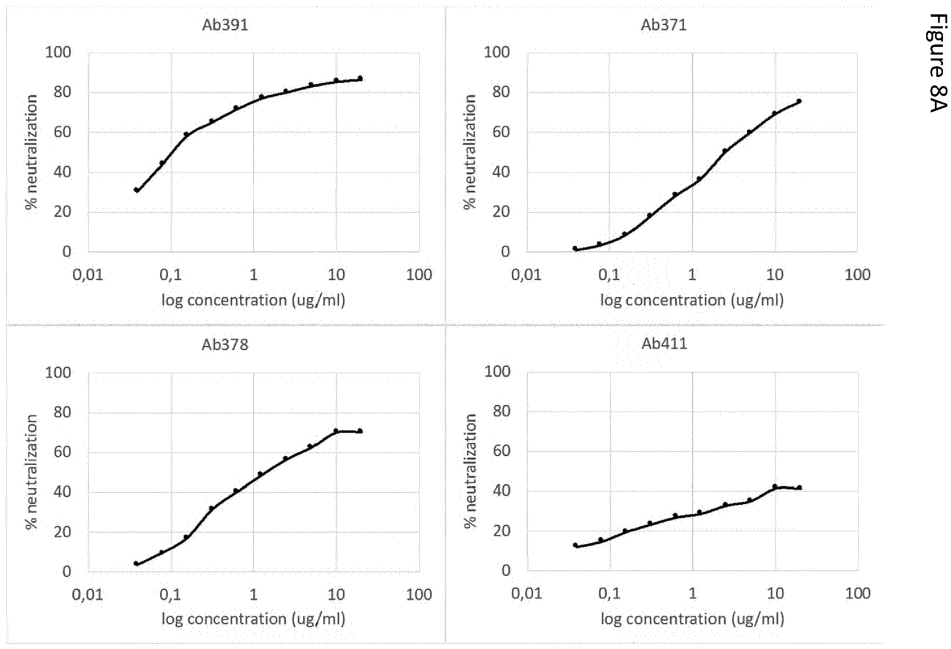

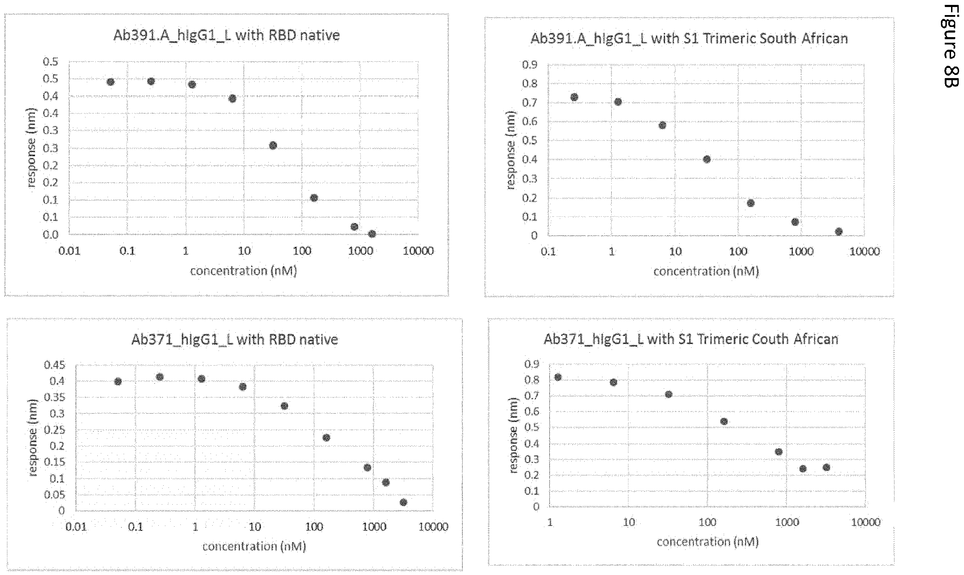

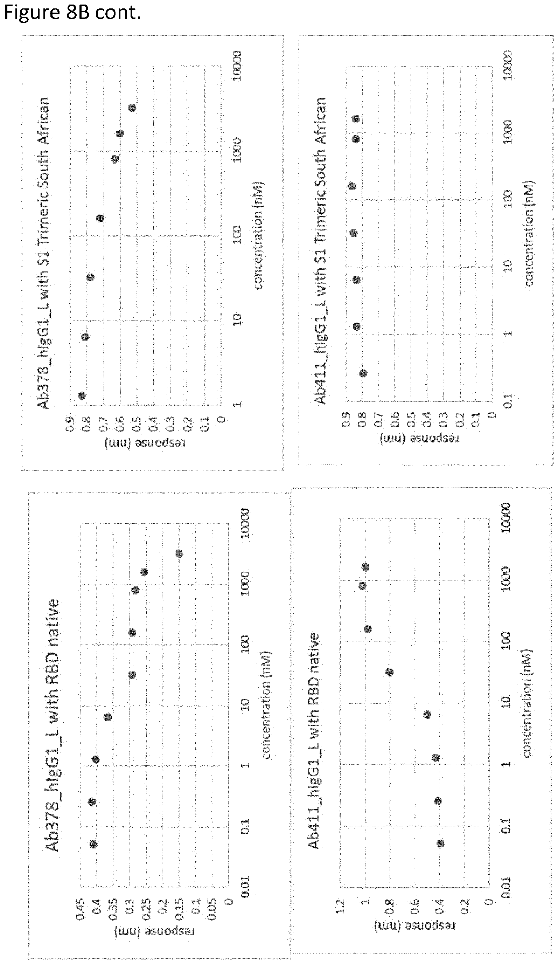

**Fig 8** - (A) Neutralization titration assay. The neutralization titration curves of 4 ScFv-hinge-hIgGFc tested in a serial dilution (0.039 - 20 ug/ml); (B) Neutralization titration assay. The neutralization titration curves (B) for 4 full size IgG1 antibodies tested in serial dilution on Octet with the native Wuhan-Hu-1 RBD and South African B. 1.351 S1 protein. 25 nM Ace2 protein was coated on the Biosensor. 50 nM RBD or S1 was added together with 3200 to 0.0512 nM antibody or 1600 to 0.0512 nM antibody or 4000 to 0.0512nM antibody were used to detect binding of S1 to Ace2 protein. Measurements were performed on Octet96: 600 seconds loading Ace2 (25nM), followed by quenching 120 with biocytin (10ug/ml) and 200 sec wash. Then measurement of S1 (50nM) binding with or without antibody for 600 sec.
**Fig 9** - Biolayer interferometry (BLI) antibody affinity measurement. Raw sensorgram data showing the association and dissociation steps of Ab391 at different concentrations (25 - 1.56 nM) and baseline (no binding of Ab391).
**Fig 10** - Epitope binning of four antibody samples. In-tandem cross competition assay of four antibody samples showing additional binding towards RBD (25nM) loaded sensors.

### Description of the Sequences

SEQ ID NOs: 1 to 760 set forth antibody sequences provided in Table 1b.
SEQ ID NOs: 761 to 940 set forth the antibody sequences provided in Table 2c.
SEQ ID NOs: 941 to 1048 set forth the antibody sequences provided in Table 3b.
SEQ ID NOs: 1049 to 1168 set forth the antibody sequences provided in Table 4c.
SEQ ID NOs: 1169 to 1208 set forth the antibody sequences provided in Table 5.
SEQ ID NOs: 1209 to 1260 set forth the antibody sequences provided in Table 6.
SEQ ID NOs: 1261 to 1436 set forth further antibody sequences provided in Table 1b.
SEQ ID NOs: 1437 to 1444 set forth further antibody sequences provided in Table 3b.
SEQ ID NOs: 1445 sets forth the amino acid sequence of the spike protein of reference SARS-CoV-2 (2019-nCoV), Wuhan-Hu-1 strain.
SEQ ID NOs: 1446 to 1463 set forth the polynucleotide sequences for exemplary full length antibodies Ab391(a), Ab391(b), Ab371, Ab378, Ab411, Ab419(a), Ab419(b) and Ab413.
SEQ ID NOs: 1464-1481 set forth the polynucleotide sequences for the variable heavy and light chain variable regions of antibodies Ab391(a), Ab391(b), Ab371, Ab378, Ab411, Ab419(a), Ab419(b) and Ab413.

### Detailed description of the invention

### Antibodies of the invention

An antibody of the invention specifically binds to the spike protein of SAR-CoV-2, to the S1 subunit of the spike protein, or more specifically to the receptor binding domain (RBD).

An antibody of the invention may comprise at least the six CDRs of an antibody listed in any one of Tables 1 to 6, or an antibody selected from any two or more of Tables 1 to 6, e.g., selected from Table 5, or selected from Tables 5 and 6, or of an antibody selected from Ab391, Ab371, Ab411, Ab378, Ab579, Ab580, Ab430, Ab433, Ab381, Ab379, Ab387, Ab564, Ab415 and Ab413, or selected from Ab391, Ab371, Ab411, Ab378, Ab579, Ab580, Ab430, Ab433, Ab381, Ab379, Ab387, Ab564, Ab415, Ab413, Ab431, Ab419 and Ab413 as described herein.

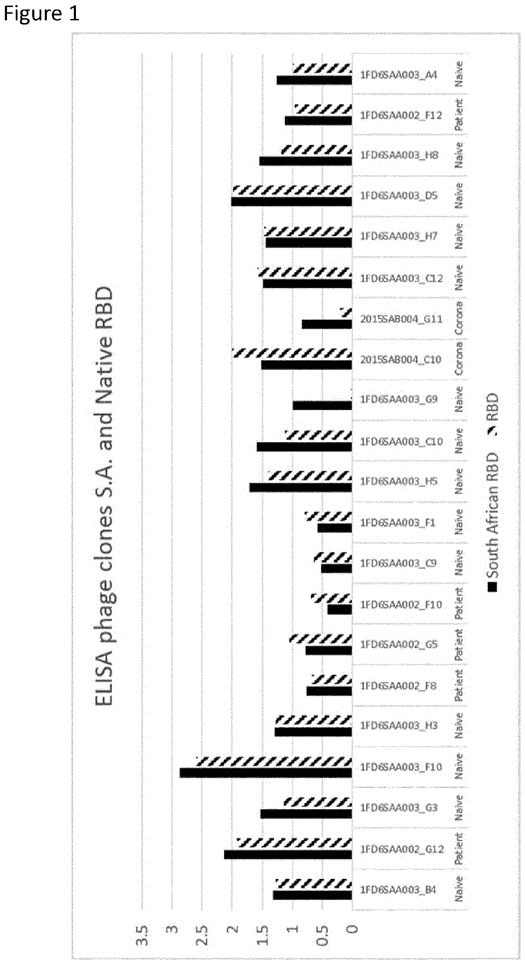

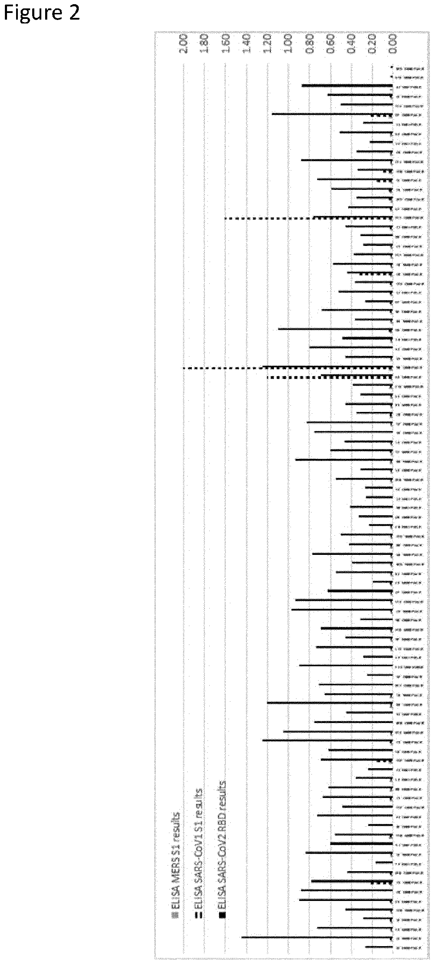

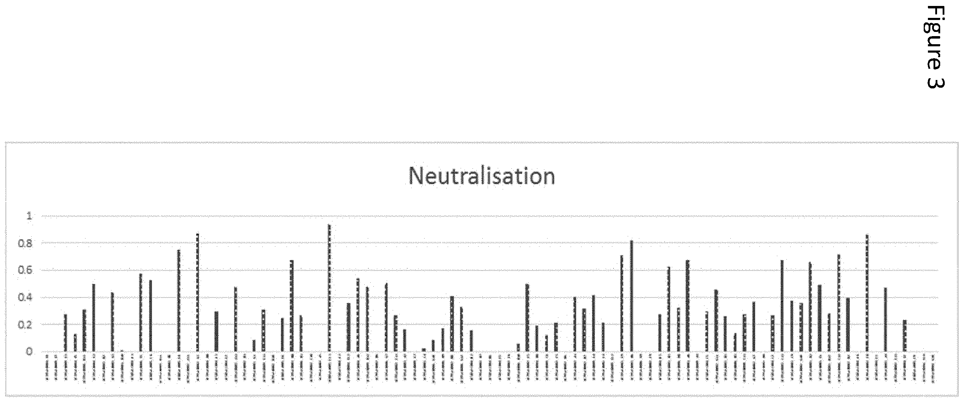

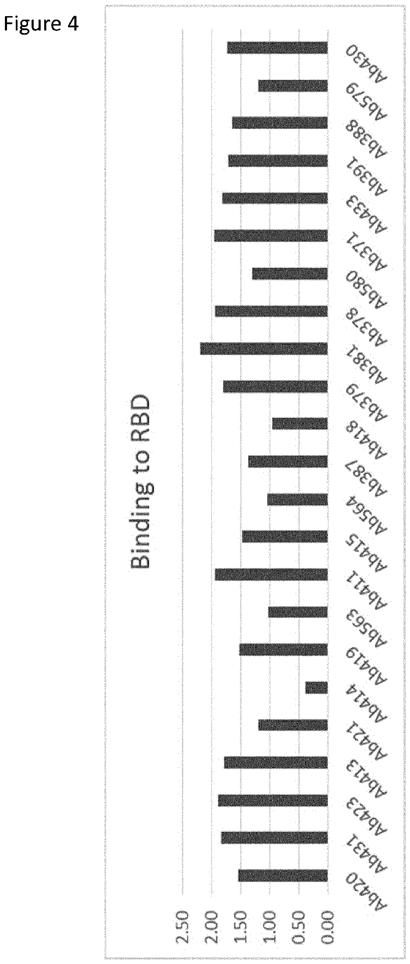

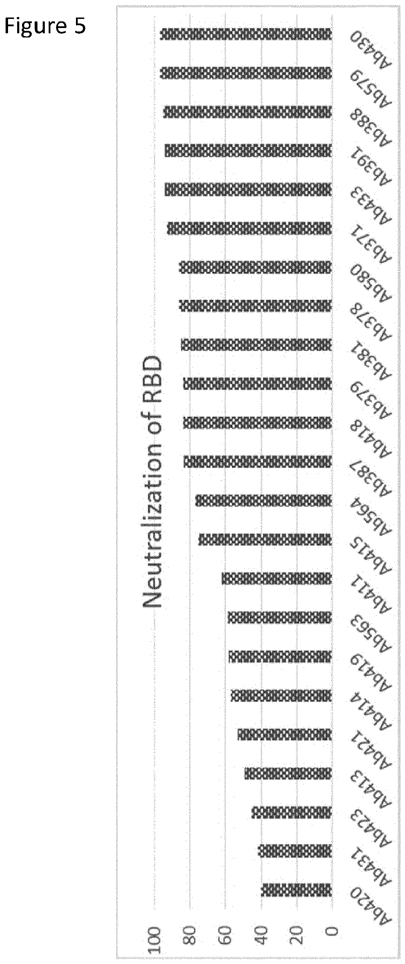

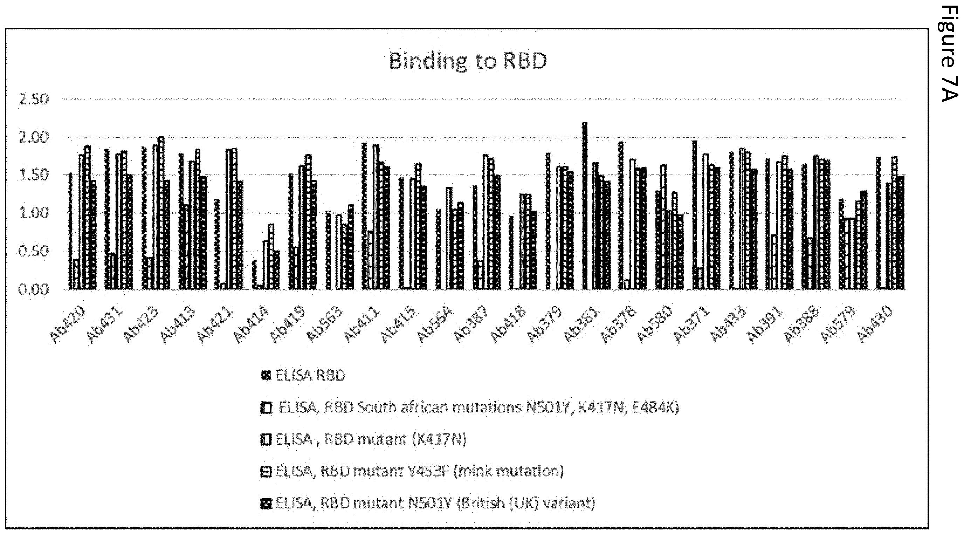

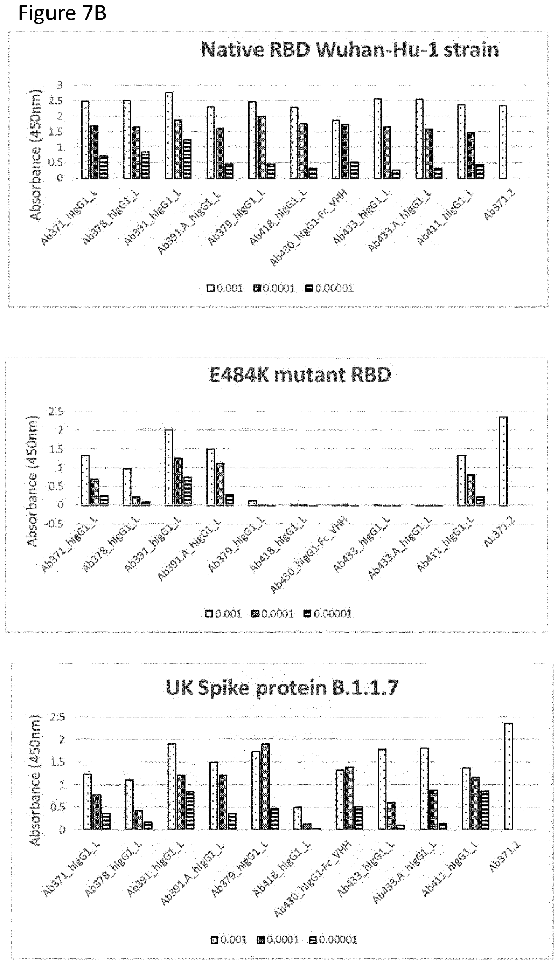

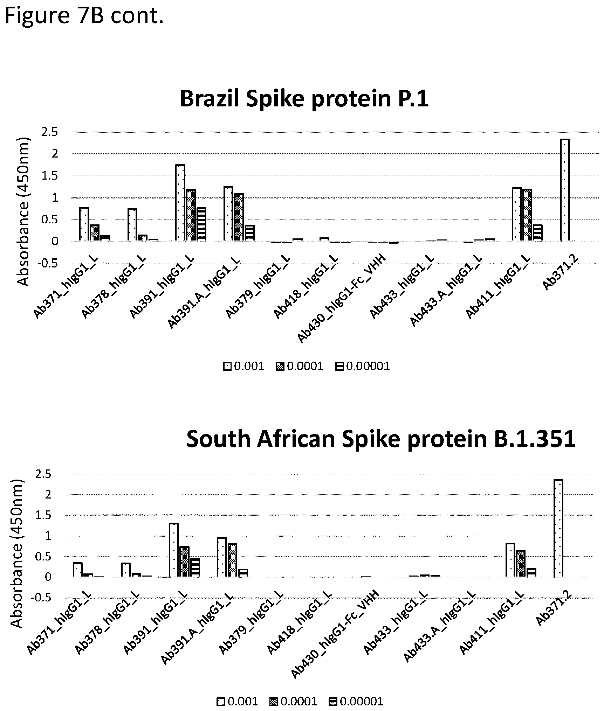

The antibody may comprise the heavy chain variable region (HCVR) and/or the light chain variable region (LCVR) of any one of these antibodies. One exception is that the antibody may comprise the three heavy chain CDRs, or the variable chain heavy chain sequence, of antibody Ab430. Ab430 is a VHH obtained from a Corona patient library as described in the Examples below and having no light chain sequence. The antibody may be one shown in Figure 1 or Figure 2 to have high or low cross reactivity with SARS-CoV1 S1 and/or MERS S1 and or variant B.1.351. The antibody may be one shown in Figure 3 and/or Figure 5 to have high neutralisation of ACE-2 to SARS-CoV2 binding, e.g. an antibody having a neutralization efficiency of at least 40%, or at least 50%, or at least 60%, or 70%, 80%, 90% or 95%, as shown in Figure 5. The antibody may be one shown in Figure 4 and/or Figure 7 A and/or B to have high binding affinity for SARS-CoV2 RBD. The antibody may be one shown in Figure 7A or B to have high binding affinity for any one or more of the variant RBDs, e.g. the South African variant having mutations N501Y, K417N and E484K.

The CDRs of the heavy chain (CDRH) and light chain variable domain (CDRL) are located at residues 27-38 (CDR1), residues 56-65 (CDR2) and residues 105-117 (CDR3) of each chain according to the IMGT numbering system (http://www.imgt.org; Lefranc MP, 1997, J, Immunol. Today, 18, 509). This numbering system is used in the present specification except where otherwise indicated.

The antibody of the invention may comprise at least four, five, or all six CDRs of an antibody selected from any one of Tables 1 to 6 or the other groups of antibodies described herein. The antibody may comprise at least one, at least two, or all three heavy chain CDRs (CDRHs). The antibody may comprise at least one, at least two, or all three light chain CDRs (CDRLs). The antibody typically comprises all six (i.e. three heavy and three light chain) CDRs.

The antibody of the invention may comprise a heavy chain variable region having ≥80%, ≥85%, ≥90%, ≥95%, ≥96%, ≥97%, ≥98%, ≥99% or 100% sequence identity to the heavy chain variable region amino acid sequence of any of these antibodies.

The antibody of the invention may comprise a light chain variable region having ≥80%, ≥85%, ≥90%, ≥95%, ≥96%, ≥97%, ≥98%, ≥99% or 100% sequence identity to the light chain variable region amino acid sequence of any of these antibodies.

The antibody of the invention may comprise a heavy chain variable region having ≥80%, ≥85%, ≥90%, ≥95%, ≥96%, ≥97%, ≥98%, ≥99% or 100% sequence identity to the heavy chain variable region amino acid sequence of any of these antibodies and a light chain variable region having ≥80%, ≥85%, ≥90%, ≥95%, ≥96%, ≥97%, ≥98%, ≥99% or 100% sequence identity (or the same minimal percentage identity as defines the heavy chain variable region) to the matching light chain variable region amino acid sequence of the same antibody, as shown in Tables 1 to 6. For example, in one embodiment, the antibody comprises heavy and light chain variable regions having the amino acid sequences of (a) SEQ ID NOs: 1169 or 1177 and 1173; (b) SEQ ID NOs: 1185 and 1189; (c) SEQ ID NOs: 1201 and 1205; (d) SEQ ID NOs: 1193 and 1197; (e) SEQ ID NOs: 769 and 773; (f) SEQ ID NOs: 809 and 813; or (g) SEQ ID NOs: 785 and 789.

Typically, the non-identical amino acids of a variable region are not in the CDRs. Hence, an antibody of the invention may comprise 100% sequence identity over all six CDRs of a reference antibody, but only ≥80%, ≥85%, ≥90%, ≥95%, ≥96%, ≥97%, ≥98%, ≥99% or 100% sequence identity to the heavy and/or light chain variable regions of the same reference antibody.

In one embodiment, the invention provides any one of the antibodies listed in any one of Tables 1 to 6, or an antibody selected from any two or more of Tables 1 to 6, or of an antibody selected from Ab391, Ab371, Ab411 and Ab378, or an antibody selected from Ab391, Ab371, Ab411, Ab378, Ab431, Ab419 and Ab413, or an antibody selected from Ab391, Ab371, Ab411, Ab378, Ab579, Ab580, Ab430, Ab433, Ab381, Ab379, Ab387, Ab564, Ab415 and Ab413, or an antibody selected from Ab391, Ab371, Ab411, Ab378, Ab579, Ab580, Ab430, Ab433, Ab381, Ab379, Ab387, Ab564, Ab415, Ab413, Ab431, Ab419 and Ab413, or selected from any of the other groups described herein.

An antibody of the invention may comprise a modification from the amino acid sequence of an antibody in any one of Tables 1 to 6, whilst maintaining the activity and/or function of the antibody. The modification may be a substitution, deletion and/or addition. For example, the modification may comprise 1, 2, 3, 4, 5, up to 10, up to 20, up to 30 or more amino acid substitutions and/or deletions from the amino acid sequence of the antibody in one of Tables 1 to 6. For example, the modification may comprise an amino acid substituted with an alternative amino acid having similar properties. Some properties of the 20 main amino acids, which can be used to select suitable substituents, are as follows:

| | | | |
|---|---|---|---|
| Ala | aliphatic, hydrophobic, neutral | Met | hydrophobic, neutral |
| Cys | polar, hydrophobic, neutral | Asn | polar, hydrophilic, neutral |
| Asp | polar, hydrophilic, charged (-) | Pro | hydrophobic, neutral |
| Glu | polar, hydrophilic, charged (-) | Gln | polar, hydrophilic, neutral |
| Phe | aromatic, hydrophobic, neutral | Arg | polar, hydrophilic, charged (+) |
| Gly | aliphatic, neutral | Ser | polar, hydrophilic, neutral |
| His | aromatic, polar, hydrophilic, charged (+) | Thr | polar, hydrophilic, neutral |
| Ile | aliphatic, hydrophobic, neutral | Val | aliphatic, hydrophobic, neutral |
| Lys | polar, hydrophilic, charged (+) | Trp | aromatic, hydrophobic, neutral |
| Leu | aliphatic, hydrophobic, neutral | Tyr | aromatic, polar, hydrophobic |

The modification may comprise a derivatised amino acid, e.g. a labelled or non-natural amino acid, providing the function of the antibody is not significantly adversely affected.

Modification of antibodies of the invention as described above may be prepared during synthesis of the antibody or by post-production modification, or when the antibody is in recombinant form using the known techniques of site-directed mutagenesis, random mutagenesis, or enzymatic cleavage and/or ligation of nucleic acids.

Antibodies of the invention may be modified (e.g. as described above) to improve the potency of said antibodies or to adapt said antibodies to new SARS-CoV-2 variants. The modifications may be amino acid substitutions to adapt the antibody to substitutions in a virus variant. For example, the known mode of binding of an antibody to the spike protein (e.g. by crystal structure determination, or modelling) may be used to identify the amino acids of the antibody that interact with the substitution in the virus variant. This information can then be used to identify possible substitutions of the antibody that will compensate for the change in the epitope characteristics. For example, a substitution of a hydrophobic amino acid in the spike protein to a negatively changes amino acid may be compensated by substituting the amino acid from the antibody that interacts with said amino acid in the spike protein to a positively charged amino acid. Methods for identifying residues of an antibody that may be substituted are known in the art.

Antibodies of the invention may be isolated antibodies. An isolated antibody is an antibody which is substantially free of other antibodies having different antigenic specificities.

The term 'antibody' as used herein may relate to whole, full-length antibodies (i.e. comprising two antibody heavy chains and two light chains inter-connected by disulphide bonds), as well as antigen-binding fragments thereof. The term may also encompass VHH antibodies (heavy-chain antibodies), having two heavy chains only and no light chains, and antigen binding fragments thereof. Antibodies typically comprise immunologically active portions of immunoglobulin (Ig) molecules, i.e., molecules that contain an antigen binding site that specifically binds (immunoreacts with) an antigen. By "specifically binds" or "immunoreacts with" is meant that the antibody reacts with one or more antigenic determinants of an antigen, i.e. in the normal way that antibodies bind to antigen. Each heavy chain is typically comprised of a heavy chain variable region (abbreviated herein as HCVR or VH) and at least one heavy chain constant region. Each light chain is typically comprised of a light chain variable region (abbreviated herein as LCVR or VL) and a light chain constant region. Typically the variable regions of the heavy and light chains contain a binding domain that interacts with an antigen, except for VHH antibodies where the variable regions of the heavy chains only comprise the antigen binding domain. The VH and VL regions can be further subdivided into regions of hypervariability, termed complementarity determining regions (CDR), interspersed with regions that are more conserved, termed framework regions (FR). Antibodies may include, but are not limited to, polyclonal, monoclonal, chimeric, recombinant, dAb (domain antibody), single chain, Fab (fragment antigen-binding regions), Fab' and F(ab')2 fragments, scFvs (single chain variable fragments), and Fab expression libraries.

An antibody of the invention may be a monoclonal antibody. Monoclonal antibodies (mAbs) of the invention may be produced by a variety of techniques, including conventional monoclonal antibody methodology, for example those disclosed in "Monoclonal Antibodies: a manual of techniques"(Zola H, 1987, CRC Press) and in "Monoclonal Hybridoma Antibodies: techniques and applications" (Hurrell JGR, 1982 CRC Press).

The constant region domains of an antibody molecule of the invention, if present, may be selected having regard to the proposed function of the antibody molecule, and in particular the effector functions which may be required. For example, the constant region domains may be human IgA, IgD, IgE, IgG or IgM domains. In particular, human IgG constant region domains may be used, especially of the IgG1 and IgG3 isotypes when the antibody molecule is intended for therapeutic uses where antibody effector functions are required. Alternatively, IgG2 and IgG4 isotypes may be used when the antibody molecule is intended for therapeutic purposes and antibody effector functions are not required. Typically, the constant regions are of human origin. In particular, human IgG (i.e. IgG1, IgG2, IgG3 or IgG4) constant region domains may be used. Most typically, a human IgG1 constant region is used, or mutant variant thereof that lacks effector functions, such as a variant having the VH domain LaLaNa mutation of the human Fc of IgG1.

The light chain constant region may be either lambda or kappa. The inventors found a strong bias for the use of Lamda light chains in the antibodies described herein. Hence the light chain constant region may typically be lambda.

An antibody of the invention may be a chimeric antibody, a CDR-grafted antibody, a nanobody, a human or humanised antibody. Typically, the antibody is a human antibody. Fully human antibodies are those antibodies in which the variable regions and the constant regions (where present) of both the heavy and the light chains are all of human origin, or substantially identical to sequences of human origin, but not necessarily from the same antibody.

The antibody of the invention may be an antigen-binding fragment. An antigen-binding fragment of the invention binds to the same epitope as a reference full length antibody or parent antibody, i.e. the antibody from which the antigen-binding fragment is derived. An antigen-binding fragment of the invention typically retains the parts of the parent antibody that interact with the epitope. Typically, the antigen-binding fragment retains the same or similar binding affinity to the antigen as the reference antibody. Methods for creating and manufacturing antibody fragments are well known in the art (see for example Verma R et al., 1998, J. Immunol. Methods, 216, 165-181).

An antigen-binding fragment does not necessarily have an identical sequence to the parent antibody. In one embodiment, the antigen-binding fragment may have ≥70%, ≥80%, ≥85%, ≥90%, ≥95%, ≥96%, ≥97%, ≥98%, ≥99%, 100% sequence identity with the respective variable region domains of the parent antibody. Typically, the non-identical amino acids of a variable region are not in the CDRs.

Methods for screening antibodies of the invention that do not share 100% amino acid sequence identity with one of the antibodies disclosed herein, that possess the desired specificity, affinity and functional activity include enzyme linked immunosorbent assays, biacore, focus reduction neutralisation assay (FRNT), and other techniques known within the art or described herein.

An antibody of the invention is typically able to neutralise at least one biological activity of SAR-CoV-2 (a neutralising antibody), particularly to neutralise virus infectivity or block binding of the spike protein or a binding portion thereof, e.g. S1 or RBD, to the ACE2-receptor or a RBD-binding portion thereof. Blocking of the interaction between spike and ACE2-receptor can be total or partial. For example, an antibody of the invention may reduce spike-ACE2 formation by ≥40%, ≥50%, ≥60%, ≥70%, ≥80%, ≥90%, ≥95%, ≥99% or 100%. Blocking of spike-ACE2 formation can be determined or measured by any suitable means known in the art, or described herein, e.g. using an ELISA binding or neutralisation assay.

An antibody of the invention may have an affinity constant (K_{D}) value for the spike protein of SARS-CoV-2 of ≤5nM, ≤4nM, ≤3nM, ≤2nM, ≤1nM, ≤0.5nM, ≤0.4nM, ≤0.3nM, ≤0.2nM, ≤0.1nM or ≤0.05nM. The K_{D} values of some of the antibodies described herein are provided in Table 8.

The KD value can be measured by any suitable means known in the art, for example, by ELISA or Surface Plasmon Resonance (Biacore) at 25 °C.

Antibodies of the invention may have any combination of one or more of the properties described herein.

Antibodies of the invention may bind to the same epitope as, or compete for binding to SARS-CoV-2 spike protein with, any one of the antibodies described herein (i.e. in particular with antibodies having the CDR sequences or the heavy and light chain variable regions described above).

The skilled person is able to determine the binding site (epitope) of an antibody, whether an antibody binds to the same epitope as, or competes for binding with, an antibody described herein, using techniques known in the art.

For example, to determine if a test antibody binds to the same epitope as an antibody described herein (referred to a "reference antibody" in the following paragraphs), the reference antibody may be allowed to bind to a protein or peptide under saturating conditions. Next, the ability of a test antibody to bind to the protein or peptide is assessed. If the test antibody is able to bind to the protein or peptide following saturation binding with the reference antibody, it can be concluded that the test antibody binds to a different epitope than the reference antibody. On the other hand, if the test antibody is not able to bind to protein or peptide following saturation binding with the reference antibody, then the test antibody may bind to the same epitope as the epitope bound by the reference antibody of the invention.

To determine if an antibody competes for binding with a reference antibody, the above-described binding methodology is performed in two orientations. In a first orientation, the reference antibody is allowed to bind to a protein/peptide under saturating conditions followed by assessment of binding of the test antibody to the protein/peptide molecule. In a second orientation, the test antibody is allowed to bind to the protein/peptide under saturating conditions followed by assessment of binding of the reference antibody to the protein/peptide. If, in both orientations, only the first (saturating) antibody is capable of binding to the protein/peptide, then it is concluded that the test antibody and the reference antibody compete for binding to the protein/peptide. As will be appreciated by the skilled person, an antibody that competes for binding with a reference antibody may not necessarily bind to the identical epitope as the reference antibody, but may sterically block binding of the reference antibody by binding an overlapping or adjacent epitope.

Two antibodies bind to the same or overlapping epitope if each competitively inhibits (blocks) binding of the other to the antigen. Alternatively, two antibodies have the same epitope if essentially all amino acid mutations in the antigen that reduce or eliminate binding of one antibody reduce or eliminate binding of the other. Two antibodies have overlapping epitopes if some amino acid mutations that reduce or eliminate binding of one antibody reduce or eliminate binding of the other.

Additional routine experimentation (e.g., peptide mutation and binding analyses) can then be carried out to confirm whether the observed lack of binding of the test antibody is in fact due to binding to the same epitope as the reference antibody or if steric blocking (or another phenomenon) is responsible for the lack of observed binding. Experiments of this sort can be performed using ELISA, RIA, surface plasmon resonance, flow cytometry or any other quantitative or qualitative antibody-binding assay available in the art.

As well as sequences defined by percentage identity or number of sequence changes, the invention further provides an antibody defined by its ability to cross-compete with one of the specific antibodies set out herein. It may be that the antibody also has one of the recited levels of sequence identity or number of sequence changes as well.

Cross-competing antibodies can be identified using any suitable method in the art, for example by using competition ELISA or BIAcore assays where binding of the cross competing antibody to a particular epitope on the spike protein prevents the binding of an antibody of the invention or *vice versa.* In one embodiment, the antibody produces ≥50%, ≥60%, ≥70%, ≥80%, ≥90% or 100% reduction of binding of the specific antibody disclosed herein.

The antibodies described below in the Examples may be used as reference antibodies.

Other techniques that may be used to determine antibody epitopes include hydrogen/deuterium exchange, X-ray crystallography and peptide display libraries. A combination of these techniques may be used to determine the epitope of the test antibody.

The approaches described herein could be applied equally to other data, e.g. surface plasmon resonance or ELISA, and provides a general way of rapidly determining locations from highly redundant competition experiments.

### Combinations of Antibodies

In some cases, the invention relates to a combination of any two or more, e.g. any 2, 3, 4 or 5, of the antibodies of the invention described herein. The combination antibodies may be selected from any one of Tables 1 to 6, or have relevant amino acid sequences thereof as described herein, e.g. the six CDR sequences or the heavy and/or light chain variable sequences of these antibodies, and/or sequences sharing high sequence identity with the heavy and/or light chain variable sequences of these antibodies, as described herein. Alternatively, the combination antibodies may be selected from those listed in any two or more of these Tables, or have relevant sequences thereof. For example, in one embodiment, the combination of antibodies is selected from those listed in Tables 5 and 6. In another embodiment, the combination of antibodies includes one antibody only that has sequences of an antibody listed in Table 3, and one antibody only that has sequences of an antibody listed in Table 4. In some embodiments, these antibodies may be combined with other antibodies selected from Table 2, or selected from any of the other Tables. In another embodiment, the combination of antibody variable regions may be selected from those in Table 2 and/or Table 5, except that mAb391 is replaced by the sequences of any other antibody selected from Table 3; and/or mAb411 is replaced by the sequences of any other antibody selected from Table 4. Typically, when a combination of antibodies from Table 5 or Table 6 is selected, only one of Ab391(a) or Ab391(b) but not both, or one of Ab419(a) or Ab419(b) but not both, or one of Ab433(a) or Ab433(b) but not both, is selected.

In some cases, the combination of antibodies, or relevant sequences thereof, are selected from the antibodies/sequences listed in Table 5, or from the antibodies/sequences listed in Tables 5 and 6, or selected from antibodies Ab391, Ab371, Ab411, Ab378, Ab579, Ab580, Ab430, Ab433, Ab381, Ab379, Ab387, Ab564, Ab415 and Ab413, or selected from antibodies Ab391, Ab371, Ab411, Ab378, Ab579, Ab580, Ab430, Ab433, Ab381, Ab379, Ab387, Ab564, Ab415, Ab413, Ab431, Ab419 and Ab413 as described herein. In one embodiment, the combination of antibodies includes an antibody having a set of six CDRs having the sequences of SEQ ID NOs: 1170, 1171, 1172, 1174, 1175 and 1176, a heavy chain variable sequence comprising the sequence of SEQ ID NO: 1169 or 1177, a light chain variable sequence comprising the sequence of SEQ ID NO: 1173, a heavy chain variable sequence comprising at least 70%, at least 80%, at least 85%, at least 90%, at least 95%, at least 98%, or at least 99% sequence identity to SEQ ID NO: 1169 or 1177, and/or a light chain variable sequence comprising at least 70%, at least 80%, at least 85%, at least 90%, at least 95%, at least 98%, or at least 99% sequence identity to SEQ ID NO: 1173. In one embodiment, the combination of antibodies includes an antibody having a set of six CDRs having the sequences of SEQ ID NOs: 1186, 1187, 1188, 1190, 1191 and 1192, a heavy chain variable sequence comprising the sequence of SEQ ID NO: 1185, a light chain variable sequence comprising the sequence of SEQ ID NO: 1189, a heavy chain variable sequence comprising at least 70%, at least 80%, at least 85%, at least 90%, at least 95%, at least 98%, or at least 99% sequence identity to SEQ ID NO:1185, and/or a light chain variable sequence comprising at least 70%, at least 80%, at least 85%, at least 90%, at least 95%, at least 98%, or at least 99% sequence identity to SEQ ID NO: 1189. In one embodiment, the combination of antibodies includes an antibody having a set of six CDRs having the sequences of SEQ ID NOs: 1202, 1203, 1204, 1206, 1207 and 1208, a heavy chain variable sequence comprising the sequence of SEQ ID NO: 1201, a light chain variable sequence comprising the sequence of SEQ ID NO: 1205, a heavy chain variable sequence comprising at least 70%, at least 80%, at least 85%, at least 90%, at least 95%, at least 98%, or at least 99% sequence identity to SEQ ID NO: 1201, and/or a light chain variable sequence comprising at least 70%, at least 80%, at least 85%, at least 90%, at least 95%, at least 98%, or at least 99% sequence identity to SEQ ID NO: 1205. In one embodiment, the combination of antibodies includes an antibody having a set of six CDRs having the sequences of SEQ ID NOs: 1194, 1195, 1196, 1198, 1199 and 1200, a heavy chain variable sequence comprising the sequence of SEQ ID NO: 1193, a light chain variable sequence comprising the sequence of SEQ ID NO: 1197, a heavy chain variable sequence comprising at least 70%, at least 80%, at least 85%, at least 90%, at least 95%, at least 98%, or at least 99% sequence identity to SEQ ID NO: 1193, and/or a light chain variable sequence comprising at least 70%, at least 80%, at least 85%, at least 90%, at least 95%, at least 98%, or at least 99% sequence identity to SEQ ID NO: 1197.

In some cases the combination comprises any two, any three, or all four of these antibodies (defined by their CDRs or heavy or light chain variable sequences). For example, the combination may comprise or consist of antibodies having the CDR set or heavy and/or light chain variable sequences (or variants thereof as described herein) of antibodies Ab391 and Ab371; or antibodies Ab391 and Ab411; or antibodies Ab391 and Ab378; or antibodies Ab371 and Ab411; or antibodies Ab371 and Ab378; or antibodies Ab411 and Ab378; or antibodies Ab391, Ab371 and Ab411; or antibodies A391, Ab411 and Ab378, or antibodies A371, Ab411 and Ab378, or antibodies Ab391, A371, Ab411 and Ab378. In some embodiments the combination further comprises one or more, e.g. two or three or four, further antibodies having relevant sequences of antibodies listed in Table 6, or selected from Ab391, Ab371, Ab411, Ab378, Ab579, Ab580, Ab430, Ab433, Ab381, Ab379, Ab387, Ab564, Ab415 and Ab413. In some embodiments the combination further comprises one or more, e.g. two or three or four, further antibodies having relevant sequences of antibodies selected from those listed in any one or more of Tables 1 to 6.

In one embodiment the combination of antibodies comprises or consists of four antibodies having the following set of CDR sequences: (a) SEQ ID NOs: 1170, 1171, 1172, 1174, 1175 and 1176; (b) SEQ ID NOs: 1186, 1187, 1188, 1190, 1191 and 1192; (c) SEQ ID NOs: 1202, 1203, 1204, 1206, 1207 and 1208; and (d) SEQ ID NOs: 1194, 1195, 1196, 1198, 1199 and 1200; and/or having the following set of heavy chain variable regions: (a) SEQ NO: 1169 or 1177; (b) SEQ ID NO: 1185, (c) SEQ ID NO: 1201, and (d) SEQ ID NO: 1193; and/or having the following set of heavy chain variable regions: (a) a sequence having at least 70%, or at least 80%, 85%, 90%, 95%, 96%, 97%, 98% or 99% sequence identify to SEQ NO: 1169 or SEQ NO:1177; (b)) a sequence having at least 70%, or at least 80%, 85%, 90%, 95%, 96%, 97%, 98% or 99% sequence identify to SEQ ID NO: 1185, (c) a sequence having at least 70%, or at least 80%, 85%, 90%, 95%, 96%, 97%, 98% or 99% sequence identify to SEQ ID NO: 1201, and (d) a sequence having at least 70%, or at least 80%, 85%, 90%, 95%, 96%, 97%, 98% or 99% sequence identify to SEQ ID NO: 1193; and/or having the following set of light chain variable regions: (a) SEQ NO: 1173; (b) SEQ ID NO: 1189, (c) SEQ ID NO: 1205, and (d) SEQ ID NO: 1197; and/or having the following set of heavy chain variable regions: (a) a sequence having at least 70%, or at least 80%, 85%, 90%, 95%, 96%, 97%, 98% or 99% sequence identify to SEQ NO:1173; (b)) a sequence having at least 70%, or at least 80%, 85%, 90%, 95%, 96%, 97%, 98% or 99% sequence identify to SEQ ID NO: 1189, (c) a sequence having at least 70%, or at least 80%, 85%, 90%, 95%, 96%, 97%, 98% or 99% sequence identify to SEQ ID NO: 1205, and (d) a sequence having at least 70%, or at least 80%, 85%, 90%, 95%, 96%, 97%, 98% or 99% sequence identify to SEQ ID NO: 1197.

In any of these combinations, the antibody having sequences of Ab411 may in some cases be replaced with an antibody having relevant sequences as described herein of any one of antibodies Ab431, Ab419 or Ab413 as described herein.

In one embodiment, the combination of antibodies comprises or consists of those having the CDRs or heavy and/or light chain variable regions of any two, any three, any four or any five antibodies, each of which is shown in Figure 5 to be in a different bin to each other antibody in the selected combination.

In one embodiment, the combination of antibodies comprises or consists of those having one or more other functional characteristics described herein.

A combination of the antibodies of the invention may be useful as a therapeutic cocktail. Hence, the invention also provides a pharmaceutical composition comprising a combination of the antibodies of the invention, as explained further below.

A combination of the antibodies of the invention may be useful for diagnosis. Hence, the invention also provides a diagnostic kit comprising a combination of the antibodies of the invention. Also provided herein are methods of diagnosing a disease or complication associated with coronavirus infections in a subject, as explained further below.

### SARS-CoV-2 Variants

Where not otherwise specified herein, the SARS-CoV-2 strain referred to herein is SARS-CoV-2 (2019-nCoV), Wuhan-Hu-1 strain, having the spike protein amino acid sequence of SEQ ID NO: 1445 and variants described herein are defined relative to this strain or to any other strain having the same spike protein sequence as SARS-CoV-2 (2019-nCoV) (SEQ ID NO: 1445).

### Polynucleotides, vectors and host cells

The invention also provides one or more isolated polynucleotides (e.g. DNA or RNA) encoding the antibody of the invention. In some cases, the complete sequence encoding the complete antibody may be spread across more than one polynucleotide, but the polynucleotides combined are able to encode an antibody of the invention. For example, the polynucleotides may encode the heavy and/or light chain variable regions(s) of an antibody of the invention. The polynucleotides may encode the full heavy and/or light chain of an antibody of the invention. Typically, one polynucleotide would encode each of the heavy and light chains.

In particular embodiments, the polynucleotide(s) of the invention comprise or consist of the polynucleotide sequences of any one of SEQ ID Nos: 1446 to 1463, or SEQ IDNos: 1464 to 1481.

Polynucleotides which encode an antibody of the invention can be obtained by methods well known to those skilled in the art. For example, DNA sequences coding for part or all of the antibody heavy and light chains may be synthesised as desired from the corresponding amino acid sequences.

General methods by which the vectors may be constructed, transfection methods and culture methods are well known to those skilled in the art. In this respect, reference is made to "Current Protocols in Molecular Biology", 1999, F. M. Ausubel (ed), Wiley Interscience, New York and the Maniatis Manual produced by Cold Spring Harbor Publishing.

A polynucleotide of the invention may be provided in the form of an expression cassette, which includes control sequences operably linked to the inserted sequence, thus allowing for expression of the antibody of the invention *in vivo.* Hence, the invention also provides one or more expression cassettes encoding the one or more polynucleotides that encode an antibody of the invention. These expression cassettes, in turn, are typically provided within vectors (e.g. plasmids or recombinant viral vectors). Hence, in one embodiment, the invention provides a vector encoding an antibody of the invention. In another embodiment, the invention provides vectors which collectively encode an antibody of the invention. The vectors may be cloning vectors or expression vectors. A suitable vector may be any vector which is capable of carrying a sufficient amount of genetic information, and allowing expression of a polypeptide of the invention.

The polynucleotides, expression cassettes or vectors of the invention are introduced into a host cell, e.g. by transfection. Hence, the invention also provides a host cell comprising the one or more polynucleotides, expression cassettes or vectors of the invention. The polynucleotides, expression cassettes or vectors of the invention may be introduced transiently or permanently into the host cell, allowing expression of an antibody from the one or more polynucleotides, expression cassettes or vectors. Such host cells include transient, or preferably stable higher eukaryotic cell lines, such as mammalian cells or insect cells, lower eukaryotic cells, such as yeast, or prokaryotic cells, such as bacteria cells. Particular examples of cells include mammalian HEK293, such as HEK293F, HEK293T, HEK293S or HEK Expi293F, CHO, HeLa, NS0 and COS cells, or any other cell line used herein, such as the ones used in the Examples. Preferably the cell line selected will be one which is not only stable, but also allows for mature glycosylation.

The invention also provides a process for the production of an antibody of the invention, comprising culturing a host cell containing one or more vectors of the invention under conditions suitable for the expression of the antibody from the one or more polynucleotides of the invention, and isolating the antibody from said culture.

### Pharmaceutical composition

The invention provides a pharmaceutical composition comprising an antibody of the invention or a combination of the antibodies of the invention. The pharmaceutical compositions or kits described herein may comprise, in addition to one or more antibodies, a pharmaceutically acceptable excipient, carrier, diluent, buffer, stabiliser, preservative, adjuvant or other materials well known to those skilled in the art. Such materials are preferably non-toxic and preferably do not interfere with the pharmaceutical activity of the active ingredient(s). Examples of suitable aqueous carriers include water, buffered water and saline. The composition of the invention may include one or more pharmaceutically acceptable salts. A "pharmaceutically acceptable salt" refers to a salt that retains the desired biological activity of the parent compound and does not impart any undesired toxicological effects. Examples of such salts include acid addition salts and base addition salts. Other suitable pharmaceutically acceptable carriers include ethanol, polyols (such as glycerol, propylene glycol, polyethylene glycol, and the like), and suitable mixtures thereof, vegetable oils, such as olive oil, and injectable organic esters, such as ethyl oleate. In many cases, it will be desirable to include isotonic agents, for example, sugars, polyalcohols such as mannitol, sorbitol, or sodium chloride in the composition. The precise nature of the carrier or other material may depend on the route of administration.

Therapeutic compositions typically must be sterile and stable under the conditions of manufacture and storage. The composition can be formulated as a solution, microemulsion, liposome, or other ordered structure suitable to high drug concentration.

Pharmaceutical compositions of the invention may comprise additional therapeutic agents, for example an anti-viral agent. The anti-viral agent may bind to coronavirus and inhibit viral activity. Alternatively, the anti-viral agent may not bind directly to coronavirus but still affect viral activity/infectivity. The anti-viral agent could be a further anti-coronavirus antibody, which binds somewhere on SARS-CoV-2 other than the spike protein. Examples of an anti-viral agent useful with the invention include Remdesivir, Lopinavir, ritonavir, APN01, and Favilavir. In other cases the additional therapeutic agent may be an anti-inflammatory agent, such as a corticosteroid (e.g. Dexamethasone) or a non-steroidal anti-inflammatory drug (e.g. Tocilizumab), or the additional therapeutic agent may be an anti-coronavirus vaccine.

The pharmaceutical composition may be administered subcutaneously, intravenously, intradermally, intramuscularly, intranasally or orally.

Also within the scope of the invention are kits comprising antibodies or other compositions of the invention and instructions for use, for example in any method of the invention. The kit may further contain one or more additional reagents, such as an additional therapeutic or prophylactic agent as discussed herein.

### Methods and uses of the invention

The invention further relates to the use of the antibodies or combinations of antibodies or pharmaceutical compositions described herein, e.g. in a method for treatment of the human or animal body by therapy, or in a diagnostic method. The term "treatment" as used herein includes therapeutic and prophylactic treatment. Administration is typically in a "prophylactically effective amount" or a "therapeutically effective amount" (although prophylaxis may be considered therapy/treatment), this being sufficient to result in a clinical response or to show clinical benefit to the individual, e.g. an effective amount to prevent or delay an infection, or the onset of a disease or condition, to ameliorate one or more symptoms, to induce or prolong remission, or to delay relapse or recurrence. The methods and uses of the invention may comprise inhibiting the disease state (such as COVID-19), e.g. arresting its development; and/or relieving the disease state (such as COVID-19), e.g. causing regression of the disease state, e.g. until a desired endpoint is reached. The methods and uses of the invention may comprise the amelioration or the reduction of the severity, duration or frequency of a symptom of the disease state (such as COVID-19) (e.g. lessen the pain or discomfort), and such amelioration may or may not be directly affecting the disease. The symptoms or complications may be fever, headache, fatigue, loss of appetite, myalgia, diarrhoea, vomiting, abdominal pain, dehydration, respiratory tract infections, cytokine storm, acute respiratory distress syndrome (ARDS) sepsis, and/or organ failure (e.g. heart, kidneys, liver, GI, lungs). In some cases the methods and uses of the invention may lead to a decrease in the viral load of coronavirus (e.g. SARS-CoV-2), e.g. by ≥10%, ≥20%, ≥30%, ≥40%, ≥50%, ≥60%, ≥70%, ≥80%, ≥90%, or 100% compared to pre-treatment. Methods of determining viral load are well known in the art, e.g. infection assays. In some cases, the methods and uses of the invention may comprise preventing the coronavirus infection, or a disease or condition associated therewith, from occurring in a subject (e.g. humans), in particular, when such subject is predisposed to complications associated with coronavirus infection.

In some embodiments, the invention relates to methods of treating or preventing a coronavirus or beta-coronavirus (e.g. SARS-CoV-2) infection, or a disease or complication associated therewith, e.g. COVID-19. In one embodiment the invention relates to methods of treating or preventing infection by a virus that expresses a spike protein, an S1 domain, or an RBD having an epitope that is specifically bound by an antibody of the invention, or a disease or complication associated therewith. Any suitable SARS-CoV-2 strain may be treated, for example any strain described herein, such as in the Examples. Hence, in some cases the invention relates to methods of treating or preventing infection with SARS-CoV-2 (2019-nCoV), Wuhan-Hu-1 strain, or a mutant or variant thereof comprising any one or more of the following mutations in the reference spike protein amino acid sequence of SEQ ID NO: 1445: N501Y, K417N, E484K, and Y453F, or a strain comprising mutations N501Y, K417N and E484K. The RBD of the strain may have additional mutations, such as amino acid additions, substitutions or deletions. The RBD of the strain typically has at least 80%, or at least 85%, 90%, 92%, 95%, 96%, 97%, 98%, or 99% sequence of identity to SEQ ID NO: 1445, or to one of the specific variants of SEQ ID NO: 1445 described herein, e.g. having one of more of substitutions N501Y, K417N, E484K, and Y453F. Other mutations in the spike protein of known SARS-CoV-2 strains, relative to SEQ ID NO: 1445, that may be treated are: deletion of residues 69-70; deletions of residues 69-70 and substitution N501Y; deletion of residue 144; substitution E484K; substitution A570D; substitution D614G; substitution P681H; substitution T716I; substitution S982A; substitution D1118H; substitutions K417N, E484K, N501Y, L18F, D80G, D215G, R246I, D614G, and/or A701V and/or deletion of residue 242-244; deletion of residues 242-244 and substitution N501Y; deletion of residues 242-244 and substitution E484K; substitutions K417T, E484K, N501Y, L18F, T20N, P26S, D138Y, R190S, H655Y, and/or T1027I; substitutions L18F, T20N, P26S, D138Y, R190S, K417T, E484K, N501Y D614G, H655Y, T1027I, and/or V1176F; L425R and/or E484Q; Spike D614G, Spike E154K, Spike E484Q, Spike Spike L452R and Spike P681R; Spike D614G, Spike E154K, Spike E484Q, Spike E1072K, Spike K1073R, Spike L452R, Spike P681R; Spike D614G, Spike E154K, Spike E484Q, Spike G142D, Spike H1101D, Spike L452R, Spike P681R and Spike Q1071H; HV69-70 deletion, N501Y and D614G; H69del, V70del, Y144del, N501Y, A570D, D614G and P681H; H69del, V70del, Y144del, N501Y, A570D, D614G, P681H, T716I, S982A and D1118H; K417N, E484K and N501Y; K417N, E484K, N501Y and D614G; L18F, T20N, P26S, D138Y, R190S, K417T, E484K, N501Y, D614G, H655Y, T1027I and V1176F; ΔH69/ΔV70, Y453F and D614G; and substitution of lysine (K) at position 417 to asparagine (N) or threonine (T).

The SARS-CoV-2 strain may in some cases comprise any one or any combination of mutations described herein.

The method may comprise administering a therapeutically or prophylactically effective amount of an antibody, a combination of antibodies, or a pharmaceutical composition of the invention. In some cases, the method comprises identifying the presence of coronavirus in a sample, e.g. SARS-CoV-2, from the subject, for example using an antibody or method described herein, and treating the subject, for example using an antibody or method described herein. The invention also relates to an antibody, a combination of antibodies, or a pharmaceutical composition according to the invention for use in a method of a treating coronavirus (e.g. SARS-CoV-2) infection, as described herein, or a disease or complication associated therewith, e.g. COVID-19.

The invention also relates to a method of formulating a composition for treating coronavirus (e.g. SARS-CoV-2) infections, a disease or complication associated therewith, e.g. COVID-19, wherein said method comprises mixing an antibody, a combination of antibodies, or a pharmaceutical composition according to the invention with an acceptable carrier to prepare said composition.

The invention also relates to the use of an antibody, a combination of antibodies, or a pharmaceutical composition according to the invention for the manufacture of a medicament for treating a coronavirus (e.g. SARS-CoV-2) infection, as described herein, or a disease or complication associated therewith, e.g. COVID-19.

The invention also relates to identifying subjects that have a coronavirus infection, such as by SARS-CoV-2. For example, the methods and uses of the invention may involve identifying the presence of coronavirus (e.g. SARS-CoV-2), or a protein or a protein fragment thereof, in a sample. The detection may be carried out *in vitro* or *in vivo.* In certain embodiments, the invention relates to population screening. The invention relates to identifying any suitable SARS-CoV-2 strain, for example as described herein.

The methods and uses of the invention may include contacting a suitable sample from a subject, e.g. a blood, serum, mucus, saliva or other sample as described herein, with an antibody or a combination of antibodies of the invention, and detecting the presence or absence of an antibody-antigen complex, wherein the presence of the antibody-antigen complex indicates that the subject is infected with SARS-CoV-2.

Methods of determining the presence of an antibody-antigen complex are known in the art. For example, in vitro detection techniques include enzyme linked immunosorbent assays (ELISAs), Western blots, immunoprecipitations, and immunofluorescence. In vivo techniques include introducing into a subject a labelled anti-analyte protein antibody. For example, the antibody can be labelled with a radioactive marker whose presence and location in a subject can be detected by standard imaging techniques. The detection techniques may provide a qualitative or a quantitative readout depending on the assay employed.

Typically, the invention relates to methods and uses for a human subject in need thereof. However, non-human animals such as rats, rabbits, sheep, pigs, cows, cats, or dogs is also contemplated.

The subject may be asymptomatic or pre-symptomatic. The subject may be early, middle or late phase of the disease. The subject may be male or female. In certain embodiments, the subject is typically male. The subject may not have been infected with coronavirus, such as SARS-CoV-2. In embodiments of the invention relating to prevention or treatment, the subject may or may not have been diagnosed to be infected with coronavirus, such as SARS-CoV-2.

The invention relates to analysing samples from subjects. The sample may be tissues, cells and biological fluids isolated from a subject, as well as tissues, cells and fluids present within a subject. The sample may be blood and a fraction or component of blood including blood serum, blood plasma, or lymph. Typically, the sample is from a throat swab, nasal swab, or saliva. The antibody-antigen complex detection assays may be performed *in situ*, in which case the sample may be a tissue section (fixed and/or frozen) of the tissue obtained from biopsies or resections from a subject.

Antibodies, antibody combinations or pharmaceutical compositions may be administered subcutaneous, intravenous, intradermal, oral, intranasal, intramuscular or intracranial. Typically, administration is intravenous or subcutaneous.

The dose of an antibody may vary depending on the age and size of a subject, as well as on the disease, conditions and route of administration. Antibodies may be administered at a dose of about 0.1 mg/kg body weight to a dose of about 100 mg/kg body weight, such as at a dose of about 5 mg/kg to about 10 mg/kg. Antibodies may also be administered at a dose of about 50 mg/kg, 10 mg/kg or about 5 mg/kg body weight.

A combination of the invention may for example be administered at a dose of about 5 mg/kg to about 10 mg/kg for each antibody, or at a dose of about 10 mg/kg or about 5 mg/kg for each antibody. Alternatively, a combination may be administered at a dose of about 5 mg/kg total (e.g. a dose of 1.25 mg/kg of each antibody for a four antibody combination).

The antibody or combination of antibodies of the invention may be administered in a multiple dosage regimen. For example, the initial dose may be followed by administration of a second or plurality of subsequent doses. The second and subsequent doses may be separated by an appropriate time.

The antibodies of the invention are typically used in a single pharmaceutical composition/combination (co-formulated). However, the invention also generally includes the combined use of antibodies of the invention in separate preparations/compositions/ administrations. The invention also includes combined use of the antibodies with additional therapeutic agents, as described above.

Combined administration of the two or more agents and/or antibodies may be achieved in a number of different ways. In one embodiment, all the components may be administered together in a single composition. In another embodiment, each component may be administered separately as part of a combined therapy. For example, the antibody of the invention may be administered before, after or concurrently with another antibody of the invention. For example, the antibody of the invention may be administered before, after or concurrently with an anti-viral agent or an anti-inflammatory agent.

In embodiments where the invention relates to detecting the presence of coronavirus, e.g. SARS-CoV-2, or a protein or a protein fragment thereof, in a sample, the antibody may contain a detectable label. Methods of attaching a label to an antibody are known in the art, e.g. by direct labelling of the antibody by coupling (i.e., physically linking) a detectable substance to the antibody. Alternatively, the antibody may be indirectly labelled, e.g. by reactivity with another reagent that is directly labelled. Examples of indirect labelling include detection of a primary antibody using a fluorescently-labelled secondary antibody and end-labelling of a DNA probe with biotin such that it can be detected with fluorescently-labelled streptavidin.

The detection may further comprise: (i) an agent known to be useful for detecting the presence of coronavirus, e.g. SARS-CoV-2, or a protein or a protein fragment thereof, e.g. an antibody against other epitopes of the spike protein, or other proteins of the coronavirus, such as an anti-nucleocapsid antibody; and/or (ii) an agent known to not be capable of detecting the presence of coronavirus, e.g. SARS-CoV-2, or a protein or a protein fragment thereof, i.e. providing a negative control.

In certain embodiments, the antibody is modified to have increased stability. Suitable modifications are known in the art.

The invention also encompasses kits for detecting the presence of coronavirus, e.g. SARS-CoV-2, in a sample. For example, the kit may comprise: a labelled antibody or a combination of labelled antibodies of the invention; means for determining the amount of coronavirus, e.g. SARS-CoV-2, in a sample; and means for comparing the amount of coronavirus, e.g. SARS-CoV-2, in the sample with a standard. The labelled antibody or the combination of labelled antibodies can be packaged in a suitable container. The kit can further comprise instructions for using the kit to detect coronavirus, e.g. SARS-CoV-2, in a sample. The kit may further comprise other agents known to be useful for detecting the presence of coronavirus, as discussed above.

For example, the antibodies or combinations of antibodies of the invention may be used in a lateral flow test. Typically, the lateral flow test kit is a hand-held device with an absorbent pad, which based on a series of capillary beds, such as pieces of porous paper, microstructured polymer, or sintered polymer. The test runs the liquid sample along the surface of the pad with reactive molecules that show a visual positive or negative result. The test may further comprise using other agents known to be useful for detecting the presence of coronavirus, e.g. SARS-CoV-2, or a protein or a protein fragment thereof, as discussed above, such as anti- an anti-nucleocapsid antibody.

### Other

The present disclosure includes the combination of the aspects and features described except where such a combination is clearly impermissible or is stated to be expressly avoided. As used in this specification and the claims, the singular forms "a", "an", and "the" include plural referents unless the content clearly dictates otherwise. Thus, for example, reference to "an antibody" includes two or more such antibodies.

Section headings are used herein for convenience only and are not to be construed as limiting in any way.

When referring to "≥*x*" herein, this means equal to or greater than x. When referred to "≤*x*" herein, this means less than or equal to x.

For the purpose of this invention, in order to determine the percent identity of two sequences (such as two antibody sequences), the sequences are aligned for optimal comparison purposes (e.g., gaps can be introduced in a first sequence for optimal alignment with a second sequence). The amino acids at each position are then compared. When a position in the first sequence is occupied by the same amino acid as the corresponding position in the second sequence, then the amino acids are identical at that position. The percent identity between the two sequences is a function of the number of identical positions shared by the sequences (*i.e*., % identity = number of identical positions /total number of positions in the reference sequence x 100).

Typically the sequence comparison is carried out over the length of the reference sequence, for example, SEQ ID NO: 1445 herein. If the sequence is shorter than the reference sequence, the gaps or missing positions should be considered to be non-identical positions.

The skilled person is aware of different computer programs that are available to determine the homology or identity between two sequences using a mathematical algorithm. In an embodiment, the percent identity between two amino acid or nucleic acid sequences is determined using the Needleman and Wunsch (1970) algorithm which has been incorporated into the GAP program in the Accelrys GCG software package (available at http://www.accelrys.com/products/gcg/), using either a Blosum 62 matrix or a PAM250 matrix, and a gap weight of 16, 14, 12, 10, 8, 6, or 4 and a length weight of 1, 2, 3, 4, 5, or 6. Other examples of suitable programs are the BESTFIT program provided by the UWGCG Package (for example used on its default settings) (Devereux et al (1984) Nucleic Acids Research 12, 387-395) and the PILEUP and BLAST algorithms c (for example used on its default settings), for example as described in Altschul S. F. (1993) J Mol Evol 36:290-300; Altschul, S, F et al (1990) J Mol Biol 215:403-10. Software for performing BLAST analyses is publicly available through the National Center for Biotechnology Information (http://www.ncbi.nlm.nih.gov/).

The amino acid position numberings provided herein used the IMGT numbering system), although in some instances the KABAT numbering system or the absolute numbering of the amino acids based on the sequence listing may be used.

All publications, patents and patent applications cited herein, whether supra or infra, are hereby incorporated by reference in their entirety.

The following examples illustrate the invention.

### Examples

### Example 1

Receptor binding domain (RBD) and spike protein (S1) of SARS-CoV-2 were used for biopanning of libraries since these domains are crucial antibody binding domains. ScFv libraries generated from a panel of 50 naive human individuals, a panel of 20 autoimmune and Chagas disease patients, a panel from COVID-19 patients were used. All libraries were panned separately on SARS-CoV-2 targets. Each panning round the stringency was increased. Three panning rounds were performed in total. Pannings were performed on S1,S1,S1, on S1,S1,RBD and RBD,RBD,RBD proteins respectively. In total 1200 individual phages were analyzed by ELISA. This yielded 94 different individual antibody sequences from the ScFv libraries (Table 1a-c). 8 selected by ELISA on S1, 2 from Human patient, 3 from Human naive pool, 3 from COVID-19. 86 selected by ELISA on RBD. 16 from Human patient, 43 from Human naive pool, 27 from COVID-19 library.

Additional selections were carried out using the South African strain B.1.351 S1 protein. Each panning round the stringency was increased. Three panning rounds were performed in total. Pannings were performed on B.1.351 S1,S1,RBD proteins respectively. This yielded 22 different individual antibody sequences from the ScFv libraries (Table Id) of which 20 clones showed good reactivity on the SARS-CoV-2 as well as on the B.1.351 mutant. 5 selected by ELISA on B.1.351 S1 from Human patient, 15 from Human naive pool, 2 from COVID-19 (Figure 1).

Phages from Table 1a-c were tested on the SARS-CoV-2 S1 domain, on the SARS-CoV S1 domain and on the MERS S1 domain. This showed that only 3 antibody groups showed strong cross reactivity with the SARS-CoV domain, and 6 antibodies showed a low cross reactivity with SARS-CoV S1 domain. All cross reactive antibodies with SARS-CoV S1 domain originated from the Covid-19 library (Figure 2).

Gene analysis of the subset of selected clones on SARS-CoV-2 showed that 4 germline families VH1, VH3, VH5 and VH6 were used for the variable heavy of the human ScFv, VHs originating form 8 alleles from the VH1 family, originating from 7 alleles from the VH3 family, 1 allele from the VH5 family, and 1 allele for the VH6 family. For the light chains it showed that 8 germline genes were used, VL1, VL2, VL3, VL6, VL8, VK1, VK2, and VK3 were used for the ScFv, VLs originated from 18 alleles. There was a strong bias for the use of the Lamda light chains.

### Example 2

Neutralization studies were initially performed with the phage particles and the SARS-CoV-2 Surrogate Virus Neutralization Test Kit from GenScript (L00847)) (Figure 3).

Table 2 shows the antibodies chosen to work with as sFv-Fc constructs.

Of two clones a large number of variants were found, indicating that extensive mutations are allowed in combination with these CDR3s. See Tables 3 and 4. Ultimately only one clone was produced from each of these tables, namely clone mAb391 and clone mAb411.

### Example 3

After expression, the antibody scFv-Fc and VHH-Fc fusions were again tested for binding to RBD at 1 µg/mL (Figure 4) and neutralization at a concentration of 65 µg/ml. ScFv-Fc or VHH-Fc constructs that displayed neutralization efficiencies of 50% and higher were used for further analysis (Figure 5).

### Example 4

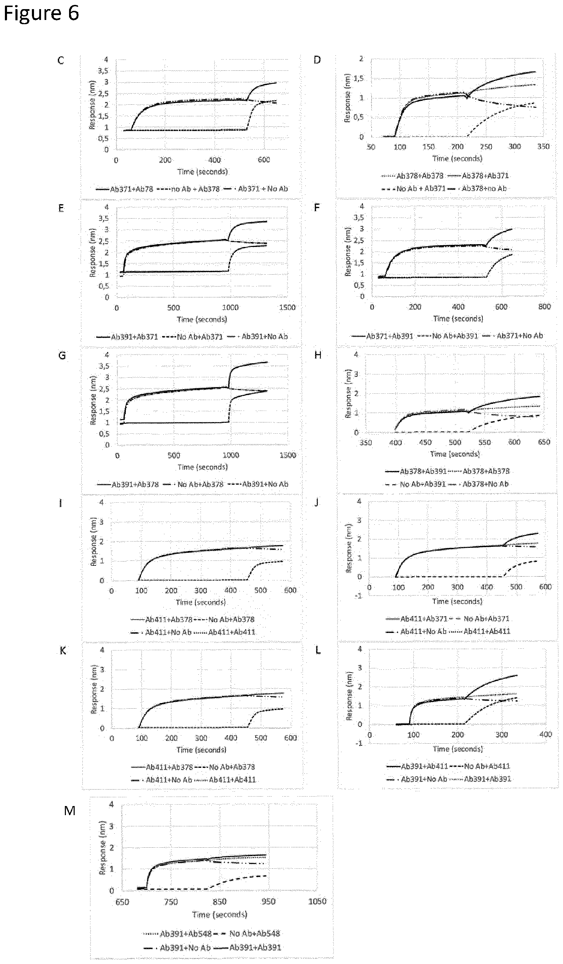

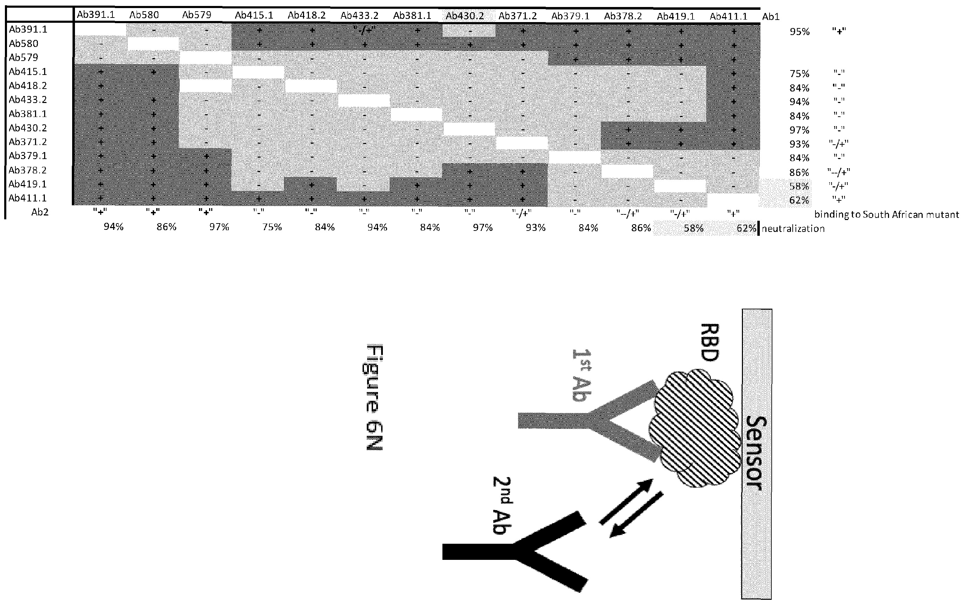

Clones that showed good neutralization were tested in an epitope binning experiment on the Octet96. From this effort clones were obtained that were able to bind the target simultaneously (Figure 6).

### Example 5

Binding studies were performed using various mutants that were available such as B.1.351 (south African), B.1.1.7 (British), (and PI (Brazilian variant)), as well as mutants of the RBD domain of AA 501, 417, 484, 437. This showed in an ELISA that a large number of clones lost binding to the South African mutant (Figure 7A, 7B), whereas against the other mutations only minor differences occurred.

A subset of clones that were still reactive with all mutants tested, especially the South African mutant, were selected. These were clone mAb391, mAb411 (or Ab 431, Ab419 or Ab413), mAb371, mAb378. Sequences of the antibodies selected for the cocktail are listed in Table 5. Of these 4 clones, clone mAb391, mAb411 showed no or minor differences in binding with the South African mutant and WT virus. Clone mAb371 showed a larger effect, and mAb378 showed the strongest effect of the mutant on binding the RBD domains. Sequences for additional antibodies are listed in Table 6. The ELISA results of Figure 7B are shown in Table 7.

### Example 6

Titration analysis with the different antibodies was performed and 50% neutralization titers (NT50) were determined as follows:. mAb391 at 0.078 ug/ml. mAb371 at 1 ug/ml, mAb378 at 0.5 and mAb411 at 0.146 (Figure 8A).

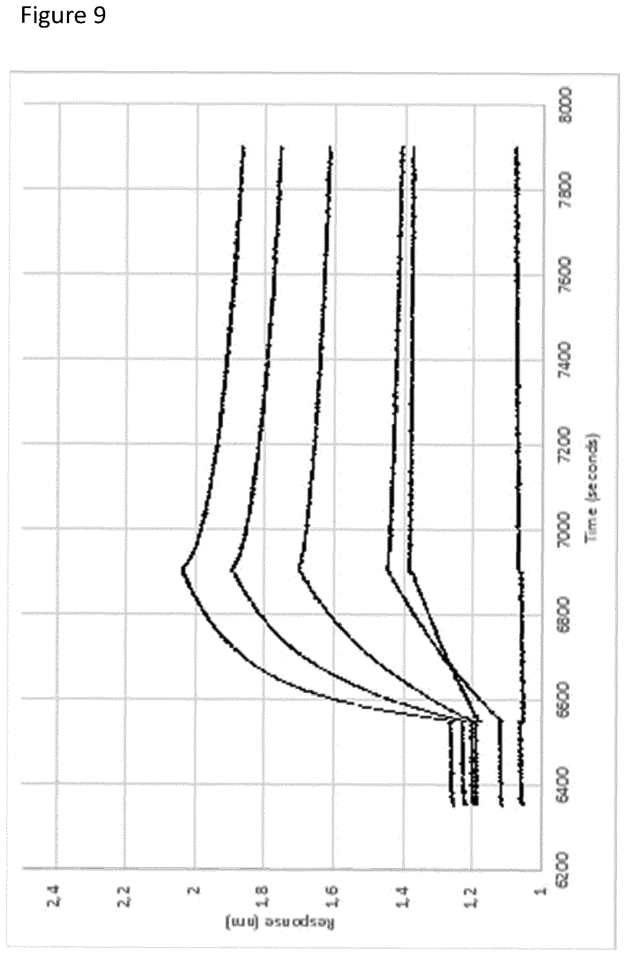

The kinetic binding affinities (*K*_{D}) of the 4 mAbs to the RBD were further determined with a biolayer interferometry (BLI) assay (Octet96) (see Figure 9 and Table 8).

### Example 7

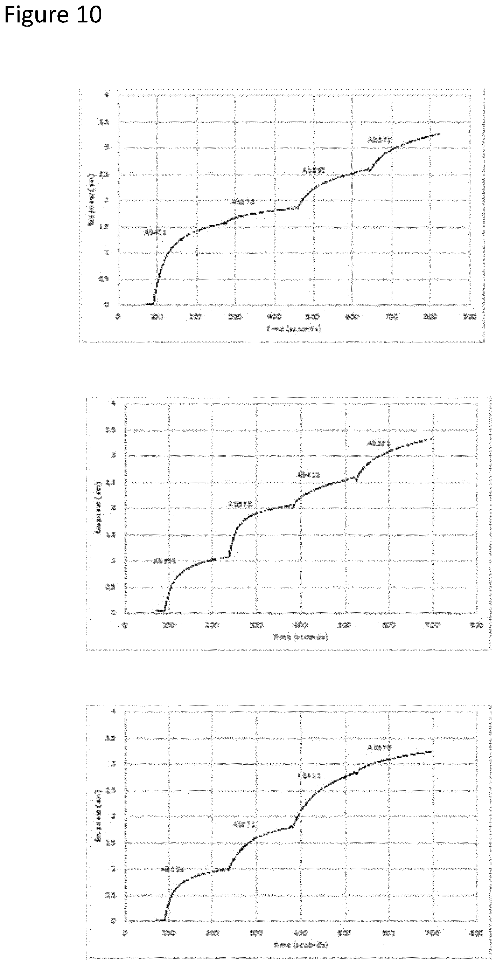

It was tested whether the four selected clones could still bind to their target when they were incubated with their target sequentially (Figure 10). It was observed during these studies that clone mAb411 and mAb378 did show interference. Especially when mAb411 was incubated prior to clone mAb378. It is anticipated that this will not have any effects since clone mAb411 is reactive with mutant aa 484 and clone mAb378 is less reactive with this mutant. **Example 8**

Clones were sub-cloned into antibody expression vectors to express human mAbs or VHH-antibodies. Neutralization experiments were performed once more using the Octet96 with human ACE2-biot and the British and South African mutants (Figure 8B).

### TABLES

**Table 1a**

| Panning polyclonal sample | Sequence Name | Neutralisation | ELISA MERS S1 result | ELISA SARS-CoV1 S1 result | ELISA SARS-CoV2 RBD result |
|---|---|---|---|---|---|
| **Patient** | 1F39SAD004_F4 | | | | 0.26 |
| **Corona** | 1F39SAD009_F7 | | 0.03 | 0.04 | 1.45 |
| **Naive** | 1F39SAD009_E3 | 0.28 | 0.03 | 0.03 | 0.73 |
| **Patient** | 1F39SAD004_F5 | 0.13 | 0.02 | 0.03 | 0.29 |
| **Naive** | 1F39SAD006_H11 | 0.31 | 0.03 | 0.03 | 0.45 |
| **Patient** | 1F39SAD004_E2 | 0.50 | 0.03 | 0.03 | 0.89 |
| **Corona** | 1E79SAD003_H2 | | | | 0.88 |
| **Corona** | 1F39SAD003_G3 | 0.44 | 0.04 | 0.21 | 0.78 |
| **Patient** | 1F39SALKXn_B10 | 0.01 | 0.03 | 0.03 | 0.44 |
| **Patient** | 1F58SAC004_A1 | | | | 0.17 |
| **Corona** | 1F39SAD006_F3 | 0.57 | 0.03 | 0.03 | 0.83 |
| **Naive** | 1F88SAA005_C6 | 0.53 | | | 0.60 |
| **Corona** | 1F39SAD009_D11 | | 0.04 | 0.03 | 0.55 |
| **Corona** | 1F39SAD003_F8 | | | | 0.24 |
| **Naive** | 1F88SAA005_E4 | 0.75 | | | 0.73 |
| **Patient** | 1E79SAD002_A11 | | | | 0.48 |
| **Patient** | 1E79SAD002_G3 | 0.87 | 0.03 | 0.03 | 0.68 |
| **Naive** | 1F39SAD004_B8 | 0.00 | 0.03 | 0.03 | 0.62 |
| **Corona** | 1F58SAC004_A3 | 0.30 | 0.03 | 0.02 | 0.36 |
| **Corona** | 1F58SAC004_E2 | | | | 0.24 |
| **Corona** | 1F39SAD003_A11 | 0.48 | 0.04 | 0.16 | 0.69 |
| **Naive** | 1E79SAD002_B1 | | | | 0.62 |
| **Corona** | 1E79SAD003_G1 | 0.09 | 0.03 | 0.03 | 1.25 |
| **Naive** | 1F39SAD009_E11 | 0.31 | 0.03 | 0.03 | 1.05 |
| **Patient** | 1E79SAD002_H10 | | | | 0.75 |
| **Naive** | 1F88SAA005 E6 | 0.25 | | | 0.45 |
| **Corona** | 1F39SAD003_D8 | 0.67 | 0.03 | 0.03 | 1.20 |
| **Corona** | 1F39SAD006_D1 | 0.27 | 0.03 | 0.03 | 0.65 |
| **Patient** | 1E79SAD002_C10 | | | | 0.71 |
| **Naive** | 1E79SAD002_A5 | | | | 0.25 |
| **Corona** | 1F88SAA005_E11 | 0.94 | | | 0.90 |
| **Naive** | 1F58SAC004_A2 | | | | 0.28 |
| **Naive** | 1F39SAD004_F12 | 0.36 | 0.03 | 0.03 | 0.73 |
| **Patient** | 1F39SAD004_A6 | 0.54 | 0.03 | 0.03 | 0.46 |
| **Naive** | 1F39SAD009_B12 | 0.48 | 0.03 | 0.03 | 0.69 |
| **Naive** | 1E79SAD002_B6 | | | | 0.31 |
| **Naive** | 1F39SAD006_G7 | 0.50 | 0.03 | 0.03 | 0.97 |
| **Patient** | 1E79SAD002_F11 | 0.27 | 0.03 | 0.03 | 0.93 |
| **Corona** | 1F39SAD003_A2 | 0.17 | 0.03 | 0.03 | 0.62 |
| **Naive** | 1F39SAD009_E7 | | 0.03 | 0.03 | 0.20 |
| **Corona** | 1E79SAD003_C4 | 0.03 | 0.03 | 0.03 | 0.55 |
| **Naive** | 1F39SAD006_G10 | 0.09 | 0.03 | 0.03 | 0.40 |
| **Naive** | 1F39SAD006_D9 | 0.17 | 0.03 | 0.03 | 0.78 |
| **Naive** | 1E79SAD002_D8 | 0.41 | 0.03 | 0.02 | 0.42 |
| **Naive** | 1F39SAD006_G12 | 0.33 | 0.02 | 0.02 | 0.50 |
| **Corona** | 1F58SAC004_D2 | 0.15 | 0.03 | 0.03 | 0.23 |
| **Naive** | 1E79SAD002_B7 | | | | 0.33 |
| **Naive** | 1F58SAC004_B6 | | | | 0.41 |
| **Naive** | 1F58SAC004_E5 | | | | 0.25 |
| **Naive** | 1E79SAD002_F6 | | | | 0.27 |
| **Naive** | 1F39SAD006_B10 | 0.06 | 0.03 | 0.03 | 0.55 |
| **Naive** | 1E79SAD002_E5 | 0.50 | 0.03 | 0.03 | 0.31 |
| **Naive** | 1F39SAD004_D8 | 0.19 | 0.03 | 0.03 | 0.93 |
| **Naive** | 1F39SAD009_C6 | 0.12 | 0.03 | 0.03 | 0.60 |
| **Naive** | 1E79SAD002_F5 | 0.21 | 0.02 | 0.03 | 0.46 |
| **Naive** | 1E79SAD002_H6 | | | | 0.75 |
| **Naive** | 1E79SAD002_A1 | 0.40 | 0.03 | 0.02 | 0.83 |
| **Naive** | 1E79SAD002_D7 | 0.32 | 0.03 | 0.03 | 0.35 |
| **Naive** | 1F39SAD009_E4 | 0.41 | 0.03 | 0.03 | 0.46 |
| **Naive** | 1E79SAD002_F4 | 0.22 | 0.03 | 0.02 | 0.32 |
| **Patient** | 1F39SAD009_F12 | | 0.04 | 0.03 | 0.38 |
| **Corona** | 1F39SAD003_E9 | 0.71 | 0.03 | 1.20 | 0.69 |
| **Corona** | 1F39SAD003_D6 | 0.82 | 0.04 | 2.24 | 1.25 |
| **Naive** | 1F39SAD006_G9 | 0.00 | 0.03 | 0.03 | 0.46 |
| **Patient** | 1E79SAD002_F9 | | | | 0.80 |
| **Naive** | 1F58SAC004_H5 | 0.28 | 0.03 | 0.02 | 0.49 |
| **Corona** | 1F39SAD003_B3 | 0.63 | 0.03 | 0.07 | 1.10 |
| **Naive** | 1F39SAD006_H8 | 0.32 | 0.03 | 0.03 | 0.37 |
| **Patient** | 1F39SAD001_A6 | 0.67 | 0.03 | 0.03 | 0.68 |
| **Corona** | 1F39SAD009_A4 | | 0.03 | 0.03 | 0.27 |
| **Naive** | 1F58SAC004_C5 | 0.30 | 0.03 | 0.03 | 0.52 |
| **Patient** | 1E79SAD002_G11 | 0.46 | 0.03 | 0.03 | 0.37 |
| **Corona** | 1F39SAD003_D3 | 0.26 | 0.03 | 0.33 | 0.44 |
| **Corona** | 1F39SAD006_D3 | 0.14 | 0.03 | 0.03 | 0.57 |
| **Naive** | 1F39SAD000_C11 | 0.28 | 0.03 | 0.03 | 0.37 |
| **Naive** | 1E79SAD002_G7 | 0.36 | 0.03 | 0.02 | 0.29 |
| **Naive** | 1E79SAD002_B8 | | | | 0.31 |
| **Naive** | 1F58SAC004_C2 | 0.27 | 0.03 | 0.03 | 0.46 |
| **Corona** | 1F39SAD003_C11 | 0.67 | 0.03 | 1.61 | 0.76 |
| **Corona** | 1F39SAD003_C9 | 0.37 | 0.03 | 0.04 | 0.43 |
| **Patient** | 1E79SAD002_G10 | 0.36 | 0.02 | 0.06 | 0.34 |
| **Corona** | 1F39SAD003_H2 | 0.66 | 0.03 | 0.04 | 0.60 |
| **Corona** | 1F39SAD003_F1 | 0.50 | 0.03 | 0.16 | 0.72 |
| **Corona** | 1F39SAD003_D12 | 0.28 | 0.04 | 0.12 | 0.34 |
| **Naive** | 1F39SAD006_C12 | 0.71 | 0.03 | 0.02 | 0.88 |
| **Naive** | 1E79SAD002_H2 | 0.39 | 0.03 | 0.03 | 0.35 |
| **Naive** | 1F58SAC004_A6 | | | | 0.22 |
| **Naive** | 1E79SAD002_F8 | 0.86 | 0.03 | 0.03 | 0.51 |
| **Corona** | 1F58SAC004_E3 | | | | 0.29 |
| **Corona** | 1F39SAD003_A4 | 0.47 | 0.04 | 0.22 | 1.16 |
| **Patient** | 1E79SAD002_E11 | | | | 0.50 |
| **Patient** | 1F39SAD004_F2 | 0.24 | 0.03 | 0.03 | 0.63 |
| **Corona** | 1F88SAA005_F9 | | 0.03 | 0.02 | 0.87 |
| **Naive** | 1F39SAD006_F10 | | | | 0.03 |
| **Naive** | 1F39SAD004_G10 | | | | 0.03 |
| **Patient** | 1F39SAD004_F4 | | | | 0.26 |
| **Corona** | 1F39SAD009_F7 | | 0.03 | 0.04 | 1.45 |
| **Naive** | 1F39SAD009_E3 | 0.28 | 0.03 | 0.03 | 0.73 |
| **Patient** | 1F39SAD004_F5 | 0.13 | 0.02 | 0.03 | 0.29 |
| **Naive** | 1F39SAD006_H11 | 0.31 | 0.03 | 0.03 | 0.45 |
| **Patient** | 1F39SAD004_E2 | 0.50 | 0.03 | 0.03 | 0.89 |
| **Corona** | 1E79SAD003_H2 | | | | 0.88 |
| **Corona** | 1F39SAD003_G3 | 0.44 | 0.04 | 0.21 | 0.78 |
| **Patient** | 1F39SAD001_B10 | 0.01 | 0.03 | 0.03 | 0.44 |
| **Patient** | 1F58SAC004_A1 | | | | 0.17 |
| **Corona** | 1F39SAD006_F3 | 0.57 | 0.03 | 0.03 | 0.83 |
| **Naive** | 1F88SAA005_C6 | 0.53 | | | 0.60 |
| **Corona** | 1F39SAD009_D11 | | 0.04 | 0.03 | 0.55 |
| **Corona** | 1F39SAD003_F8 | | | | 0.24 |
| **Naive** | 1F88SAA005_E4 | 0.75 | | | 0.73 |
| **Patient** | 1E79SAD002_A11 | | | | 0.48 |
| **Patient** | 1E79SAD002_G3 | 0.87 | 0.03 | 0.03 | 0.68 |
| **Naive** | 1F39SAD004 B8 | 0.00 | 0.03 | 0.03 | 0.62 |
| **Corona** | 1F58SAC004_A3 | 0.30 | 0.03 | 0.02 | 0.36 |
| **Corona** | 1F58SAC004_E2 | | | | 0.24 |
| **Corona** | 1F39SAD003_A11 | 0.48 | 0.04 | 0.16 | 0.69 |
| **Naive** | 1E79SAD002_B1 | | | | 0.62 |
| **Corona** | 1E79SAD003_G1 | 0.09 | 0.03 | 0.03 | 1.25 |
| **Naive** | 1F39SAD009_E11 | 0.31 | 0.03 | 0.03 | 1.05 |
| **Patient** | 1E79SAD002_H10 | | | | 0.75 |
| **Naive** | 1F88SAA005_E6 | 0.25 | | | 0.45 |
| **Corona** | 1F39SAD003_D8 | 0.67 | 0.03 | 0.03 | 1.20 |
| **Corona** | 1F39SAD006_D1 | 0.27 | 0.03 | 0.03 | 0.65 |
| **Patient** | 1E79SAD002_C10 | | | | 0.71 |
| **Naive** | 1E79SAD002_A5 | | | | 0.25 |
| **Corona** | 1F88SAA005_E11 | 0.94 | | | 0.90 |
| **Naive** | 1F58SAC004_A2 | | | | 0.28 |
| **Naive** | 1F39SAD004_F12 | 0.36 | 0.03 | 0.03 | 0.73 |
| **Patient** | 1F39SAD004_A6 | 0.54 | 0.03 | 0.03 | 0.46 |
| **Naive** | 1F39SAD009_B12 | 0.48 | 0.03 | 0.03 | 0.69 |
| **Naive** | 1E79SAD002_B6 | | | | 0.31 |
| **Naive** | 1F39SAD006_G7 | 0.50 | 0.03 | 0.03 | 0.97 |
| **Patient** | 1E79SAD002_F11 | 0.27 | 0.03 | 0.03 | 0.93 |
| **Corona** | 1F39SAD003_A2 | 0.17 | 0.03 | 0.03 | 0.62 |
| **Naive** | 1F39SAD009_E7 | | 0.03 | 0.03 | 0.20 |
| **Corona** | 1E79SAD003_C4 | 0.03 | 0.03 | 0.03 | 0.55 |
| **Naive** | 1F39SAD006_G10 | 0.09 | 0.03 | 0.03 | 0.40 |
| **Naive** | 1F39SAD006_D9 | 0.17 | 0.03 | 0.03 | 0.78 |
| **Naive** | 1E79SAD002_D8 | 0.41 | 0.03 | 0.02 | 0.42 |
| **Naive** | 1F39SAD006_G12 | 0.33 | 0.02 | 0.02 | 0.50 |
| **Corona** | 1F58SAC004_D2 | 0.15 | 0.03 | 0.03 | 0.23 |
| **Naive** | 1E79SAD002_B7 | | | | 0.33 |
| **Naive** | 1F58SAC004_B6 | | | | 0.41 |
| **Naive** | 1F58SAC004_E5 | | | | 0.25 |
| **Naive** | 1E79SAD002_F6 | | | | 0.27 |
| **Naive** | 1F39SAD006_B10 | 0.06 | 0.03 | 0.03 | 0.55 |
| **Naive** | 1E79SAD002_E5 | 0.50 | 0.03 | 0.03 | 0.31 |
| **Naive** | 1F39SAD004_D8 | 0.19 | 0.03 | 0.03 | 0.93 |
| **Naive** | 1F39SAD009_C6 | 0.12 | 0.03 | 0.03 | 0.60 |
| **Naive** | 1E79SAD002_F5 | 0.21 | 0.02 | 0.03 | 0.46 |
| **Naive** | 1E79SAD002_H6 | | | | 0.75 |
| **Naive** | 1E79SAD002_A1 | 0.40 | 0.03 | 0.02 | 0.83 |
| **Naive** | 1E79SAD002_D7 | 0.32 | 0.03 | 0.03 | 0.35 |
| **Naive** | 1F39SAD009_E4 | 0.41 | 0.03 | 0.03 | 0.46 |
| **Naive** | 1E79SAD002_F4 | 0.22 | 0.03 | 0.02 | 0.32 |
| **Patient** | 1F39SAD009_F12 | | 0.04 | 0.03 | 0.38 |
| **Corona** | 1F39SAD003_E9 | 0.71 | 0.03 | 1.20 | 0.69 |
| **Corona** | 1F39SAD003_D6 | 0.82 | 0.04 | 2.24 | 1.25 |
| **Naive** | 1F39SAD006_G9 | 0.00 | 0.03 | 0.03 | 0.46 |
| **Patient** | 1E79SAD002_F9 | | | | 0.80 |
| **Naive** | 1F58SAC004_H5 | 0.28 | 0.03 | 0.02 | 0.49 |
| **Corona** | 1F39SAD003_B3 | 0.63 | 0.03 | 0.07 | 1.10 |
| **Naive** | 1F39SAD006_H8 | 0.32 | 0.03 | 0.03 | 0.37 |
| **Patient** | 1F39SAD001_A6 | 0.67 | 0.03 | 0.03 | 0.68 |
| **Corona** | 1F39SAD009_A4 | | 0.03 | 0.03 | 0.27 |
| **Naive** | 1F58SAC004_C5 | 0.30 | 0.03 | 0.03 | 0.52 |
| **Patient** | 1E79SAD002_G11 | 0.46 | 0.03 | 0.03 | 0.37 |
| **Corona** | 1F39SAD003_D3 | 0.26 | 0.03 | 0.33 | 0.44 |
| **Corona** | 1F39SAD006_D3 | 0.14 | 0.03 | 0.03 | 0.57 |
| **Naive** | 1F39SAD000_C11 | 0.28 | 0.03 | 0.03 | 0.37 |
| **Naive** | 1E79SAD002_G7 | 0.36 | 0.03 | 0.02 | 0.29 |
| **Naive** | 1E79SAD002_B8 | | | | 0.31 |
| **Naive** | 1F58SAC004_C2 | 0.27 | 0.03 | 0.03 | 0.46 |
| **Corona** | 1F39SAD003_C11 | 0.67 | 0.03 | 1.61 | 0.76 |
| **Corona** | 1F39SAD003_C9 | 0.37 | 0.03 | 0.04 | 0.43 |
| **Patient** | 1E79SAD002_G10 | 0.36 | 0.02 | 0.06 | 0.34 |
| **Corona** | 1F39SAD003 H2 | 0.66 | 0.03 | 0.04 | 0.60 |
| **Corona** | 1F39SAD003_F1 | 0.50 | 0.03 | 0.16 | 0.72 |
| **Corona** | 1F39SAD003_D12 | 0.28 | 0.04 | 0.12 | 0.34 |
| **Naive** | 1F39SAD006_C12 | 0.71 | 0.03 | 0.02 | 0.88 |
| **Naive** | 1E79SAD002_H2 | 0.39 | 0.03 | 0.03 | 0.35 |
| **Naive** | 1F58SAC004_A6 | | | | 0.22 |
| **Naive** | 1E79SAD002_F8 | 0.86 | 0.03 | 0.03 | 0.51 |
| **Corona** | 1F58SAC004_E3 | | | | 0.29 |
| **Corona** | 1F39SAD003_A4 | 0.47 | 0.04 | 0.22 | 1.16 |
| **Patient** | 1E79SAD002_E11 | | | | 0.50 |
| **Patient** | 1F39SAD004_F2 | 0.24 | 0.03 | 0.03 | 0.63 |
| **Corona** | 1F88SAA005_F9 | | 0.03 | 0.02 | 0.87 |
| **Naive** | 1F39SAD006_F10 | | | | 0.03 |
| **Naive** | 1F39SAD004_G10 | | | | 0.03 |

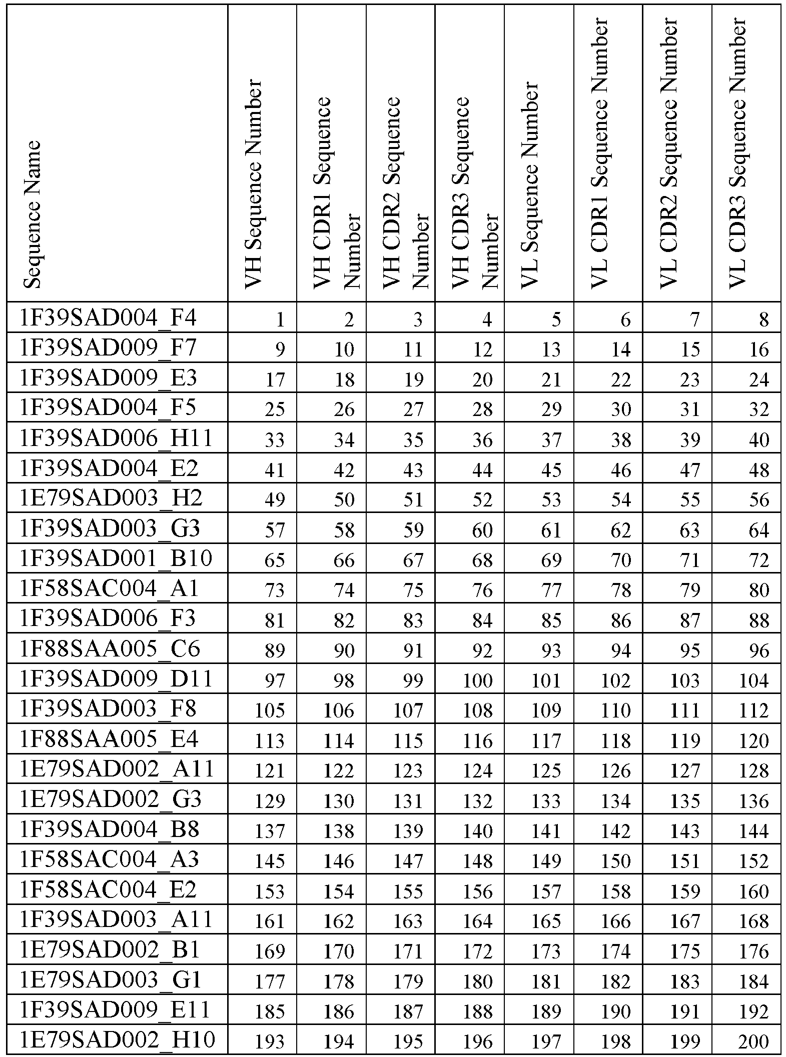

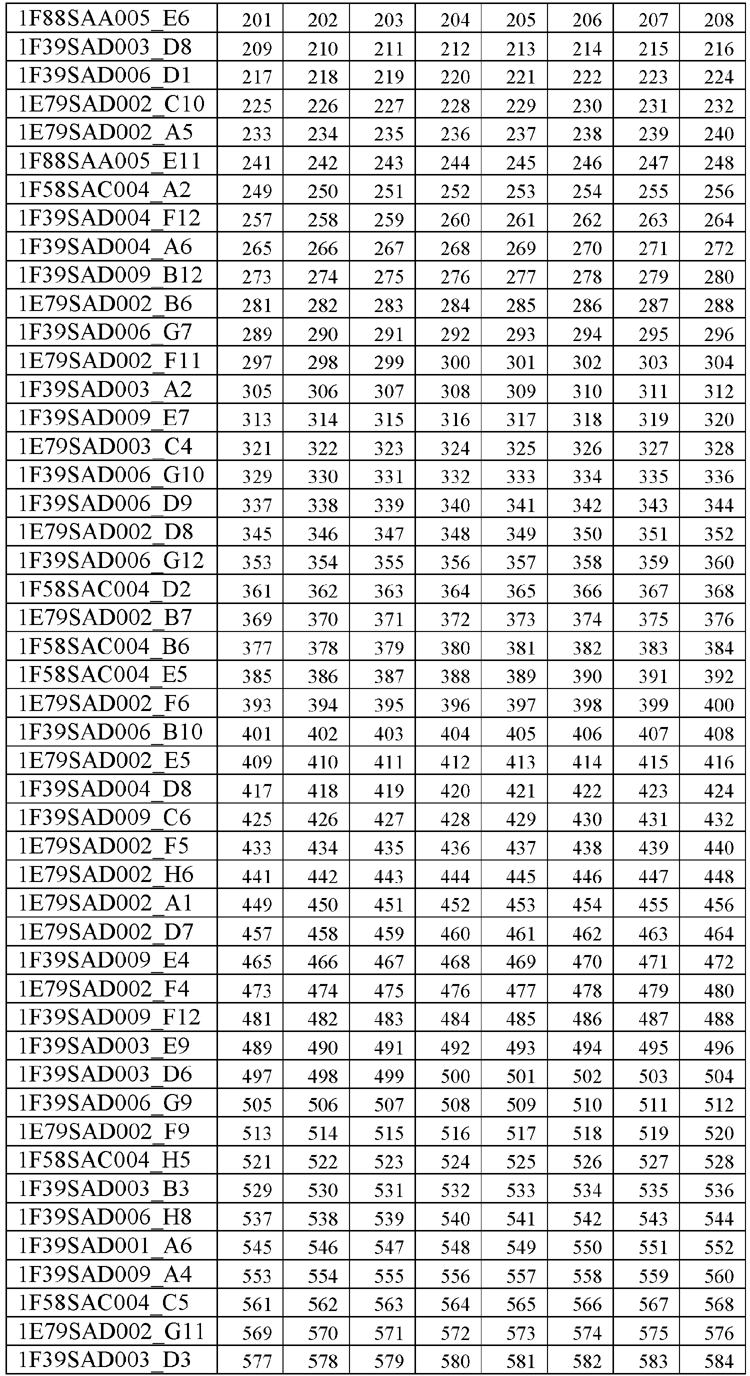

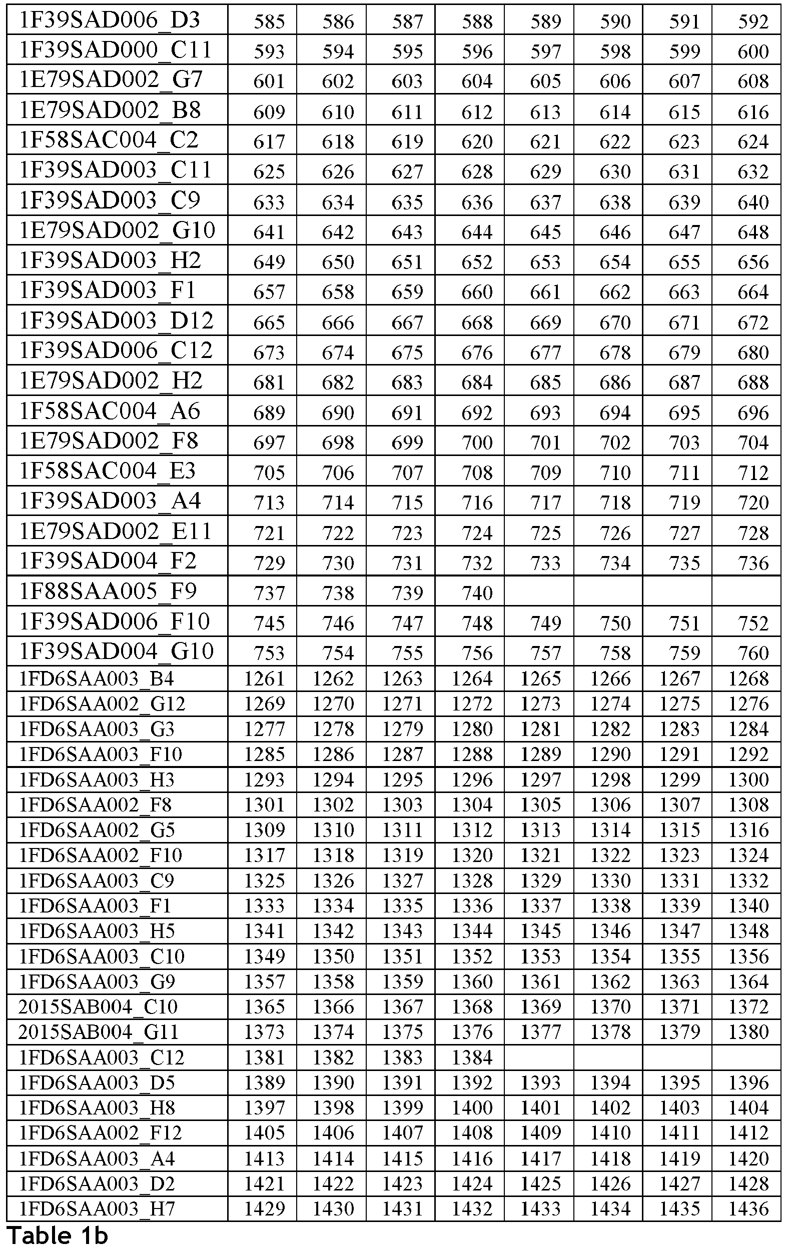

**Table 1c**

| Sequence Name | VH V-Germline | VH D-Germline | VH J-Germline | VL V-Germline | VL J-Germline |
|---|---|---|---|---|---|
| 1F39SAD004_F4 | GL_nt_setA_VH1-18_IGHV | GL_nt_setA_IGHD3-10 | GL_nt_setA_IGHJ1 | GL_nt_setA_VL3-21_IGLV | GL_nt_setA_IGLJ2 |
| 1F39SAD009_F7 | GL_nt_setA_VH6-1_IGHV | GL_nt_setA_IGHD2-8 | GL_nt_setA_IGHJ3 | GL_nt_setA_VL2-14_IGLV | GL_nt_setA_IGLJ1 |
| 1F39SAD009_E3 | GL_nt_setA_VH5-51_IGHV | GL_nt_setA_IGHD6-19 | GL_nt_setA_IGHJ3 | GL_nt_setA_VL1-44_IGLV | GL_nt_setA_IGLJ2 |
| 1F39SAD004_F5 | GL_nt_setA_VH1-69D_IGHV | GL_nt_setA_IGHD3-9 | GL_nt_setA_IGHJ6 | GL_nt_setA_VK2-28_IGKV | GL_nt_setA_IGKJ1 |
| 1F39SAD006_H11 | GL_nt_setA_VH3-30-5_IGHV | GL_nt_setA_IGHD2-21 | GL_nt_setA_IGHJ6 | GL_nt_setA_VL1-44_IGLV | GL_nt_setA_IGLJ7 |
| 1F39SAD004_E2 | GL_nt_setA_VH3-9_IGHV | GL_nt_setA_IGHD6-13 | GL_nt_setA_IGHJ6 | GL_nt_setA_VL3-21_IGLV | GL_nt_setA_IGLJ2 |
| 1E79SAD003_H2 | GL_nt_setA_VH1-2_IGHV | GL_nt_setA_IGHD3-16 | GL_nt_setA_IGHJ6 | GL_nt_setA_VL2-14_IGLV | GL_nt_setA_IGLJ1 |
| 1F39SAD003_G3 | GL_nt_setA_VH1-18_IGHV | GL_nt_setA_IGHD6-13 | GL_nt_setA_IGHJ4 | GL_nt_setA_VL3-1_IGLV | GL_nt_setA_IGLJ1 |
| 1F39SAD001_B10 | GL_nt_setA_VH3-30-5_IGHV | GL_nt_setA_IGHD6-25 | GL_nt_setA_IGHJ6 | GL_nt_setA_VK1-12_IGKV | GL_nt_setA_IGKJ4 |
| 1F58SAC004_A1 | GL_nt_setA_VH3-30-5_IGHV | GL_nt_setA_IGHD5-12 | GL_nt_setA_IGHJ3 | GL_nt_setA_VK3-11_IGKV | GL_nt_setA_IGKJ4 |
| 1F39SAD006_F3 | GL_nt_setA_VH3-33_IGHV | GL_nt_setA_IGHD5-12 | GL_nt_setA_IGHJ4 | GL_nt_setA_VL1-44_IGLV | GL_nt_setA_IGLJ7 |
| 1F88SAA005_C6 | GL_nt_setA_VH3-9_IGHV | GL_nt_setA_IGHD3-22 | GL_nt_setA_IGHJ4 | GL_nt_setA_VL1-44_IGLV | GL_nt_setA_IGLJ2 |
| 1F39SAD009_D11 | GL_nt_setA_VH1-2_IGHV | GL_nt_setA_IGHD2-8 | GL_nt_setA_IGHJ3 | GL_nt_setA_VK1-39_IGKV | GL_nt_setA_IGKJ2 |
| 1F39SAD003_F8 | GL_nt_setA_VH3-30-3_IGHV | GL_nt_setA_IGHD3-10 | GL_nt_setA_IGHJ6 | GL_nt_setA_VL3-1_IGLV | GL_nt_setA_IGLJ2 |
| 1F88SAA005_E4 | GL_nt_setA_VH1-18_IGHV | GL_nt_setA_IGHD4-17 | GL_nt_setA_IGHJ2 | GL_nt_setA_VL1-47_IGLV | GL_nt_setA_IGLJ1 |
| 1E79SAD002_A11 | GL_nt_setA_VH1-69D_IGHV | GL_nt_setA_IGHD5-12 | GL_nt_setA_IGHJ6 | GL_nt_setA_VL1-44_IGLV | GL_nt_setA_IGLJ2 |
| 1E79SAD002_G3 | GL_nt_setA_VH1-69D_IGHV | GL_nt_setA_IGHD5-12 | GL_nt_setA_IGHJ3 | GL_nt_setA_VL1-44_IGLV | GL_nt_setA_IGLJ2 |
| 1F39SAD004_B8 | GL_nt_setA_VH3-30-3_IGHV | GL_nt_setA_IGHD2-21 | GL_nt_setA_IGHJ3 | GL_nt_setA_VL3-1_IGLV | GL_nt_setA_IGLJ2 |
| 1F58SAC004_A3 | GL_nt_setA_VH5-51_IGHV | GL_nt_setA_IGHD1-26 | GL_nt_setA_IGHJ5 | GL_nt_setA_VL1-44_IGLV | GL_nt_setA_IGLJ2 |
| 1F58SAC004_E2 | GL_nt_setA_VH5-51_IGHV | GL_nt_setA_IGHD1-26 | GL_nt_setA_IGHJ5 | GL_nt_setA_VL1-44_IGLV | GL_nt_setA_IGLJ2 |
| 1F39SAD003_A11 | GL_nt_setA VH1-3_IGHV | GL_nt_setA_IGHD1-26 | GL_nt_setA_IGHJ5 | GL_nt_setA_VL3-1_IGLV | GL_nt_setA_IGLJ7 |
| 1E79SAD002_B1 | GL_nt_setA_VH1-69D_IGHV | GL_nt_setA_IGHD4-4 | GL_nt_setA_IGHJ4 | GL_nt_setA_VL6-57_IGLV | GL_nt_setA_IGLJ2 |
| 1E79SAD003_G1 | GL_nt_setA_VH1-2_IGHV | GL_nt_setA_IGHD3-16 | GL_nt_setA_IGHJ6 | GL_nt_setA_VL2-14_IGLV | GL_nt_setA_IGLJ1 |
| 1F39SAD009_E11 | GL_nt_setA_VH1-8_IGHV | GL_nt_setA_IGHD3-9 | GL_nt_setA_IGHJ4 | GL_nt_setA_VL1-44_IGLV | GL_nt_setA_IGLJ2 |
| 1E79SAD002_H10 | GL_nt_setA_VH3-30-5_IGHV | GL_nt_setA_IGHD6-19 | GL_nt_setA_IGHJ5 | GL_nt_setA_VL3-21_IGLV | GL_nt_setA_IGLJ2 |
| 1F88SAA005_E6 | GL_nt_setA_VH3-30-5_IGHV | GL_nt_setA_IGHD6-13 | GL_nt_setA_IGHJ1 | GL_nt_setA_VL2-8_IGLV | GL_nt_setA_IGLJ2 |
| 1F39SAD003_D8 | GL_nt_setA_VH1-18_IGHV | GL_nt_setA_IGHD2-15 | GL_nt_setA_IGHJ5 | GL_nt_setA_VL2-23_IGLV | GL_nt_setA_IGLJ2 |
| 1F39SAD006_DI | GL_nt_setA_VH3-53_IGHV | GL_nt_setA_IGHD3-10 | GL_nt_setA_IGHJ4 | GL_nt_setA_VL1-40_IGLV | GL_nt_setA_IGLJ1 |
| 1E79SAD002_CI0 | GL_nt_setA_VH3-11_IGHV | GL_nt_setA_IGHD2-2 | GL_nt_setA_IGHJ3 | GL_nt_setA_VL1-51_IGLV | GL_nt_setA_IGLJ2 |
| 1E79SAD002_A5 | GL_nt_setA_VH1-2_IGHV | GL_nt_setA_IGHD2-15 | GL_nt_setA_IGHJ4 | GL_nt_setA_VL8-61_IGLV | GL_nt_setA_IGLJ2 |
| 1F88SAA005_E11 | GL_nt_setA_VH1-2_IGHV | GL_nt_setA_IGHD3-22 | GL_nt_setA_IGHJ5 | GL_nt_setA_VL1-44_IGLV | GL_nt_setA_IGLJ2 |
| 1F58SAC004_A2 | GL_nt_setA_VH1-8_IGHV | GL_nt_setA_IGHD4-17 | GL_nt_setA_IGHJ5 | GL_nt_setA_VK3-11_IGKV | GL_nt_setA_IGKJ4 |
| 1F39SAD004_F12 | GL_nt_setA_VH3-30-3_IGHV | GL_nt_setA_IGHD1-7 | GL_nt_setA_IGHJ3 | GL_nt_setA_VL1-44_IGLV | GL_nt_setA_IGLJ2 |
| 1F39SAD004_A6 | GL_nt_setA_VH1-69D_IGHV | GL_nt_setA_IGHD6-13 | GL_nt_setA_IGHJ5 | GL_nt_setA_VL3-21_IGLV | GL_nt_setA_IGLJ2 |
| 1F39SAD009_B12 | GL_nt_setA_VH5-51_IGHV | GL_nt_setA_IGHD5-12 | GL_nt_setA_IGHJ3 | GL_nt_setA_VL1-44_IGLV | GL_nt_setA_IGLJ2 |
| 1E79SAD002_B6 | GL_nt_setA_VH5-51_IGHV | GL_nt_setA_IGHD5-12 | GL_nt_setA_IGHJ3 | GL_nt_setA_VL1-44 _IGLV | GL_nt_setA_IGLJ2 |
| 1F39SAD006_G7 | GL_nt_setA_VH1-18_IGHV | GL_nt_setA_IGHD4-17 | GL_nt_setA_IGHJ4 | GL_nt_setA_VL1-44_IGLV | GL_nt_setA_IGLJ2 |
| 1E79SAD002_F11 | GL_nt_setA_VH1-18_IGHV | GL_nt_setA_IGHD3-10 | GL_nt_setA_IGHJ1 | GL_nt_setA_VL3-21_IGLV | GL_nt_setA_IGLJ2 |
| 1F39SAD003_A2 | GL_nt_setA_VH6-1_IGHV | GL_nt_setA_IGHD3-16 | GL_nt_setA_IGHJ3 | GL_nt_seta_VL2-14_IGLV | GL_nt_setA_IGLJ2 |
| 1F39SAD009_E7 | GL_nt_setA_VH1-69D_IGHV | GL_nt_setA_IGHD2-2 | GL_nt_setA_IGHJ6 | GL_nt_setA_VL3-1_IGLV | GL_nt_setA_IGLJ2 |
| 1E79SAD003_C4 | GL_nt_setA_VH5-51_IGHV | GL_nt_setA_IGHD2-21 | GL_nt_setA_IGHJ3 | GL_nt_setA_VL1-44_IGLV | GL_nt_setA_IGLJ1 |
| 1F39SAD006_G10 | GL_nt_setA_VH5-51_IGHV | GL_nt_setA_IGHD1-26 | GL_nt_setA_IGHJ6 | GL_nt_setA_VL1-44_IGLV | GL_nt_setA_IGLJ2 |
| 1F39SAD006_D9 | GL_nt_setA_VH5-51_IGHV | GL_nt_setA_IGHD4-17 | GL_nt_setA_IGHJ5 | GL_nt_setA_VL1-44_IGLV | GL_nt_setA_IGLJ2 |
| 1E79SAD002_D8 | GL_nt_setA_VH5-51_IGHV | GL_nt_setA_IGHD3-16 | GL_nt_setA_IGHJ3 | GL_nt_setA_VL1-44_IGLV | GL_nt_setA_IGLJ1 |
| 1F39SAD006_G12 | GL_nt_setA_VH5-51_IGHV | GL_nt_setA_IGHD5-12 | GL_nt_setA_IGHJ4 | GL_nt_setA_VL1-44_IGLV | GL_nt_setA_IGLJ2 |
| 1F58SAC004_D2 | GL_nt_setA_VH5-51_IGHV | GL_nt_setA_IGHD3-10 | GL_nt_setA_IGHJ4 | GL_nt_setA_VL1-44_IGLV | GL_nt_setA_IGLJ2 |
| 1E79SAD002_B7 | GL_nt_setA_VH5-51_IGHV | GL_nt_setA_IGHD6-13 | GL_nt_setA_IGHJ5 | GL_nt_setA_VL1-47_IGLV | GL_nt_setA_IGLJ2 |
| 1F58SAC004_B6 | GL_nt_setA_VH5-51_IGHV | GL_nt_setA_IGHD3-10 | GL_nt_setA_IGHJ3 | GL_nt_setA_VL1-47_IGLV | GL_nt_setA_IGLJ2 |
| 1F58SAC004_E5 | GL_nt_setA_VH5-51_IGHV | GL_nt_setA_IGHD4-17 | GL_nt_setA_IGHJ5 | GL_nt_setA_VL1-44_IGLV | GL_nt_setA_IGLJ2 |
| 1E79SAD002_F6 | GL_nt_setA_VH5-51_IGHV | GL_nt_setA_IGHD1-1 | GL_nt_setA_IGHJ6 | GL_nt_setA_VL1-44_IGLV | GL_nt_setA_IGLJ2 |
| 1F39SAD006_B10 | GL_nt_setA_VH5-51_IGHV | GL_nt_setA_IGHD5-12 | GL_nt_setA_IGHJ4 | GL_nt_setA_VL1-44_IGLV | GL_nt_setA_IGLJ2 |
| 1E79SAD002_E5 | GL_nt_setA_VH5-51_IGHV | GL_nt_setA_IGHD3-3 | GL_nt_setA_IGHJ3 | GL_nt_setA_VL1-44_IGLV | GL_nt_setA_IGLJ2 |
| 1F39SAD004_D8 | GL_nt_setA_VH5-51_IGHV | GL_nt_setA_IGHD3-3 | GL_nt_setA_IGHJ3 | GL_nt_setA_VL1-44_IGLV | GL_nt_setA_IGLJ2 |
| 1F39SAD009_C6 | GL_nt_setA_VH5-51_IGHV | GL_nt_setA_IGHD5-12 | GL_nt_setA_IGHJ4 | GL_nt_setA_VL1-44_IGLV | GL_nt_setA_IGLJ2 |
| 1E79SAD002_F5 | GL_nt_setA_VH5-51_IGHV | GL_nt_setA_IGHD5-18 | GL_nt_setA_IGHJ4 | GL_nt_setA_VL1-44_IGLV | GL_nt_setA_IGLJ1 |
| 1E79SAD002_H6 | GL_nt_setA_VH5-51_IGHV | GL_nt_setA_IGHD3-10 | GL_nt_setA_IGHJ4 | GL_nt_setA_VL1-44_IGLV | GL_nt_setA_IGLJ1 |
| 1E79SAD002_A1 | GL_nt_setA_VH5-51_IGHV | GL_nt_setA_IGHD5-12 | GL_nt_setA_IGHJ4 | GL_nt_setA_VL1-44_IGLV | GL_nt_setA_IGLJ1 |
| 1E79SAD002_D7 | GL_nt_setA_VH5-51_IGHV | GL_nt_setA_IGHD5-12 | GL_nt_setA_IGHJ4 | GL_nt_setA_VL1-44_IGLV | GL_nt_setA_IGLJ2 |
| 1F39SAD009 E4 | GL_nt_setA_VH5-51_IGHV | GL_nt_setA_IGHD5-12 | GL_nt_setA_IGHJ3 | GL_nt_setA_VL1-44_IGLV | GL_nt_setA_IGLJ2 |
| 1E79SAD002_F4 | GL_nt_setA_VH5-51_IGHV | GL_nt_setA_IGHD3-3 | GL_nt_setA_IGHJ3 | GL_nt_setA_VL1-44_IGLV | GL_nt_setA_IGLJ2 |
| 1F39SAD009_F12 | GL_nt_setA_VH1-69D_IGHV | GL_nt_setA_IGHD6-19 | GL_nt_setA_IGHJ3 | GL_nt_setA_VL2-14_IGLV | GL_nt_setA_IGLJ1 |
| 1F39SAD003_E9 | GL_nt_setA_VH1-46_IGHV | GL_nt_setA_IGHD3-22 | GL_nt_setA_IGHJ3 | GL_nt_setA_VL3-21_IGLV | GL_nt_setA_IGLJ2 |
| 1F39SAD003_D6 | GL_nt_setA_VH1-46_IGHV | GL_nt_setA_IGHD3-22 | GL_nt_setA_IGHJ3 | GL_nt_setA_VL3-21_IGLV | GL_nt_setA_IGLJ1 |
| 1F39SAD006_G9 | GL_nt_setA_VH1-18_IGHV | GL_nt_setA_IGHD5-12 | GL_nt_setA_IGHJ6 | GL_nt_setA_VL2-23_IGLV | GL_nt_setA_IGLJ2 |
| 1E79SAD002_F9 | GL_nt_setA_VH1-45_IGHV | GL_nt_setA_IGHD3-16 | GL_nt_setA_IGHJ3 | GL_nt_setA_VL1-44_IGLV | GL_nt_setA_IGLJ1 |
| 1F58SAC004_H5 | GL_nt_setA_VH5-51_IGHV | GL_nt_setA_IGHD7-27 | GL_nt_setA_IGHJ3 | GL_nt_setA_VL1-44_IGLV | GL_nt_setA_IGLJ2 |
| 1F39SAD003_B3 | GL_nt_setA_VH5-51_IGHV | GL_nt_setA_IGHD1-7 | GL_nt_setA_IGHJ5 | GL_nt_setA_VL1-44_IGLV | GL_nt_setA_IGLJ1 |
| 1F39SAD006_H8 | GL_nt_setA_VH5-51_IGHV | GL_nt_setA_IGHD7-27 | GL_nt_setA_IGHJ6 | GL_nt_setA_VL1-44_IGLV | GL_nt_setA_IGLJ2 |
| 1F39SAD001_A6 | GL_nt_setA_VH1-46_IGHV | GL_nt_setA_IGHD4-4 | GL_nt_setA_IGHJ4 | GL_nt_setA_VL6-57_IGLV | GL_nt_setA_IGLJ2 |
| 1F39SAD009_A4 | GL_nt_setA_VH5-51_IGHV | GL_nt_setA_IGHD1-26 | GL_nt_setA_IGHJ5 | GL_nt_setA_VL1-44_IGLV | GL_nt_setA_IGLJ2 |
| 1F58SAC004_C5 | GL_nt_setA_VH5-51_IGHV | GL_nt_setA_IGHD2-15 | GL_nt_setA_IGHJ5 | GL_nt_setA_VL1-44_IGLV | GL_nt_setA_IGLJ7 |
| 1E79SAD002_G11 | GL_nt_setA_VH3-33_IGHV | GL_nt_setA_IGHD6-6 | GL_nt_setA_IGHJ3 | GL_nt_setA_VL3-21_IGLV | GL_nt_setA_IGLJ7 |
| 1F39SAD003_D3 | GL_nt_setA_VH1-46_IGHV | GL_nt_setA_IGHD3-22 | GL_nt_setA_IGHJ3 | GL_nt_setA_VL3-1_IGLV | GL_nt_setA_IGLJ1 |
| 1F39SAD006_D3 | GL_nt_setA_VH5-51_IGHV | GL_nt_setA_IGHD6-19 | GL_nt_setA_IGHJ5 | GL_nt_setA_VL1-44_IGLV | GL_nt_setA_IGLJ1 |
| 1F39SAD000_C11 | GL_nt_setA_VH5-51_IGHV | GL_nt_setA_IGHD3-16 | GL_nt_setA_IGHJ5 | GL_nt_setA_VL1-44_IGLV | GL_nt_setA_IGLJ2 |
| 1E79SAD002_G7 | GL_nt_setA_VH5-51_IGHV | GL_nt_setA_IGHD3-3 | GL_nt_setA_IGHJ5 | GL_nt_setA_VL1-44_IGLV | GL_nt_setA_IGLJ2 |
| 1E79SAD002_B8 | GL_nt_setA_VH5-51_IGHV | GL_nt_setA_IGHD5-12 | GL_nt_setA_IGHJ3 | GL_nt_setA_VL1-44_IGLV | GL_nt_setA_IGLJ2 |
| 1F58SAC004_C2 | GL_nt_setA_VH1-69D_IGHV | GL_nt_setA_IGHD6-19 | GL_nt_setA_IGHJ3 | GL_nt_setA_VL1-47_IGLV | GL_nt_setA_IGLJ2 |
| 1F39SAD003_C11 | GL_nt_setA_VH1-24_IGHV | GL_nt_setA_IGHD5-18 | GL_nt_setA_IGHJ4 | GL_nt_setA_VL2-14_IGLV | GL_nt_setA_IGLJ2 |
| 1F39SAD003_C9 | GL_nt_setA_VH1-24_IGHV | GL_nt_setA_IGHD5-18 | GL_nt_setA_IGHJ4 | GL_nt_setA_VK3-20_IGKV | GL_nt_setA_IGKJ1 |
| 1E79SAD002_G10 | GL_nt_setA_VH1-69D_IGHV | GL_nt_setA_IGHD5-18 | GL_nt_setA_IGHJ3 | GL_nt_setA_VK1-39_IGKV | GL_nt_setA_IGKJ2 |
| 1F39SAD003_H2 | GL_nt_setA_VH1-2_IGHV | GL_nt_setA_IGHD5-18 | GL_nt_setA_IGHJ1 | GL_nt_setA_VL6-57_IGLV | GL_nt_setA_IGLJ2 |
| 1F39SAD003_F1 | GL_nt_setA_VH1-24_IGHV | GL_nt_setA_IGHD6-13 | GL_nt_setA_IGHJ5 | GL_nt_setA_VL2-8_IGLV | GL_nt_setA_IGLJ1 |
| 1F39SAD003_D12 | GL_nt_setA_VH1-24_IGHV | GL_nt_setA_IGHD3-10 | GL_nt_setA_IGHJ5 | GL_nt_setA_VL1-51_IGLV | GL_nt_setA_IGLJ2 |
| 1F39SAD006_C12 | GL_nt_setA_VH1-2_IGHV | GL_nt_setA_IGHD6-19 | GL_nt_setA_IGHJ4 | GL_nt_setA_VL1-44_IGLV | GL_nt_setA_IGLJ2 |
| 1E79SAD002_H2 | GL_nt_setA_VH5-51_IGHV | GL_nt_setA_IGHD5-18 | GL_nt_setA_IGHJ4 | GL_nt_setA_VL1-44_IGLV | GL_nt_setA_IGLJ2 |
| 1F58SAC004_A6 | GL_nt_setA_VH3-30-5_IGHV | GL_nt_setA_IGHD6-19 | GL_nt_setA_IGHJ3 | GL_nt_setA_VL3-1_IGLV | GL_nt_setA_IGLJ2 |
| 1E79SAD002_F8 | GL_nt_setA_VH1-2_IGHV | GL_nt_setA_IGHD1-20 | GL_nt_setA_IGHJ3 | GL_nt_setA_VL1-44_IGLV | GL_nt_setA_IGLJ2 |
| 1F58SAC004_E3 | GL_nt_setA_VH3-30-5_IGHV | GL_nt_setA_IGHD4-17 | GL_nt_setA_IGHJ6 | GL_nt_setA_VL3-1_IGLV | GL_nt_setA_IGLJ7 |
| 1F39SAD003_A4 | GL_nt_setA_VH3-30-3_IGHV | GL_nt_setA_IGHD4-17 | GL_nt_setA_IGHJ3 | GL_nt_setA_VL3-21_IGLV | GL_nt_setA_IGLJ2 |
| 1E79SAD002_E11 | GL_nt_setA_VH5-51_IGHV | GL_nt_setA_IGHD6-19 | GL_nt_setA_IGHJ3 | GL_nt_setA_VK1D-13_IGKV | GL_nt_setA_IGKJ4 |
| 1F39SAD004_F2 | GL_nt_setA_VH5-51_IGHV | GL_nt_setA_IGHD6-19 | GL_nt_setA_IGHJ3 | GL_nt_setA_VK3-11_IGKV | GL_nt_setA_IGKJ5 |
| 1F88SAA005_F9 | GL_nt_setA_VH1-24_IGHV | GL_nt_setA_IGHD2-21 | GL_nt_setA_IGHJ3 | | |
| 1F39SAD006_F10 | GL_nt_setA_VH1-18_IGHV | GL_nt_setA_IGHD4-17 | GL_nt_setA_IGHJ3 | GL_nt_setA_VL2-11_IGLV | GL_nt_setA_IGLJ2 |
| 1F39SAD004_G10 | GL_nt_setA_VH3-23D_IGHV | GL_nt_setA_IGHD3-10 | GL_nt_setA_IGHJ4 | GL_nt_setA_VL1-44_IGLV | GL_nt_setA_IGLJ7 |
| 1F39SAD004_F4 | GL_nt_setA_VH1-18_IGHV | GL_nt_setA_IGHD3-10 | GL_nt_setA_IGHJ1 | GL_nt_setA_VL3-21_IGLV | GL_nt_setA_IGLJ2 |
| 1F39SAD009_F7 | GL_nt_setA_VH6-1_IGHV | GL_nt_setA_IGHD2-8 | GL_nt_setA_IGHJ3 | GL_nt_setA_VL2-14_IGLV | GL_nt_setA_IGLJ1 |
| 1F39SAD009_E3 | GL_nt_setA_VH5-51_IGHV | GL_nt_setA_IGHD6-19 | GL_nt_setA_IGHJ3 | GL_nt_setA_VL1-44_IGLV | GL_nt_setA_IGLJ2 |
| 1F39SAD004_F5 | GL_nt_setA_VH1-69D_IGHV | GL_nt_setA_IGHD3-9 | GL_nt_setA_IGHJ6 | GL_nt_setA_VK2-28_IGKV | GL_nt_setA_IGKJ1 |
| 1F39SAD006_H11 | GL_nt_setA_VH3-30-5_IGHV | GL_nt_setA_IGHD2-21 | GL_nt_setA_IGHJ6 | GL_nt_setA_VL1-44_IGLV | GL_nt_setA_IGLJ7 |
| 1F39SAD004_E2 | GL_nt_setA_VH3-9_IGHV | GL_nt_setA_IGHD6-13 | GL_nt_setA_IGHJ6 | GL_nt_setA_VL3-21_IGLV | GL_nt_setA_IGLJ2 |
| 1E79SAD003_H2 | GL_nt_setA_VH1-2_IGHV | GL_nt_setA_IGHD3-16 | GL_nt_setA_IGHJ6 | GL_nt_setA_VL2-14_IGLV | GL_nt_setA_IGLJ1 |
| 1F39SAD003_G3 | GL_nt_setA_VH1-18_IGHV | GL_nt_setA_IGHD6-13 | GL_nt_setA_IGHJ4 | GL_nt_setA_VL3-1_IGLV | GL_nt_setA_IGLJ1 |
| 1F39SAD001_B10 | GL_nt_setA_VH3-30-5_IGHV | GL_nt_setA_IGHD6-25 | GL_nt_setA_IGHJ6 | GL_nt_setA_VK1-12_IGKV | GL_nt_setA_IGKJ4 |
| 1F58SAC004_A1 | GL_nt_setA_VH3-30-5_IGHV | GL_nt_setA_IGHD5-12 | GL_nt_setA_IGHJ3 | GL_nt_setA_VK3-11_IGKV | GL_nt_setA_IGKJ4 |
| 1F39SAD006_F3 | GL_nt_setA_VH3-33_IGHV | GL_nt_setA_IGHD5-12 | GL_nt_setA_IGHJ4 | GL_nt_setA_VL1-44_IGLV | GL_nt_setA_IGLJ7 |
| 1F88SAA005_C6 | GL_nt_setA_VH3-9_IGHV | GL_nt_setA_IGHD3-22 | GL_nt_setA_IGHJ4 | GL_nt_setA_VL1-44_IGLV | GL_nt_setA_IGLJ2 |
| 1F39SAD009_D11 | GL_nt_setA_VH1-2_IGHV | GL_nt_setA_IGHD2-8 | GL_nt_setA_IGHJ3 | GL_nt_setA_VK1-39_IGKV | GL_nt_setA_IGKJ2 |
| 1F39SAD003_F8 | GL_nt_setA_VH3-30-3_IGHV | GL_nt_setA_IGHD3-10 | GL_nt_setA_IGHJ6 | GL_nt_setA_VL3-1_IGLV | GL_nt_setA_IGLJ2 |
| 1F88SAA005_E4 | GL_nt_setA_VH1-18_IGHV | GL_nt_setA_IGHD4-17 | GL_nt_setA_IGHJ2 | GL_nt_setA_VL1-47_IGLV | GL_nt_setA_IGLJ1 |
| 1E79SAD002_A11 | GL_nt_setA_VH1-69D_IGHV | GL_nt_setA_IGHD5-12 | GL_nt_setA_IGHJ6 | GL_nt_setA_VL1-44_IGLV | GL_nt_setA_IGLJ2 |
| 1E79SAD002_G3 | GL_nt_setA_VH1-69D_IGHV | GL_nt_setA_IGHD5-12 | GL_nt_setA_IGHJ3 | GL_nt_setA_VL1-44_IGLV | GL_nt_setA_IGLJ2 |
| 1F39SAD004_B8 | GL_nt_setA_VH3-30-3_IGHV | GL_nt_setA_IGHD2-21 | GL_nt_setA_IGHJ3 | GL_nt_setA_VL3-1_IGLV | GL_nt_setA_IGLJ2 |
| 1F58SAC004_A3 | GL_nt_setA_VH5-51_IGHV | GL_nt_setA_IGHD1-26 | GL_nt_setA_IGHJ5 | GL_nt_setA_VL1-44_IGLV | GL_nt_setA_IGLJ2 |
| 1F58SAC004_E2 | GL_nt_setA_VH5-51_IGHV | GL_nt_setA_IGHD1-26 | GL_nt_setA_IGHJ5 | GL_nt_setA_VL1-44_IGLV | GL_nt_setA_IGLJ2 |
| 1F39SAD003_A11 | GL_nt_setA_VH1-3_IGHV | GL_nt_setA_IGHD1-26 | GL_nt_setA_IGHJ5 | GL_nt_setA_VL3-1_IGLV | GL_nt_setA_IGLJ7 |
| 1E79SAD002_B1 | GL_nt_setA_VH1-69D_IGHV | GL_nt_setA_IGHD4-4 | GL_nt_setA_IGHJ4 | GL_nt_setA_VL6-57_IGLV | GL_nt_setA_IGLJ2 |
| 1E79SAD003_G1 | GL_nt_setA_VH1-2_IGHV | GL_nt_setA_IGHD3-16 | GL_nt_setA_IGHJ6 | GL_nt_setA_VL2-14_IGLV | GL_nt_setA_IGLJ1 |
| 1F39SAD009_E11 | GL_nt_setA_VH1-8_IGHV | GL_nt_setA_IGHD3-9 | GL_nt_setA_IGHJ4 | GL_nt_setA_VL1-44_IGLV | GL_nt_setA_IGLJ2 |
| 1E79SAD002_H10 | GL_nt_setA_VH3-30-5_IGHV | GL_nt_setA_IGHD6-19 | GL_nt_setA_IGHJ5 | GL_nt_setA_VL3-21_IGLV | GL_nt_setA_IGLJ2 |
| 1F88SAA005_E6 | GL_nt_setA_VH3-30-5_IGHV | GL_nt_setA_IGHD6-13 | GL_nt_setA_IGHJ1 | GL_nt_setA_VL2-8_IGLV | GL_nt_setA_IGLJ2 |
| 1F39SAD003_D8 | GL_nt_setA_VH1-18_IGHV | GL_nt_setA_IGHD2-15 | GL_nt_setA_IGHJ5 | GL_nt_setA_VL2-23_IGLV | GL_nt_setA_IGLJ2 |
| 1F39SAD006_D1 | GL_nt_setA_VH3-53_IGHV | GL_nt_setA_IGHD3-10 | GL_nt_setA_IGHJ4 | GL_nt_setA_VL1-40_IGLV | GL_nt_setA_IGLJ1 |
| 1E79SAD002_C10 | GL_nt_setA_VH3-11_IGHV | GL_nt_setA_IGHD2-2 | GL_nt_setA_IGHJ3 | GL_nt_setA_VL1-51_IGLV | GL_nt_setA_IGLJ2 |
| 1E79SAD002_A5 | GL_nt_setA_VH1-2_IGHV | GL_nt_setA_IGHD2-15 | GL_nt_setA_IGHJ4 | GL_nt_setA_VL8-61_IGLV | GL_nt_setA_IGLJ2 |
| 1F88SAA005_E11 | GL_nt_setA_VH1-2_IGHV | GL_nt_setA_IGHD3-22 | GL_nt_setA_IGHJ5 | GL_nt_setA_VL1-44_IGLV | GL_nt_setA_IGLJ2 |
| 1F58SAC004_A2 | GL_nt_setA_VH1-8_IGHV | GL_nt_setA_IGHD4-17 | GL_nt_setA_IGHJ5 | GL_nt_setA_VK3-11_IGKV | GL_nt_setA_IGKJ4 |
| 1F39SAD004_F12 | GL_nt_setA_VH3-30-3_IGHV | GL_nt_setA_IGHD1-7 | GL_nt_setA_IGHJ3 | GL_nt_setA_VL1-44_IGLV | GL_nt_setA_IGLJ2 |
| 1F39SAD004_A6 | GL_nt_setA_VH1-69D_IGHV | GL_nt_setA_IGHD6-13 | GL_nt_setA_IGHJ5 | GL_nt_setA_VL3-21_IGLV | GL_nt_setA_IGLJ2 |
| 1F39SAD009_B12 | GL_nt_setA_VH5-51_IGHV | GL_nt_setA_IGHD5-12 | GL_nt_setA_IGHJ3 | GL_nt_setA_VL1-44_IGLV | GL_nt_setA_IGLJ2 |
| 1E79SAD002_B6 | GL_nt_setA_VH5-51_IGHV | GL_nt_setA_IGHD5-12 | GL_nt_setA_IGHJ3 | GL_nt_setA_VL1-44_IGLV | GL_nt_setA_IGLJ2 |
| 1F39SAD006_G7 | GL_nt_setA_VH1-18_IGHV | GL_nt_setA_IGHD4-17 | GL_nt_setA_IGHJ4 | GL_nt_setA_VL1-44_IGLV | GL_nt_setA_IGLJ2 |
| 1E79SAD002_F11 | GL_nt_setA_VH1-18_IGHV | GL_nt_setA_IGHD3-10 | GL_nt_setA_IGHJ1 | GL_nt_setA_VL3-21_IGLV | GL_nt_setA_IGLJ2 |
| 1F39SAD003_A2 | GL_nt_setA_VH6-1_IGHV | GL_nt_setA_IGHD3-16 | GL_nt_setA_IGHJ3 | GL_nt_setA_VL2-14_IGLV | GL_nt_setA_IGLJ2 |
| 1F39SAD009_E7 | GL_nt_setA_VH1-69D_IGHV | GL_nt_setA_IGHD2-2 | GL_nt_setA_IGHJ6 | GL_nt_setA_VL3-1_IGLV | GL_nt_setA_IGLJ2 |
| 1E79SAD003_C4 | GL_nt_setA_VH5-51_IGHV | GL_nt_setA_IGHD2-21 | GL_nt_setA_IGHJ3 | GL_nt_setA_VL1-44_IGLV | GL_nt_setA_IGLJ1 |
| 1F39SAD006_G10 | GL_nt_setA_VH5-51_IGHV | GL_nt_setA_IGHD1-26 | GL_nt_setA_IGHJ6 | GL_nt_setA_VL1-44_IGLV | GL_nt_setA_IGLJ2 |
| 1F39SAD006_D9 | GL_nt_setA_VH5-51_IGHV | GL_nt_setA_IGHD4-17 | GL_nt_setA_IGHJ5 | GL_nt_setA_VL1-44_IGLV | GL_nt_setA_IGLJ2 |
| 1E79SAD002_D8 | GL_nt_setA_VH5-51_IGHV | GL_nt_setA_IGHD3-16 | GL_nt_setA_IGHJ3 | GL_nt_setA_VL1-44_IGLV | GL_nt_setA_IGLJ1 |
| 1F39SAD006_G12 | GL_nt_setA_VH5-51_IGHV | GL_nt_setA_IGHD5-12 | GL_nt_setA_IGHJ4 | GL_nt_setA_VL1-44_IGLV | GL_nt_setA_IGLJ2 |
| 1F58SAC004_D2 | GL_nt_setA_VH5-51_IGHV | GL_nt_setA_IGHD3-10 | GL_nt_setA_IGHJ4 | GL_nt_setA_VL1-44_IGLV | GL_nt_setA_IGLJ2 |
| 1E79SAD002_B7 | GL_nt_setA_VH5-51_IGHV | GL_nt_setA_IGHD6-13 | GL_nt_setA_IGHJ5 | GL_nt_setA_VL1-47_IGLV | GL_nt_setA_IGLJ2 |
| 1F58SAC004_B6 | GL_nt_setA_VH5-51_IGHV | GL_nt_setA_IGHD3-10 | GL_nt_setA_IGHJ3 | GL_nt_setA_VL1-47_IGLV | GL_nt_setA_IGLJ2 |
| 1F58SAC004_E5 | GL_nt_setA_VH5-51_IGHV | GL_nt_setA_IGHD4-17 | GL_nt_setA_IGHJ5 | GL_nt_setA_VL1-44_IGLV | GL_nt_setA_IGLJ2 |
| 1E79SAD002_F6 | GL_nt_setA_VH5-51_IGHV | GL_nt_setA_IGHD1-1 | GL_nt_setA_IGHJ6 | GL_nt_setA_VL1-44_IGLV | GL_nt_setA_IGLJ2 |
| 1F39SAD006_B10 | GL_nt_setA_VH5-51_IGHV | GL_nt_setA_IGHD5-12 | GL_nt_setA_IGHJ4 | GL_nt_setA_VL1-44_IGLV | GL_nt_setA_IGLJ2 |
| 1E79SAD002_E5 | GL_nt_setA_VH5-51_IGHV | GL_nt_setA_IGHD3-3 | GL_nt_setA_IGHJ3 | GL_nt_setA_VL1-44_IGLV | GL_nt_setA_IGLJ2 |
| 1F39SAD004_D8 | GL_nt_setA_VH5-51_IGHV | GL_nt_setA_IGHD3-3 | GL_nt_setA_IGHJ3 | GL_nt_setA_VL1-44_IGLV | GL_nt_setA_IGLJ2 |
| 1F39SAD009_C6 | GL_nt_setA_VH5-51_IGHV | GL_nt_setA_IGHD5-12 | GL_nt_setA_IGHJ4 | GL_nt_setA_VL1-44_IGLV | GL_nt_setA_IGLJ2 |
| 1E79SAD002_F5 | GL_nt_setA_VH5-51_IGHV | GL_nt_setA_IGHD5-18 | GL_nt_setA_IGHJ4 | GL_nt_setA_VL1-44_IGLV | GL_nt_setA_IGLJ1 |
| 1E79SAD002_H6 | GL_nt_setA_VH5-51_IGHV | GL_nt_setA_IGHD3-10 | GL_nt_setA_IGHJ4 | GL_nt_setA_VL1-44_IGLV | GL_nt_setA_IGLJ1 |
| 1E79SAD002_A1 | GL_nt_setA_VH5-51_IGHV | GL_nt_setA_IGHD5-12 | GL_nt_setA_IGHJ4 | GL_nt_setA_VL1-44_IGLV | GL_nt_setA_IGLJ1 |
| 1E79SAD002_D7 | GL_nt_setA_VH5-51_IGHV | GL_nt_setA_IGHD5-12 | GL_nt_setA_IGHJ4 | GL_nt_setA_VL1-44_IGLV | GL_nt_setA_IGLJ2 |
| 1F39SAD009_E4 | GL_nt_setA_VH5-51_IGHV | GL_nt_setA_IGHD5-12 | GL_nt_setA_IGHJ3 | GL_nt_setA_VL1-44_IGLV | GL_nt_setA_IGLJ2 |
| 1E79SAD002_F4 | GL_nt_setA_VH5-51_IGHV | GL_nt_setA_IGHD3-3 | GL_nt_setA_IGHJ3 | GL_nt_setA_VL1-44_IGLV | GL_nt_setA_IGLJ2 |
| 1F39SAD009_F12 | GL_nt_setA_VH1-69D_IGHV | GL_nt_setA_IGHD6-19 | GL_nt_setA_IGHJ3 | GL_nt_setA_VL2-14_IGLV | GL_nt_setA_IGLJ1 |
| 1F39SAD003_E9 | GL_nt_setA_VH1-46_IGHV | GL_nt_setA_IGHD3-22 | GL_nt_setA_IGHJ3 | GL_nt_setA_VL3-21_IGLV | GL_nt_setA_IGLJ2 |
| 1F39SAD003_D6 | GL_nt_setA_VH1-46_IGHV | GL_nt_setA_IGHD3-22 | GL_nt_setA_IGHJ3 | GL_nt_setA_VL3-21_IGLV | GL_nt_setA_IGLJ1 |
| 1F39SAD006_G9 | GL_nt_setA_VH1-18_IGHV | GL_nt_setA_IGHD5-12 | GL_nt_setA_IGHJ6 | GL_nt_setA_VL2-23_IGLV | GL_nt_setA_IGLJ2 |
| 1E79SAD002_F9 | GL_nt_setA_VH1-45_IGHV | GL_nt_setA_IGHD3-16 | GL_nt_setA_IGHJ3 | GL_nt_setA_VL1-44_IGLV | GL_nt_setA_IGLJ1 |
| 1F58SAC004_H5 | GL_nt_setA_VH5-51_IGHV | GL_nt_setA_IGHD7-27 | GL_nt_setA_IGHJ3 | GL_nt_setA_VL1-44_IGLV | GL_nt_setA_IGLJ2 |
| 1F39SAD003_B3 | GL_nt_setA_VH5-51_IGHV | GL_nt_setA_IGHD1-7 | GL_nt_setA_IGHJ5 | GL_nt_setA_VL1-44_IGLV | GL_nt_setA_IGLJ1 |
| 1F39SAD006_H8 | GL_nt_setA_VH5-51_IGHV | GL_nt_setA_IGHD7-27 | GL_nt_setA_IGHJ6 | GL_nt_setA_VL1-44_IGLV | GL_nt_setA_IGLJ2 |
| 1F39SAD001_A6 | GL_nt_setA_VH1-46_IGHV | GL_nt_setA_IGHD4-4 | GL_nt_setA_IGHJ4 | GL_nt_setA_VL6-57_IGLV | GL_nt_setA_IGLJ2 |
| 1F39SAD009_A4 | GL_nt_setA_VH5-51_IGHV | GL_nt_setA_IGHD1-26 | GL_nt_setA_IGHJ5 | GL_nt_setA_VL1-44_IGLV | GL_nt_setA_IGLJ2 |
| 1F58SAC004_C5 | GL_nt_setA_VH5-51_IGHV | GL_nt_setA_IGHD2-15 | GL_nt_setA_IGHJ5 | GL_nt_setA_VL1-44_IGLV | GL_nt_setA_IGLJ7 |
| 1E79SAD002_G11 | GL_nt_setA_VH3-33_IGHV | GL_nt_setA_IGHD6-6 | GL_nt_setA_IGHJ3 | GL_nt_setA_VL3-21_IGLV | GL_nt_setA_IGLJ7 |
| 1F39SAD003_D3 | GL_nt_setA_VH1-46_IGHV | GL_nt_setA_IGHD3-22 | GL_nt_setA_IGHJ3 | GL_nt_setA_VL3-1_IGLV | GL_nt_setA_IGLJ1 |
| 1F39SAD006_D3 | GL_nt_setA_VH5-51_IGHV | GL_nt_setA_IGHD6-19 | GL_nt_setA_IGHJ5 | GL_nt_setA_VL1-44_IGLV | GL_nt_setA_IGLJ1 |
| 1F39SAD000_C11 | GL_nt_setA_VH5-51_IGHV | GL_nt_setA_IGHD3-16 | GL_nt_setA_IGHJ5 | GL_nt_setA_VL1-44_IGLV | GL_nt_setA_IGLJ2 |
| 1E79SAD002_G7 | GL_nt_setA_VH5-51_IGHV | GL_nt_setA_IGHD3-3 | GL_nt_setA_IGHJ5 | GL_nt_setA_VL1-44_IGLV | GL_nt_setA_IGLJ2 |
| 1E79SAD002_B8 | GL_nt_setA_VH5-51_IGHV | GL_nt_setA_IGHD5-12 | GL_nt_setA_IGHJ3 | GL_nt_setA_VL1-44_IGLV | GL_nt_setA_IGLJ2 |
| 1F58SAC004_C2 | GL_nt_setA_VH1-69D_IGHV | GL_nt_setA_IGHD6-19 | GL_nt_setA_IGHJ3 | GL_nt_setA_VL1-47_IGLV | GL_nt_setA_IGLJ2 |
| 1F39SAD003_C11 | GL_nt_setA_VH1-24_IGHV | GL_nt_setA_IGHD5-18 | GL_nt_setA_IGHJ4 | GL_nt_setA_VL2-14_IGLV | GL_nt_setA_IGLJ2 |
| 1F39SAD003_C9 | GL_nt_setA_VH1-24_IGHV | GL_nt_setA_IGHD5-18 | GL_nt_setA_IGHJ4 | GL_nt_setA_VK3-20_IGKV | GL_nt_setA_IGKJ1 |
| 1E79SAD002_G10 | GL_nt_setA_VH1-69D_IGHV | GL_nt_setA_IGHD5-18 | GL_nt_setA_IGHJ3 | GL_nt_setA_VK1-39_IGKV | GL_nt_setA_IGKJ2 |
| 1F39SAD003_H2 | GL_nt_setA_VH1-2_IGHV | GL_nt_setA_IGHD5-18 | GL_nt_setA_IGHJ1 | GL_nt_setA_VL6-57_IGLV | GL_nt_setA_IGLJ2 |
| 1F39SAD003_F1 | GL_nt_setA_VH1-24_IGHV | GL_nt_setA_IGHD6-13 | GL_nt_setA_IGHJ5 | GL_nt_setA_VL2-8_IGLV | GL_nt_setA_IGLJ1 |
| 1F39SAD003_D12 | GL_nt_setA_VH1-24_IGHV | GL_nt_setA_IGHD3-10 | GL_nt_setA_IGHJ5 | GL_nt_setA_VL1-51_IGLV | GL_nt_setA_IGLJ2 |
| 1F39SAD006_C12 | GL_nt_setA_VH1-2_IGHV | GL_nt_setA_IGHD6-19 | GL_nt_setA_IGHJ4 | GL_nt_setA_VL1-44_IGLV | GL_nt_setA_IGLJ2 |
| 1E79SAD002_H2 | GL_nt_setA_VH5-51_IGHV | GL_nt_setA_IGHD5-18 | GL_nt_setA_IGHJ4 | GL_nt_setA_VL1-44_IGLV | GL_nt_setA_IGLJ2 |
| 1F58SAC004_A6 | GL_nt_setA_VH3-30-5_IGHV | GL_nt_setA_IGHD6-19 | GL_nt_setA_IGHJ3 | GL_nt_setA_VL3-1_IGLV | GL_nt_setA_IGLJ2 |
| 1E79SAD002_F8 | GL_nt_setA_VH1-2_IGHV | GL_nt_setA_IGHD1-20 | GL_nt_setA_IGHJ3 | GL_nt_setA_VL1-44_IGLV | GL_nt_setA_IGLJ2 |
| 1F58SAC004_E3 | GL_nt_setA_VH3-30-5_IGHV | GL_nt_setA_IGHD4-17 | GL_nt_setA_IGHJ6 | GL_nt_setA_VL3-1_IGLV | GL_nt_setA_IGLJ7 |
| 1F39SAD003_A4 | GL_nt_setA_VH3-30-3_IGHV | GL_nt_setA_IGHD4-17 | GL_nt_setA IGHJ3 | GL_nt_setA_VL3-21_IGLV | GL_nt_setA_IGLJ2 |
| 1E79SAD002_E11 | GL_nt_setA_VH5-51_IGHV | GL_nt_setA_IGHD6-19 | GL_nt_setA_IGHJ3 | GL_nt_setA_VK1D-13 IGKV | GL_nt_setA_IGKJ4 |
| 1F39SAD004_F2 | GL_nt_setA_VH5-51_IGHV | GL_nt_setA_IGHD6-19 | GL_nt_setA_IGHJ3 | GL_nt_setA_VK3-11_IGKV | GL_nt_setA_IGKJ5 |
| 1F88SAA005_F9 | GL_nt_setA_VH1-24_IGHV | GL_nt_setA_IGHD2-21 | GL_nt_setA_IGHJ3 | | |
| 1F39SAD006_F10 | GL_nt_setA_VH1-18_IGHV | GL_nt_setA_IGHD4-17 | GL_nt_setA_IGHJ3 | GL_nt_setA_VL2-11_IGLV | GL_nt_setA_IGLJ2 |
| 1F39SAD004_G10 | GL_nt_setA_VH3-23D_IGHV | GL_nt_setA_IGHD3-10 | GL_nt_setA_IGHJ4 | GL_nt_setA_VL1-44_IGLV | GL_nt_setA_IGLJ7 |

**Table 1d**

| library | clone | South African RBD | RBD |
|---|---|---|---|
| Naive | 1FD6SAA003_B4 | 1.3198 | 1.2773 |
| Patient | 1FD6SAA002_G12 | 2.141 | 1.9201 |
| Naive | 1FD6SAA003_G3 | 1.5311 | 1.136 |
| Naive | 1FD6SAA003_F10 | 2.8692 | 2.6068 |
| Naive | 1FD6SAA003_H3 | 1.2908 | 1.2739 |
| Patient | 1FD6SAA002_F8 | 0.7581 | 0.6766 |
| Patient | 1FD6SAA002_G5 | 0.7732 | 1.0524 |
| Patient | 1FD6SAA002_F10 | 0.4264 | 0.6864 |
| Naive | 1FD6SAA003_C9 | 0.5235 | 0.6432 |
| Naive | 1FD6SAA003_F1 | 0.5919 | 0.7893 |
| Naive | 1FD6SAA003_H5 | 1.7094 | 1.4006 |
| Naive | 1FD6SAA003_C10 | 1.5885 | 1.1225 |
| Naive | 1FD6SAA003_G9 | 0.9931 | 0.0275 |
| Corona | 2015SAB004_C10 | 1.5252 | 2.0062 |
| Corona | 2015SAB004_G11 | 0.8429 | 0.2071 |
| Naive | 1FD6SAA003_C12 | 1.482 | 1.5769 |
| Naive | 1FD6SAA003_H7 | 1.4479 | 1.4728 |
| Naive | 1FD6SAA003_D5 | 2.0179 | 1.9848 |
| Naive | 1FD6SAA003_H8 | 1.5509 | 1.1922 |
| Patient | 1FD6SAA002_F12 | 1.1339 | 0.9654 |
| Naive | 1FD6SAA003_A4 | 1.2638 | 0.9924 |
| Naive | 1FD6SAA003_D2 | 0.9195 | 0.6492 |

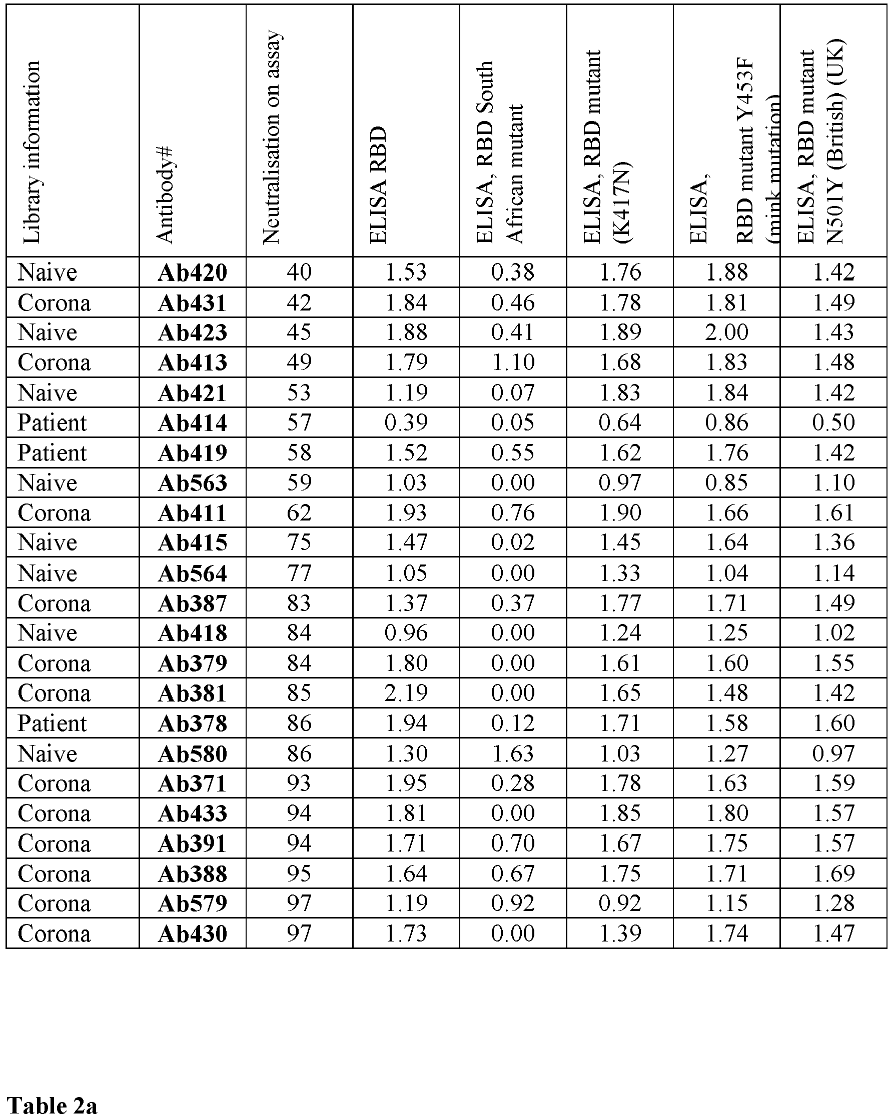

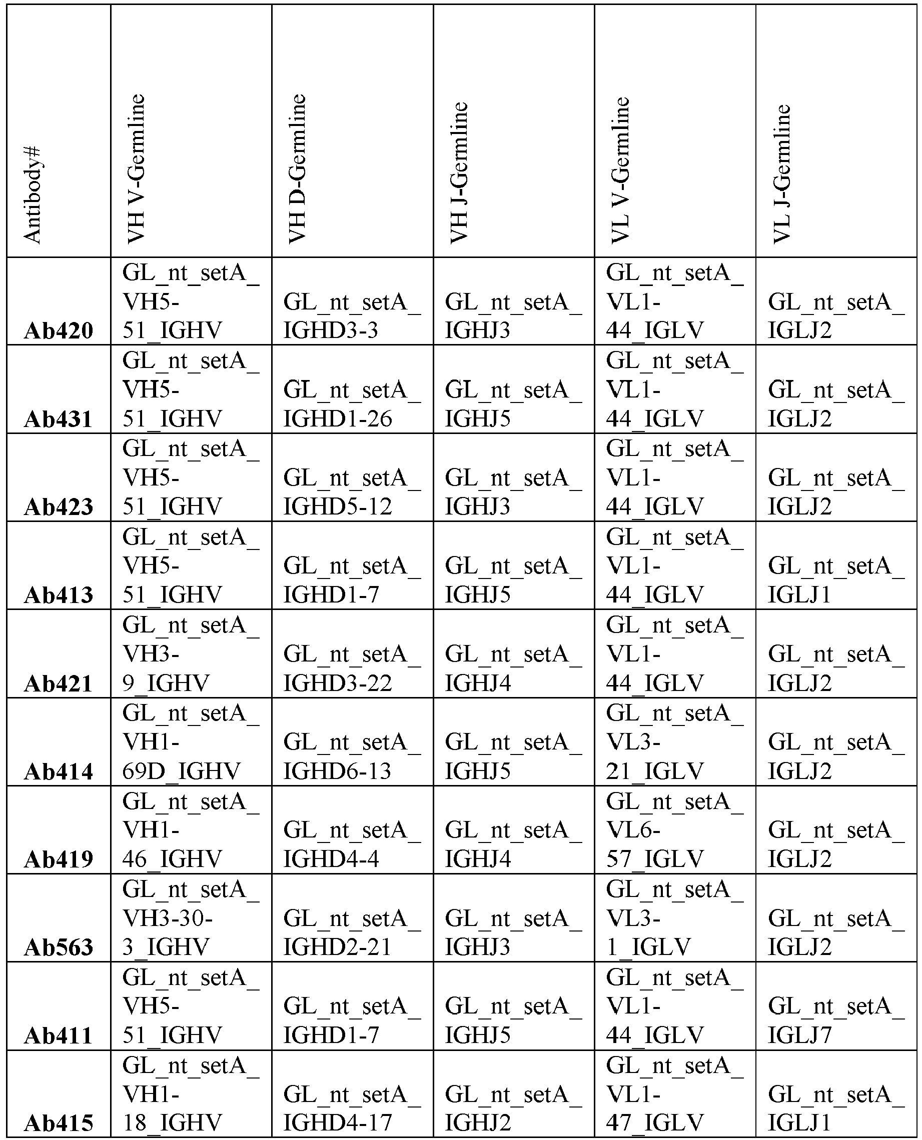

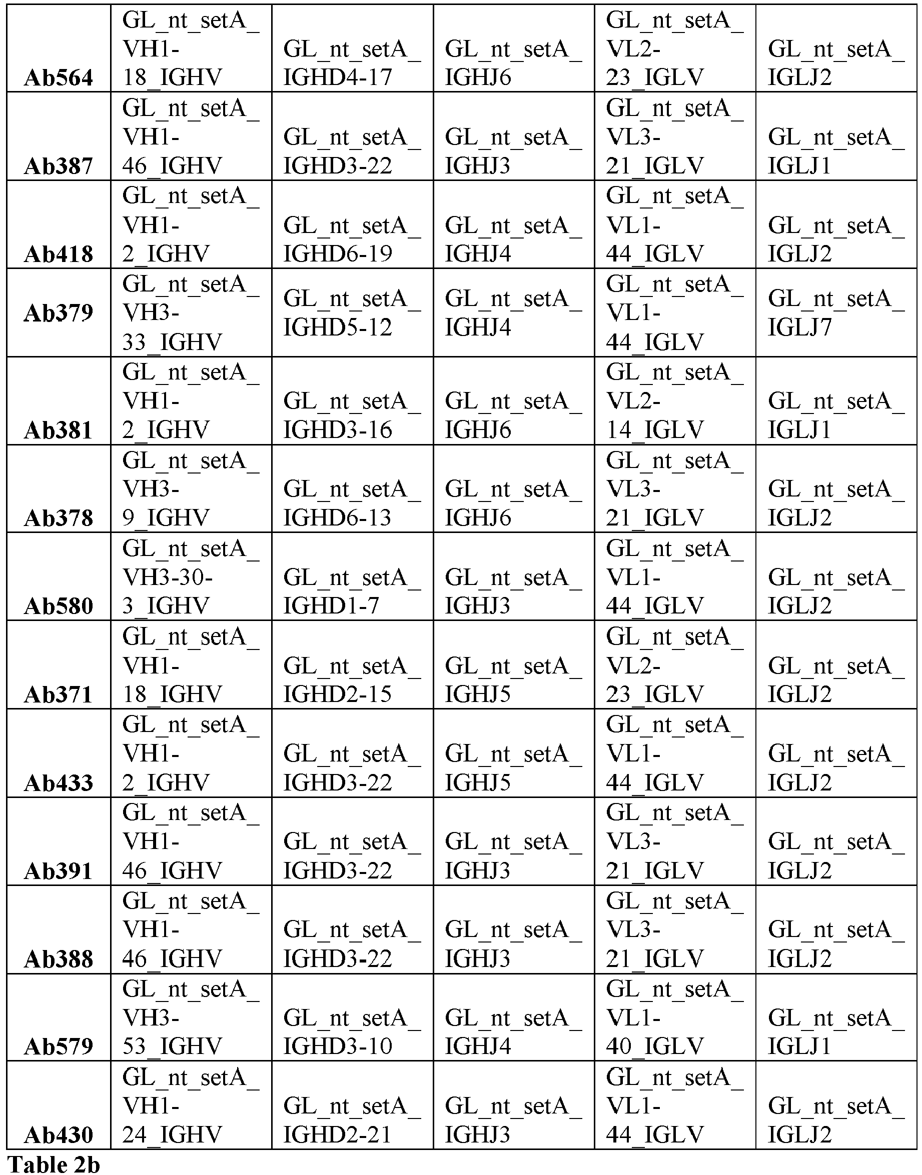

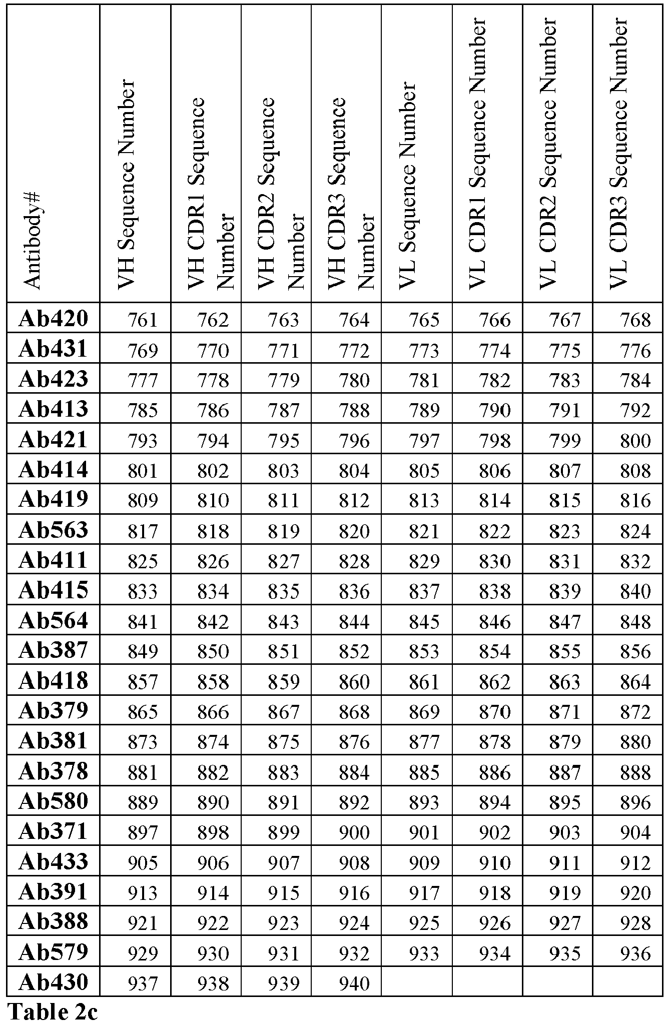

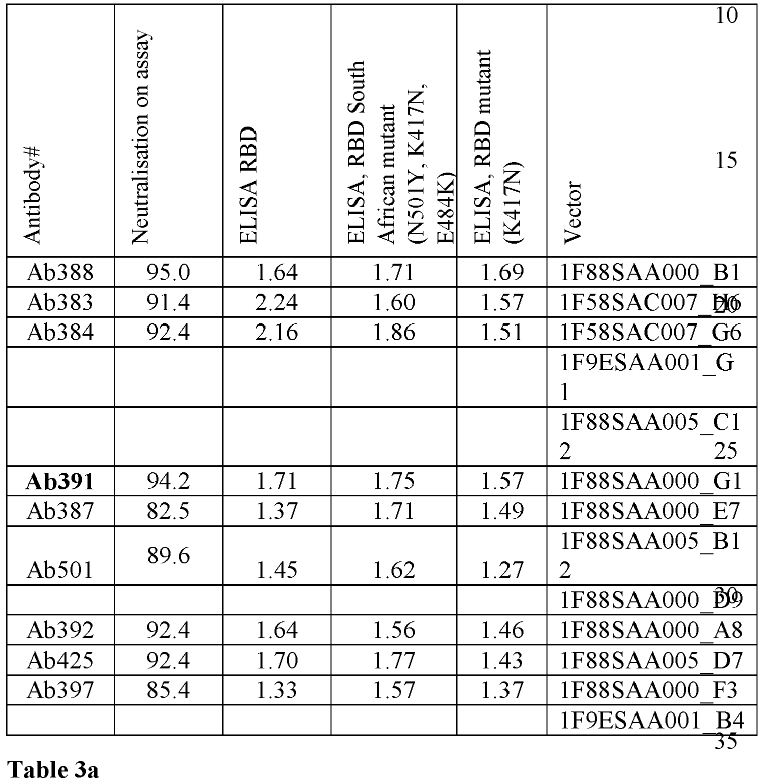

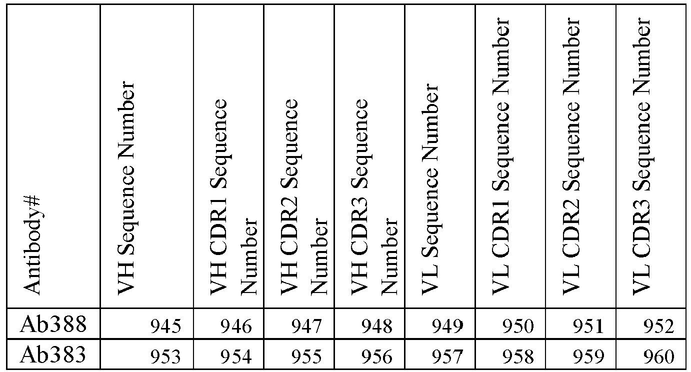

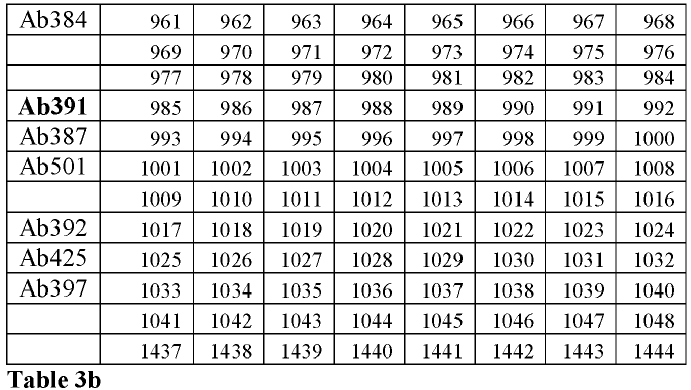

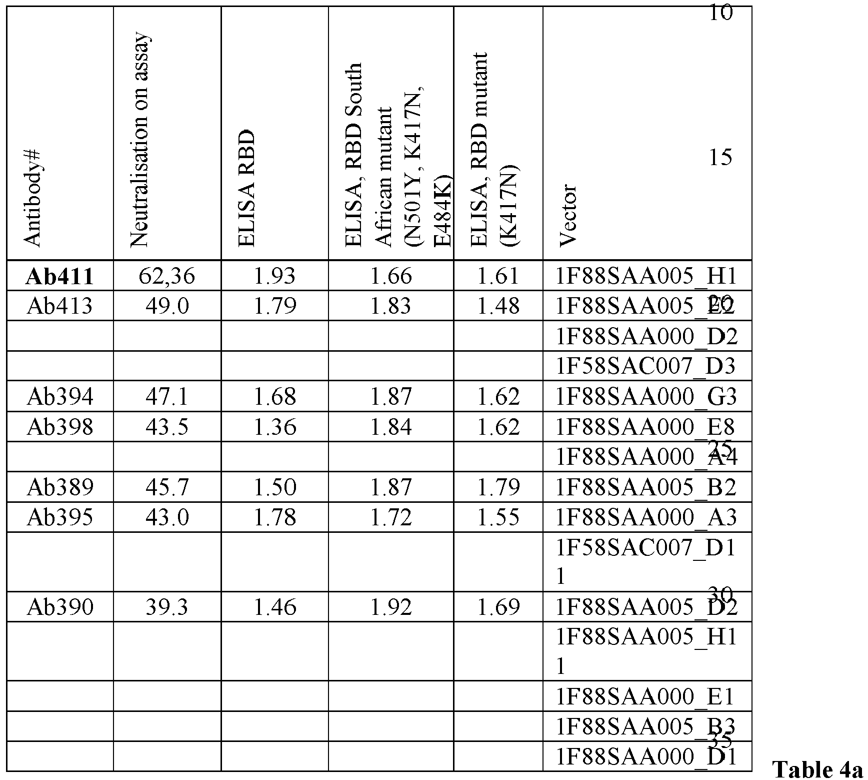

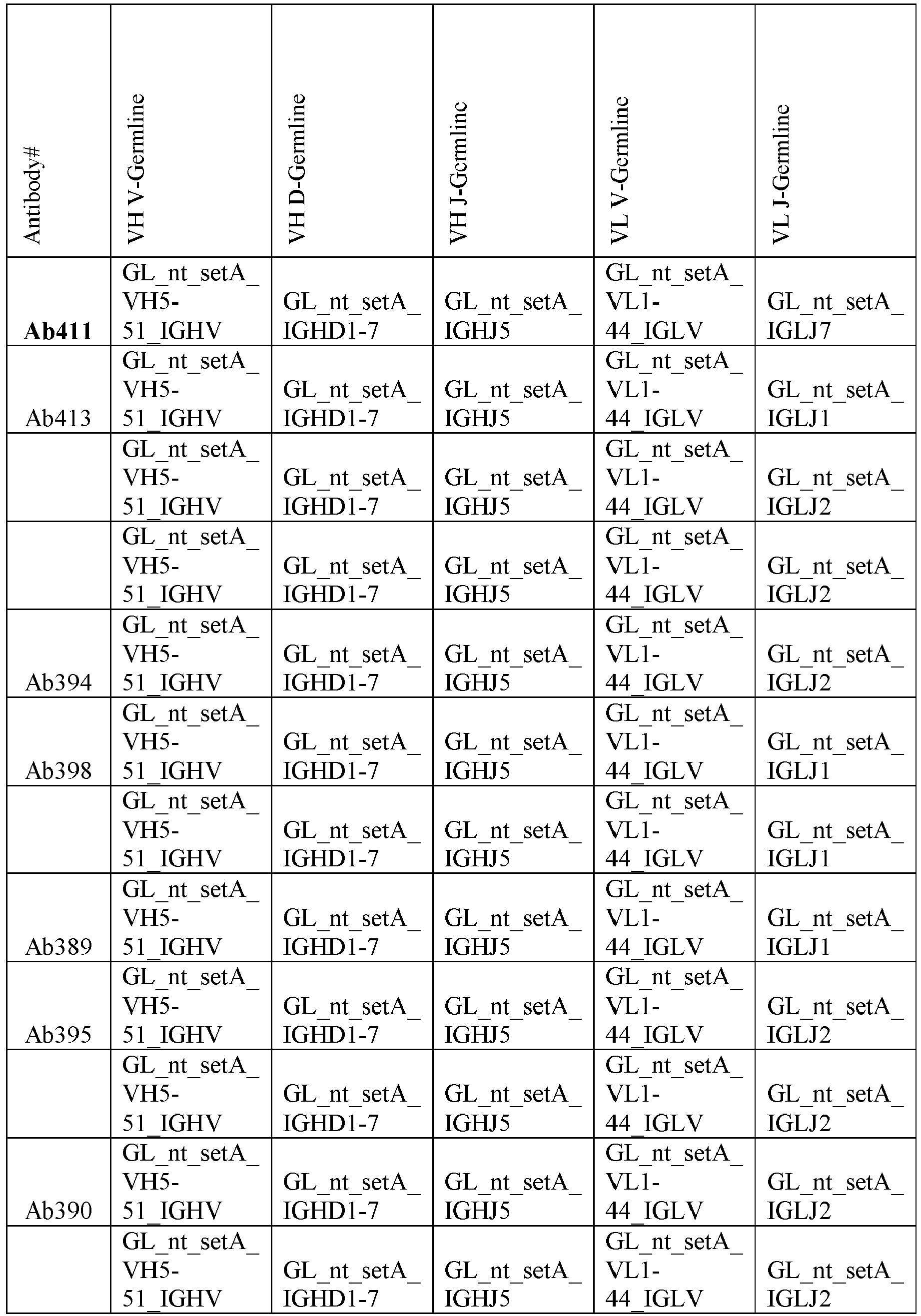

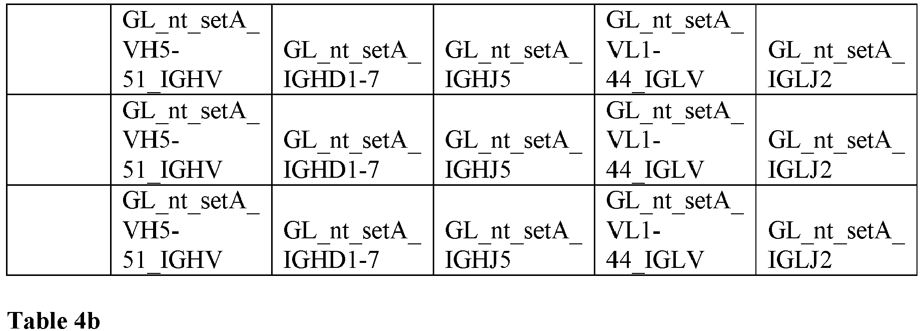

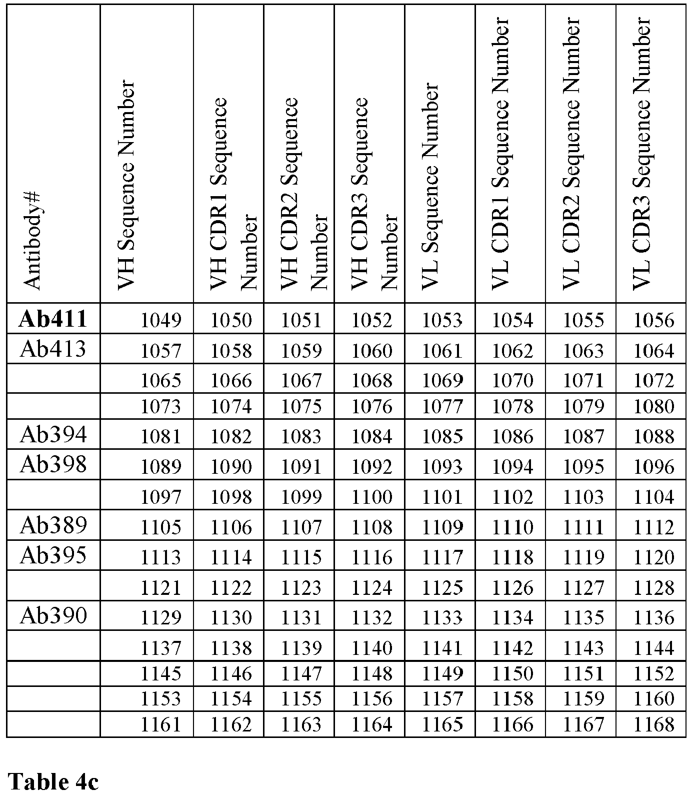

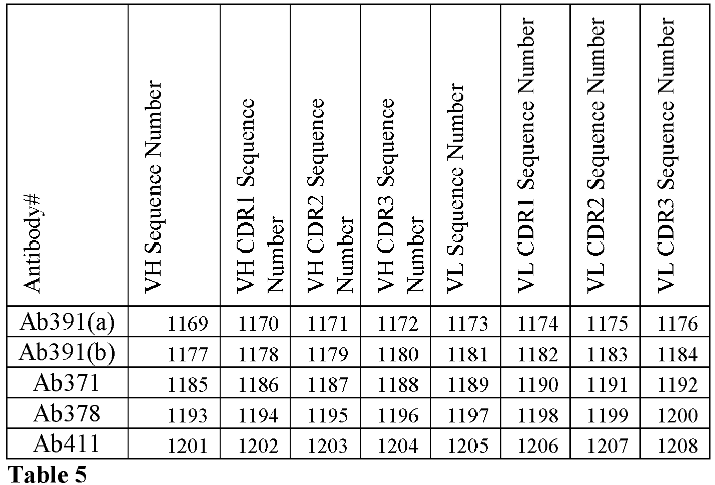

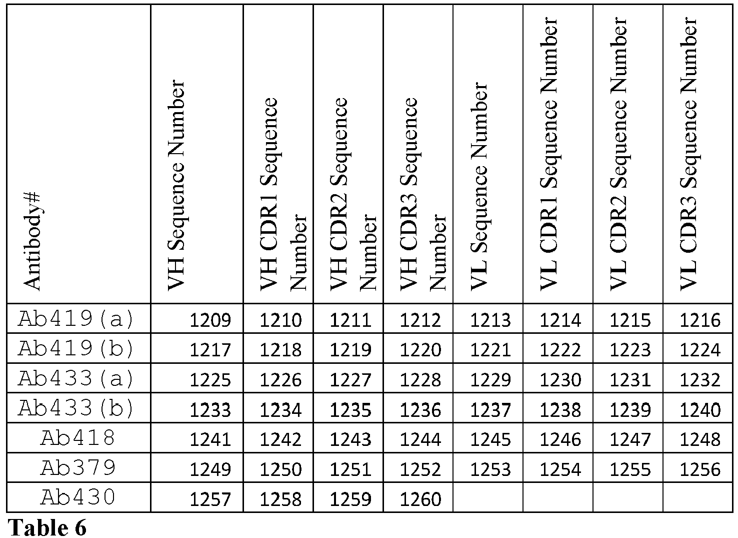

**Table 7**

| | Local fit | | | | Global group fit | | | |
|---|---|---|---|---|---|---|---|---|
| | **KD (M)** | **KD error** | **kdis(1/s)** | **ka(1/Ms)** | **KD (M)** | **KD error** | **kdis(1/s)** | **ka(1/Ms)** |
| Ab378.2 | 3.274E-10 | 3.081E-12 | 6.46E-04 | 3.27E+06 | 5.05E-10 | 3.88E-12 | 6.73E-04 | 1.33E+06 |
| Ab391.1 | 1.7632E-10 | 3.8035E-12 | 9.22E-05 | 6.45E+05 | 3.22E-10 | <1.0E-12 | 1.57E-04 | 4.87E+05 |
| Ab371.1 | 5.3898E-10 | 4.4863E-12 | 3.68E-04 | 5.55E+05 | 1.11E-09 | 3.58E-12 | 4.77E-04 | 4.29E+05 |
| Ab411.1 | 4.03E-10 | 3.50E-11 | 2.75E-04 | 1.18E+07 | 3.87E-10 | <1.0E-12 | 1.28E-04 | 3.32E+05 |

**Table 8**

| **Ab** | **Ab371_hIgG1_L** | **Ab378_hIgG1_L** | **Ab391_hIgG1_L** | **Ab391.A_hIgG1_L** | **Ab379_hIgG1_L** | **Ab418_hIgG1_L** | **Ab430_hIgG1-Fc_VHH** | **Ab433_hIgG1_L** | **Ab433.A_hIgG1_L** | **Ab411_hIgG1_L** | **Ab371.2** |
|---|---|---|---|---|---|---|---|---|---|---|---|
| **UK** | | | | | | | | | | | |
| *0.001* | 1.2329 | 1.096 | 1.8996 | 1.4912 | 1.7373 | 0.5013 | 1.3145 | 1.7785 | 1.8134 | 1.3753 | 2.3605 |
| *0.0001* | 0.7829 | 0.4207 | 1.2029 | 1.2101 | 1.9037 | 0.1186 | 1.3868 | 0.6026 | 0.882 | 1.1587 | |
| *1E-05* | 0.359 | 0.1675 | 0.8426 | 0.3636 | 0.469 | 0.0057 | 0.5148 | 0.0923 | 0.1378 | 0.8536 | |

| **Brazil** | | | | | | | | | | | |
|---|---|---|---|---|---|---|---|---|---|---|---|
| *0.001* | 0.7738 | 0.7411 | 1.7419 | 1.2507 | - 0.0169 | 0.0741 | - 0.0085 | 0.0059 | - 0.0147 | 1.2250 | 2.3355 |
| *0.0001* | 0.3752 | 0.1390 | 1.1799 | 1.0904 | - 0.0281 | - 0.0259 | - 0.0102 | 0.0202 | 0.0295 | 1.1917 | |
| *1E-05* | 0.1268 | 0.0467 | 0.7687 | 0.3583 | 0.0522 | - 0.0218 | - 0.0303 | 0.0230 | 0.0493 | 0.3699 | |

| **South Africa** | | | | | | | | | | | |
|---|---|---|---|---|---|---|---|---|---|---|---|
| *0.001* | 0.3440 | 0.3378 | 1.3043 | 0.9603 | - 0.0004 | - 0.0072 | 0.0036 | 0.0175 | - 0.0053 | 0.8192 | 2.3600 |
| *0.0001* | 0.0748 | 0.0775 | 0.7397 | 0.8157 | - 0.0045 | - 0.0058 | - 0.0075 | 0.0473 | - 0.0083 | 0.6501 | |
| *1E-05* | 0.0138 | 0.0254 | 0.4571 | 0.1863 | - 0.0084 | - 0.0068 | - 0.0105 | 0.0280 | - 0.0046 | 0.1986 | |

| **E484K RBD** | | | | | | | | | | | |
|---|---|---|---|---|---|---|---|---|---|---|---|
| *0.001* | 1.3355 | 0.9714 | 2.0177 | 1.5046 | 0.1094 | 0.0075 | 0.0012 | 0.0096 | - 0.0005 | 1.3309 | 2.3620 |
| *0.0001* | 0.6902 | 0.2079 | 1.2543 | 1.1158 | 0.0008 | 0.0125 | 0.0018 | - 0.0051 | - 0.0013 | 0.8111 | |
| *1E-05* | 0.2477 | 0.0873 | 0.7323 | 0.2737 | - 0.0081 | - 0.0049 | - 0.0048 | - 0.003 9 | - 0.0035 | 0.2181 | |

| **Native RBD** | | | | | | | | | | | |
|---|---|---|---|---|---|---|---|---|---|---|---|
| *0.001* | 2.4944 | 2.5176 | 2.7656 | 2.3012 | 2.4700 | 2.2843 | 1.8674 | 2.5706 | 2.5548 | 2.3672 | 2.3545 |
| *0.0001* | 1.6903 | 1.6562 | 1.8659 | 1.6108 | 1.9843 | 1.7422 | 1.7292 | 1.6586 | 1.5916 | 1.4799 | |
| *1E-05* | 0.7074 | 0.8490 | 1.2352 | 0.4586 | 0.4519 | 0.3125 | 0.5187 | 0.2511 | 0.3166 | 0.4329 | |

### Materials and Methods

### Phage Library generation

The libraries were constructed in house from blood samples of human Corona patient, naive human, human patient (autoimmune and Chagas disease) and Llama origin. In short, B-cells from the blood are separated by centrifugation in Ficoll. From the B-cells the mRNA is extracted using standard Trizol methods. From the mRNA cDNA is created using standard methods (oligo dT primer). From the cDNA the regions corresponding to antibody fragments (heavy chain and light chain) are amplified, and subsequently the fragments are purified by agarose gel, digested and again purified. Chains are ligated into the phagemid (pHenIX) and transformed into *Escherichia coli* TG-1 (Immunosource, cat.#60502) using electroporation. For phage display the bacteria are infected with M13 helper phage. Phage libraries were prepared essentially as described in (31). Samples from similar library origin are combined to obtain five different libraries (Human naive; complexity ∼10¹¹, Human (Autoimmune and Chagas disease) patient complexity ∼10¹⁰, IgG from Corona patient complexity ∼10⁹, IgM from corona Patient ∼10⁸ and Llama naive complexity ∼10⁹).

### Phage library panning

The Receptor binding domain (RBD) and Spike protein (S1) of SARS-CoV-2 were used for antibody selection by panning five different ScFv/VHH phage display libraries (see above). Phages were harvested using standard NaCl/PEG precipitation and blocked overnight in 1 w/v% milk in PBS (MPBS; PBS: 137 mM NaCl, 2,7 mM KCl, 10 mM Na₂HPO₄, 1,8 mM KH₂PO₄, pH 7,4) at 4 °C. The blocked phages were incubated with control beads (Dynabeads^{™} M-280 Streptavidin, Thermo Fisher Scientific, cat.#11206D) for deselection for 1 hour. Subsequently, 10 mL of depleted phages were incubated with S1- (Acro biosystems, cat.#MBS-K001) or RBD-functionalized magnetic beads (Acro biosystems, cat.#MBS-K002) for 1,5 hours in 10 wells of a deepwell plate (Thermo Fisher Scientific, cat.#780270). We used 5 ml beads at 1 mg beads per ml in H₂0, equivalent to 318 pmol S1 (25ug) or RBD protein per mg beads, per library for the Naive and Patient libraries and 2.5 ml of beads for the Llama and Corona libraries. Prior to addition of the phages, the magnetic beads were blocked for 1 hour in MPBS. Beads were incubated and washed in 0.05% Tween in PBS (PBST) and finally collected in 100 µl PBS using the King Fisher (Thermo Fisher Scientific). After one round of selection all phages that bound to the beads are used to transfect *E. coli* TG-1 bacteria, for phage amplification for the next round of panning. Similar procedures were performed in round 2 of panning, we used 1/5 of the beads compared the the first round, and increased washing stringency (2x7 min). In the third round of panning functionalized tubes were used instead of the magnetic beads. MaxiSorp Nunc-Immuno tubes (Thermo Fisher Scientific) were functionalized with either RBD (Bio-Connect, cat.#40592-V31H) or S1 protein (Bio-Connect, cat.#40591-V05H1-B). For functionalization the proteins were added to the tubes at 10 µg/ml in 50 mM carbonate buffer (pH 9.5). In the meantime the phages were blocked in MPBS for 1 hour. Blocked phages were transferred to the coated tubes and incubated for 1.5 hours. After extensive washing (20x PBST, 10x PBS) the phages were eluted using Trypsin (Thermo Fisher Scientific, cat.# 25300054) for 10 min and 20 min and neutralized with Fetal bovie serum (FBS; Serena, cat.#S-FBS-EU-015). Eluted phages were used for infection of E. *Coli* TG-1 cells. The infected bacteria were grown to single colonies for picking by RapidPick SP (Hudson Robotics). The individual phage clones were tested for binding to RBD and S1 using ELISA. Ovenight 96-wells plates were coated with 1 µg/mL protein in carbonate buffer at 4 °C. Next, the plates were blocked using a 5 w/v% milk in PBS solution (5%MPBS). Phages, in culture supernatant, were incubated for 1 hour. After washing 3x PBST and 3x PBS, a 5000x dilution of anti-M13 antibody-HRP (Bio-Connect, cat.#11973-MM05T-H) in 2 w/v% milk was added and incubated for 1 hour for detection of antigen-bound phages with TMB (Invitrogen, SB02). To detect for unspecific binding a coating of BSA was used. The SARS-CoV2-positive clones were sequenced for their scFv sequences to obtain unique phage binders.

### DNA sequencing, germline gene analysis of antibodies

The phagemids of SARS-CoV2-positive phage clones were prepared as minipreps by (Qiagen) and sequenced in the ScFv/VHH region using a specific primer for pHenIX (Table 9, Eurogentec). CLC main workbench software and the antibody analysis tool from Qiagen were used to analyze the sequences, IMGT/V-QUEST analysis of antibody sequences resulted in the report of the germline genes origins, the V-region identities, and the length of CDR for the VH and Vκ/λ.

### ELISA binding assay

After initial selection ELISA binding assays were performed with precipitated phages to identify the binding towards the SARS-CoV-2 spike protein (S1) receptor binding domain (RBD, Bio-Connect, cat.#40592-V31H) and to others, like SARS-CoV1 S1 (Bio-Connect, cat.#40591-V05H1), MERS-CoV S1 (Bio-Connect, cat.#40069-V08H), and BSA (Sigma-Aldrich, cat.#A3912) to test nonspecific binding. The proteins were coated at 4 µg/mL (RBD) or 8 µg/mL (CoV1, MERS, BSA) in Carbonate buffer (pH9.6) in a 96-well high bind ELISA plate (Greiner bio-one, cat.#655061) overnight at 4 °C. Nonspecific binding was subsequently blocked using a 2% Milk (Campina, cat.#8715300470249) in PBS solution (2%MPBS) for 1 hour. Precipitated phage samples were diluted 1:10 in 2%MPBS and transferred to the washed 96-well plate (100 µl per well). After 1 hour of incubation at room temperature, the plate was washed 3x with PBST and 3x with PBS and subsequently incubated with anti-M13 antibody conjugates with a horse radish peroxidase (HRP) (Bio-Connect, cat.#11973-MM05T-H) 1:5000 in 2%MPBS for 1 hour. After washing 3x PBST and 3x PBS, the plate was developed using 50 µl/well TMB substrate according to the manufacturer's recommendation and the absorbance was recorded after stopping the reaction by adding 50 µl/well 2M H₂SO4 at 450 nm on a Tecan Infinite F50 plate reader.

For antibodies in supernatant a concentration of 1 - 0.01 µg/ml (100 µl per well) was used and the antibody binding to the proteins was detected using an anti-human IgG conjugated with a HRP (ITK Diagnostics B.V., cat.#2010-05). Besides RBD also various single mutated versions of RBD were tested, namely the E484K, K417N, Y543F, N501Y, and some multi-mutated version.

Purified antibodies were detected using the anti-Human-HRP antibody conjugate.

### ELISA neutralization assay

An ELISA neutralization assay was used to identify phages or antibodies that prevent the binding of the RBD to the SARS-CoV-2 receptor angiotensin-converting enzyme 2 (ACE-2). Neutralization was determined using the SARS-CoV-2 Surrogate Virus Neutralization Test Kit (Genscript, cat.#L00847) according to the manufacturer's recommendations with minor changes. In short, the undiluted phages or antibodies (single dilution at 65 µg/mL end concentration or two-fold dilutions series) were incubated with RBD-HRP in HRP-dilution buffer in a 1:1 ratio and incubated at 37 °C for 1 hour. Samples (100 µL) were added to a ACE2-coated well and incubated for 1 hour at 37 °C. After washing, 100 µl of TMB Solution was added to each well and incubated for ±15 minutes (precipitated phages) or ±10 minutes (antibodies in supernatant). After quenching the solution with 50 µl of Stop solution the absorbance was recorded at 450 nm on a Tecan Infinite F50 plate reader.

Purified antibodies were detected using the anti-Human-HRP antibody conjugate.

Subsequently, a neutralization titration assay was performed for the antibodies.

### Re-cloning into mammalian expression vectors

The antibody insert was digested from the phagemid vector using the SfiI (Bioké, cat.#R0123) and NotI (Bioké, cat.#R3189) restriction sites. The insert was ligated in a mammalian expression vector (pABS-hIgG1-hinge-CH2-CH3), which contains the hinge and the Fc domain (human CH2 and CH3). First, the expression vector was linearized using the same restriction sites and then the insert was ligated using T4 DNA ligase (Bioké, cat.#M0202). The resulting antibodies are expressed as ScFv/VHH-hIgG1Fc domains. Finally, the chosen antibodies were recloned in the vectors pABS-hIgG1 and pABS-hkappa or pABS-hlambda for full size antibody expression. The antibody inserts, VL or VH, were orded at Thermo Fisher Scientific in a pMA-RQ vector surrounded by BsmI (Bioké, cat.#R0134) and BsiWI (Bioké, cat.#R0553) restriction sites. After digestion the insert was ligated into the corresponding linearized vector using T4 DNA ligase. All sequences were verified via Sanger sequencing (Macrogen Europe B.V.) using specific primer for the pABS plasmids (Table 3, Eurogentec) and analysed with CLC main workbench software.

### Cell culture

HEK293F cells (Invitrogen) were cultured in suspension in Freestyle^{™} 293 expression medium (Thermo Fisher Scientific, cat.#12338) at 37 °C, 110 rpm, 8 % CO₂ and 95 % humidity. Cells were sub-cultured 3x-week at 0.3-0.5 million/mL in culture flasks. For counting and viability check an automated cell counter (Nexcelom Cellometer auto T4 Plus) was used and cells were diluted 1:1 in 0.4% Trypan Blue (Thermo Fisher Scientific, cat.#15250061).

### Antibody expression

Plasmids encoding for the antibodies were amplified in chemically competent *E. coli* XL1-blue bacteria and harvested using Mini/Midi/Maxi prep (Qiagen, cat.#27106, cat.#27191, cat.#12143; Sigma-Aldrich, cat.#NA0410). HEK293F cells (1 million/mL) were transfected with FectoPro transfection reagent (PolyPlus-transfection, cat.#116) using 500 µg DNA/million cells for ScFv/VHH-hIgG1Fc format or 333 µg DNA/million cells of VL and 167 µg/million cells of VH for full size antibody expression. After 2.5 hours, transfection was boosted by addition of 2 mM Sodium butyrate. Cells were incubated at 37 °C, 110 rpm, 8 % CO₂ and 95 % humidity. After 4-6 days the cells were checked for viability and the antibody was harvested when the viability dropped below 60%, as detected using automated cell counting. To harvest the antibody, the culture was first centrifuged at 300 xg for 10 minutes at 4 °C to remove the cells and next at 4816 xg for 1 hour at 4 °C to remove cell debris and aggregates. The supernatant, containing the antibody in culture medium, was stored at 4 °C until further use. Antibody concentration was assessed using Octet measurements (ForteBio Octet Red96) with a Protein A coated sensor (Molecular Devices, cat.#18-5010) and a hIgG1 standard curve (Sartorius, cat.#18-1118). Typical concentrations ranged between 0.1 and 0.8 mg/mL.

### Antibody purification

Antibodies were purified using an Äkta pure 25 system (Cytiva) equipped with a 1 mL HiTrap MabSelect SuRe column (Cytiva, cat.#11003493) and two 5 mL HiTrap Desalting columns with Sephadex G-25 resin (Cytiva, cat.#17140801) for tandem purification. In short, Antibodies in cell supernatant were filtered using a 0.45 µm filter before manual loading on a sample loop, superloop or using a pump. First, antibodies were captured on the mAb Select SuRe column. The column was washed with 10 CV bind buffer (20 mM Sodium Phosphate, 150 mM NaCl, pH 7.2). Antibodies were eluted from the first column using Elution buffer (100 mM Sodium Citrate, pH 3). The pure antibody was detected using UV absorbance at 280 nm and directly loaded on the desalting columns to exchange the buffer to PBS (pH 7.2) using 5 CV. Pure antibodies were collected using a fraction collector based on the UV absorbance at 280 nm. Concentration of antibodies was determined by measuring the absorbance at 280 nm using NanoDrop2000 (Thermo Life Science) Protein.

### SEC analysis of antibodies

SEC analysis of purified antibodies were performed on an Äkta Pure 25 (Cytiva) equipped with a Superdex 200 Increase 3.2/300 (Cytiva, cat.#28990946) in PBS buffer. All antibodies were centrifuged at 13,800 ×g for 5 min to remove visible aggregates and 100 µg of antibody was used for each loading. The Unicorn 7.6 software was used to analyze the data.

### Biolayer interferometry (BLI) measurement of affinity

Kinetic assays were performed with biolayer interferometry on the Octet RED96 system (ForteBio) to measure antibody affinity. First, 25nM biotinylated RBD (Bio-connect, #40592-V08H-B) was immobilized on streptavidin biosensors. The remaining free streptavidin sites were quenched using 10 µg/ml EZ-linked Biocytin (Thermo Fisher Scientific, cat.#28022). After immobilization and quenching, the sensors were dipped into PBST for 200 seconds to establish a baseline and remove unbound ligand. Subsequently, each antibody sample was loaded onto the sensor in a serial dilution (1.56 - 50 nM) for 350 seconds, corresponding to the association step. This was followed by a dissociation step where the sensors were dipped into PBST for ±1000 seconds. After each antibody affinity measurement, the sensors were regenerated with 10 mM Glycine (pH 2.5) and neutralized with PBST. A maximum of 25 regeneration and/or 5 preservation cycles were used for the immobilized RBD sensors. Data analysis was performed with the Octet Data Anlysis HT 12.0 software. After reference substraction of the binding curves, the y-axis were aligned to average of baseline. To obtain the K_{D} values of the mAbs, the binding curves were fit by a 1:1 binding model and a global fitting method was applied.

### Epitope binning of antibodies

To identify mAbs with considerable similar or overlapping epitopes a cross competition assay was performed. Epitope binning was conducted using an in-tandem format involving the immobilization of 25 nM biotinylated RBD (Bio-connect, #40592-V08H-B) on streptavidin biosensors which are subsequently presented to the two competing antibodies. First, the RBD-loaded sensor was dipped into 100 nM of first antibody until flattening of the binding curve was visible. The sensors with antigen-antibody 1 were then incubated in the second antibody (ScFv 10 µg/mL; VHH 8 µg/mL) for 120 seconds. In each round, also binding without first or second antibody was recorded. A total of 21 × 21 sets of antibody binning were performed to obtain the full profile of antibody epitope bins. For data analysis, if a second antibody still binds RDB which has been pre-captured by a first antibody, the antibodies are defined as non-overlapping epitopes ("+"); if the second antibody does not bind the RBD pre-captured by the first antibody the antibodies are competitive ("-"). Antibody pairs with competition ("-") are grouped in the same bin.

### ELISA titration of mAb binding and fitting of EC₅₀

Corning high-binding assay plates were coated with recombinant sCoV2-RBD or sCoV-RBD protein (1 µg/ml) at 4 °C overnight and blocked with 5% skim milk at 37 °C for 2 h. Serially diluted antibodies were added at a volume of 100 µl per well for incubation at 37 °C for 2 h. The anti-human IgG Fab2 HRP-conjugated antibody was diluted 1:5000 and added at a volume of 100 µl per well for incubation at 37 °C for 1 h. The plates were washed 3-5 times with PBST (0.05% Tween-20) between incubation steps. TMB substrate was added 100 µl per well for color development for 3 min and 2 M H₂SO4 was added 50 µl per well to stop the reaction. The OD 450 nm was read by a SpectraMax microplate reader. The data points were plotted by GraphPad Prism8, and the EC₅₀ values were calculated using a three-parameter nonlinear model.

### Primers for Sanger Sequencing

**Table 9**

| Plasmid | Primer Sequence (5' → 3') |
|---|---|
| pHenIX | TCACACAGGAAACAGCTATGA |
| pABS plasmids | AGGGAGACCCAAGCTAG |

### References

1. Wang, L. et al. Importance of neutralizing monoclonal antibodies targeting multiple antigenic sites on the middle east respiratory syndrome coronavirus spike glycoprotein to avoid neutralization escape. J. Virol. 92, (2018).
2. ter Meulen, J. et al. Human monoclonal antibody combination against SARS coronavirus: synergy and coverage of escape mutants. PLoS Med. 3, e237 (2006).
3. Ku, Z., Xie, X., Davidson, E. et al. Molecular determinants and mechanism for antibody cocktail preventing SARS-CoV-2 escape. Nat Commun 12, 469 (2021).
4. Baum, A. et al. Antibody cocktail to SARS-CoV-2 spike protein prevents rapid mutational escape seen with individual antibodies. Science 369, 1014-1018 (2020).
5. Zost, S. J. et al. Potently neutralizing and protective human antibodies against SARS-CoV-2. Nature 584, 443-449 (2020).
6. Du, S. et al. Structurally resolved SARS-CoV-2 antibody shows high efficacy in severely infected hamsters and provides a potent cocktail pairing strategy. Cell 183, 1013-1023.e13 (2020).
7. Wu, Y. et al. A non-competing pair of human neutralizing antibodies block COVID-19 virus binding to its receptor ACE2. Science 368, 1274-1278 (2020).

## Claims

1. An antibody, or an antigen-binding fragment thereof, that binds to the spike protein of coronavirus SARS-CoV-2, wherein the antibody comprises a set of three heavy chain complementarity determining regions (CDRH1, CDRH2 and CDRH3) and three heavy chain complementarity determining regions (CDRL1, CDRL2 and CDRL3), wherein the set of CDRH1, CDRH2, CDRH3, CDRL1, CDRL2 and CDRL3 amino acid sequences is selected from:
(a) SEQ ID NOs: 1170, 1171, 1172, 1174, 1175 and 1176;
(b) SEQ ID NOs: 1186, 1187, 1188, 1190, 1191 and 1192;
(c) SEQ ID NOs: 1202, 1203, 1204, 1206, 1207 and 1208;
(d) SEQ ID NOs: 1194, 1195, 1196, 1198, 1199 and 1200;
(e) SEQ ID NOs: 770, 771, 724, 774, 775 and 776;
(f) SEQ ID NOs: 810, 811, 812, 814, 815 and 816; and
(g) SEQ ID NOs: 786, 787, 788, 790, 791 and 792.

2. The antibody or fragment of claim 1, comprising
(a) a heavy chain variable region having at least 80% amino acid sequence identity to any one of SEQ ID NOs: 1169, 1185, 1201, 1193, 769, 809 and 785, or comprising the amino acid sequence of any one of SEQ ID NOs: 1169, 1177, 1185, 1201, 1193, 769, 809 and 785; and/or
(b) a light chain variable region having at least 80% amino acid sequence identity to any one of SEQ ID NOs: 1173, 1189, 1205, 1197, 773, 813 and 789, or comprising the amino acid sequence of any one of SEQ ID NOs: 1173, 1189, 1205, 1197, 773, 813 and 789.

3. A combination of antibodies comprising two or more antibodies according to claim 1 or claim 2.

4. The combination of antibodies according to claim 3, comprising one of each of the four antibodies according to claim 1 (a) to (d); one of each of the four antibodies according to claim 1 (a), (b), (d) and (e); one of each of the four antibodies according to claim 1 (a), (b), (d) and (f); or one of each of the four antibodies according to claim 1 (a), (b), (d) and (g).

5. The antibody of claims 1 or claim 2, or the combination of antibodies according to claim 3 or claim 4, wherein the antibody, or one or more of the combination antibodies, comprise heavy and light chain variable regions having the amino acid sequences of:
(a) SEQ ID NOs: 1169 or 1177 and 1173;
(b) SEQ ID NOs: 1185 and 1189;
(c) SEQ ID NOs: 1201 and 1205;
(d) SEQ ID NOs: 1193 and 1197;
(e) SEQ ID NOs: 769 and 773;
(f) SEQ ID NOs: 809 and 813; and/or
(g) SEQ ID NOs: 785 and 789.

6. A pharmaceutical composition comprising the antibody according to any one of claims 1, 2 and 5, or the combination of antibodies according to any one of claims 3 to 5, and optionally at least one pharmaceutically acceptable diluent or carrier.

7. The antibody according to any one of claims 1, 2 and 5, the combination of antibodies according to any one of claims 3 to 5, or the pharmaceutical composition according to claim 6, for use in a method for treatment of a human or animal body by therapy.

8. The antibody according to any one of claims 1, 2 and 5, the combination of antibodies according to any one of claims 3 to 5, or the pharmaceutical composition according to claim 6, for use in a method of treating or preventing a coronavirus infection, or a disease or complication associated with coronavirus infection.

9. A method of treating a subject comprising administering a therapeutically effective amount of the antibody according to any one of claims 1, 2 and 5, or the combination of antibodies according to any one of claims 3 to 5, or the pharmaceutical composition according to claim 6, to the subject.

10. The method according to claim 9, wherein the method is for treating a coronavirus infection, or a disease or complication associated with coronavirus infection.

11. A method of identifying the presence of coronavirus, or a protein or a protein fragment thereof, in a sample, comprising:
(i) contacting the sample with the antibody according to any one of claims 1, 2 and 5, or the combination of antibodies according to any one of claims 3 to 5; and
(ii) detecting the presence or absence of an antibody-antigen complex,
wherein the presence of the antibody-antigen complex indicates the presence of coronavirus, or a protein or a protein fragment thereof, in the sample.

12. One or more polynucleotides encoding the antibody according to any one of claims 1, 2 and 5, or the combination of antibodies according to any one of 3 to 5.

13. One or more vectors comprising the polynucleotide or polynucleotides of claim 12.

14. A host cell comprising the vector or vectors of claim 13.

15. A method for producing an antibody according to any one of claims 1, 2 and 5, or the combination of antibodies according to any one of 3 to 5, the method comprising culturing the host cell of claim 14 and isolating the antibody or antibodies from the culture.
